(19) Europäisches Patentamt
European Patent Office
Office européen des brevets

(11) Publication number: **0 154 142**
**B1**

(12) # EUROPEAN PATENT SPECIFICATION

(45) Date of publication of patent specification: **12.07.89**

(51) Int. Cl.4: **C 07 D 491/147,**
C 07 D 495/14,
C 07 D 471/14,
C 07 D 491/22,
C 07 D 513/22, A 61 K 31/435

(21) Application number: **85100732.8**

(22) Date of filing: **25.01.85**

(54) **Substituted hexahydro arylquinolizines.**

(30) Priority: **02.02.84 US 576233**

(43) Date of publication of application:
**11.09.85 Bulletin 85/37**

(45) Publication of the grant of the patent:
**12.07.89 Bulletin 89/28**

(84) Designated Contracting States:
**AT BE CH DE FR GB IT LI LU NL SE**

(56) References cited:
**DE-A-2 924 880**
**DE-B-1 470 065**
**GB-A-1 435 573**
**GB-A-2 106 909**

**Medicinal Chemistry, 3rd edition, A. Burger, pp. 76-77**

(73) Proprietor: **MERCK & CO. INC.**
**126, East Lincoln Avenue P.O. Box 2000**
**Rahway New Jersey 07065-0900 (US)**

(72) Inventor: **Huff, Joel R.**
**738 Bergey Mill Road**
**Lederach, PA 19450 (US)**
Inventor: **Vacca, Joseph P.**
**766 Eisenhauer Drive**
**Telford, PA 18969 (US)**
Inventor: **Baldwin, John J.**
**621 Gypsy Hill Circle**
**Gwynedd Valley, PA 19437 (US)**

(74) Representative: **Blum, Rudolf Emil Ernst et al**
**c/o E. Blum & Co Patentanwälte Vorderberg 11**
**CH-8044 Zürich (CH)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention is concerned with noval substituted hexahydro arylquinolizines or pharmaceutically acceptable salts thereof which are selective $\alpha_2$-adrenoceptor antagonists and are of value in conditions where selective antagonism of the $\alpha_2$-adrenoceptor is desirable for example as antidepressant, antihypertensive, or antidiabetic agents or platelet aggregation inhibitors. It also relates to processes for preparing the novel compounds and pharmaceutical compositions comprising the novel compounds.

The concept that the complex clinical state of depression is linked to a functional deficiency of monoamines in the central nervous system is now widely accepted. Numerous biochemical and clinical observations support the proposal that many forms of depressive illness are associated with reductions in adrenergic activity at functionally important sites in the brain. Thus, classical antidepressive drugs, such as amitriptyline and imipramine, are believed to act by blocking the neuronal reuptake of norepinephrine and/or serotonin, thereby enhancing the availability of the monoamines as neurotransmitters.

In addition to $\alpha_1$-adrenergic receptors which mediate postsynaptic responses to the neurotransmitter, norepinephrine, other adrenergic receptors are present at or near sympathetic terminals. These latter receptors, $\alpha_2$-adrenergic receptors, form part of a negative feedback system which modulates noradrenergic neurotransmission by controlling the impulse-induced release of norepinephrine from presynaptic terminals. Activation of $\alpha_2$-adrenergic receptors results in a decrease in the amount of norepinephrine normally released from the nerve terminals by nerve impulses while antagonism of $\alpha_2$-adrenergic receptors increases norepinephrine release. Therefore, molecules that block $\alpha_2$-adrenergic receptors afford an alternate approach to enhancement of noradrenergic function and the treatment of depression associated with an absolute or relative deficiency of adrenergic function.

$\alpha_2$-Adrenergic receptor antagonism is also associated with antidiabetic, antihypertensive and platelet aggregation inhibition activity.

Detailed Description of the Invention

The present invention concerns compounds of structural formula I

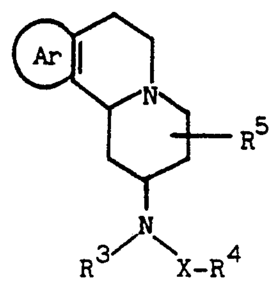

I

or pharmaceutically acceptable salts of the compounds of formula I, wherein

Ar represents an aromatic heterocycle selected from

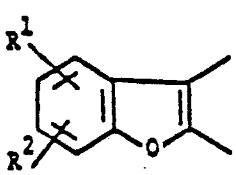
benzofuro-,

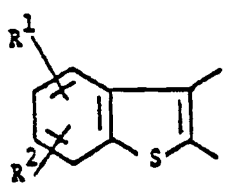
benzothieno-,

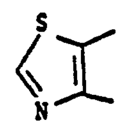
thiazolo-,

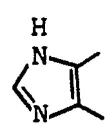
imidazo-,

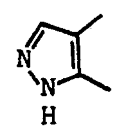
pyrazolo-,

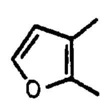
furo-,

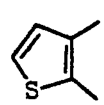
thieno-, and

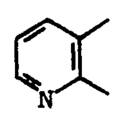
pyridino-;

$R^1$ and $R^2$ are independently:
1) hydrogen,
2) halo,
3) hydroxy,
4) $C_{1-3}$alkoxy, or
5) $C_{1-6}$alkyl;

$R^3$ is

1) hydrogen,

2) $-\overset{O}{\overset{\|}{C}}-R$, wherein R is hydrogen or $C_{1-3}$alkyl,

3) $C_{1-6}$alkyl, either unsubstituted or substituted with one or more of:

a) hydroxy,

b) carboxy,

c) $C_{1-3}$alkoxycarbonyl,

d) halo,

e) $C_{1-3}$alkoxy,

f) $-CONR^6R^7$, wherein $R^6$ and $R^7$ are independently from each other hydrogen or $C_{1-5}$alkyl or $R^6$ and $R^7$ form together with the nitrogen atom to which they are attached a pyrrolidino-, piperidino-, morpholino-, piperazino- or N—$C_{1-3}$ alkylpiperazino-ring or

g) $-NR^6R^7$; wherein $R^6$ and $R^7$ are as defined in 3f);

$$X \text{ is } -\overset{O}{\overset{\|}{C}},\quad -\overset{S}{\overset{\|}{C}}- \text{ or } -\underset{\underset{O}{\|}}{\overset{\overset{O}{\|}}{S}}-\text{; and}$$

$R^4$ is

1) $-OR^8$, wherein $R^8$ is hydrogen or $C_{1-6}$alkyl, either unsubstituted or substituted with one or more groups of formula OR, or —NRCOR, wherein R is hydrogen or $C_{1-3}$alkyl,

2) $-N(R^8)_2$, wherein $R^8$ is as defined in 1),

3) $-CO_2R^8$, wherein $R^8$ is as defined in 1),

4) $-CONR^6R^7$, wherein $R^6$ and $R^7$ are independently from each other hydrogen or $C_{1-5}$alkyl or $R^6$ and $R^7$ form together with the nitrogen atom to which they are attached a pyrrolidino-, piperidino-, morpholino-, piperazino- or N—$C_{1-3}$ alkylpiperazino-ring,

5) $C_{1-6}$alkyl, either unsubstituted or substituted with groups selected from $-OR^8$, -halo, $-CO_2R^8$ and $-CONR^6R^7$, wherein $R^6$ and $R^7$ are as defined in 4) and $R^8$ is as defined in 1,

6) $C_{2-5}$alkenyl,

7) $C_{2-5}$alkynyl,

8) $C_{3-6}$cycloalkyl,

9) 5- or 6-membered heterocycle selected from imidazo, thiazolo, oxazolo, piperidino, piperazino, pyridino and pyrazino, or

10) carbocyclic aryl of 6—10 carbon atoms, either unsubstituted or substituted with one or more of the groups selected from halo and OR, wherein R is hydrogen or $C_{1-3}$alkyl or $R^3$ and the group $-X-R^4$ form together with the nitrogen atom to which they are attached a heterocyclic structure selected from 2-oxazolidinon-1-yl and succinimidoyl and $R^5$ is

1) hydrogen,

2) $C_{1-6}$alkyl, either unsubstituted or substituted with one or more of the groups of formula $-OR^8$, $-NR^8COR^8$, or $-CO_2R^8$, wherein $R^8$ is hydrogen or $C_{1-6}$alkyl, either unsubstituted or substituted with one or more of the groups of formula OR, or —NRCOR, wherein R is hydrogen or $C_{1-3}$alkyl,

3) $-CO_2R^8$, wherein $R^8$ is as defined in 2),

4) $-CONR^6R^7$, wherein $R^6$ and $R^7$ are independently from each other hydrogen or $C_{1-5}$alkyl or $R^6$ and $R^7$ form together with the nitrogen atom to which they are attached a pyrrolidino-, piperidino-, morpholino-, piperazino- or N—$C_{1-3}$ alkylpiperazino-ring or

provided that $R^5$ is in the position 1 or the position 3, the radical $R^5$ can form together with the radical $R^3$ and the nitrogen atom to which it is attached or together with the group $-X-R^4$ and the nitrogen to which said group is attached, a 5- or 6-membered ring and with the provision that if the group $-X-R^4$ is a group having the formula

$$-\overset{O}{\overset{\|}{C}}-NH_2,\quad -\overset{S}{\overset{\|}{C}}-NH_2,\quad -\underset{\underset{O}{\|}}{\overset{\overset{O}{\|}}{S}}\text{-alkyl} \quad\text{or}\quad -\underset{\underset{O}{\|}}{\overset{\overset{O}{\|}}{S}}\text{-carbocyclic aryl,}$$

then the group Ar must not be the aromatic heterocycle thieno or pyridino.

Compounds which are somewhat structurally related to the new compounds of formula I are described in the British patent specification 1 435 573. In said compounds, however, the heterocyclic structure which corresponds to the aromatic heterocycle Ar of the claimed compounds is an indolo structure and not one of the heterocyclic structures defined in formula I.

Also in the British patent 2 106 909 there are described compounds which are somewhat structurally related to the claimed compounds. Said compounds, however, differ from the claimed compounds in that the fused ring, corresponding to the aromatic heterocyclic structure Ar of the compounds of formula I is a benzene ring.

The pharmaceutically acceptable salts of the compounds of formula I coming within the purview of this invention include the pharmaceutically acceptable acid addition salts. Acids useful for preparing these acid addition salts include, inter alia, inorganic acids, such as the hydrohalic acids (e.g., hydrochloric and hydrobromic acid), sulfuric acid, nitric acid, and phosphoric acid, and organic acids such as maleic, fumaric, tartaric, citric, acetic, benzoic, 2-acetoxybenzoic, salicyclic, succinic, theophylline, 8-chlorotheophylline, p-aminobenzoic, p-acetamidobenzoic, methanesulfonic, or ethanedisulfonic acid.

In a preferred embodiment of this invention, Ar is $R^1,R^2$-benzo[b]furo- or $R^1,R^2$-benzo[b]thieno. It is further preferred that $R^1$ and $R^2$ be hydrogen or halo and $R^3$ be $C_{1-6}$alkyl, especially methyl.

In preferred compounds of formula I, furthermore,

X is 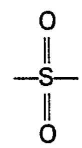 and

$R^5$ is hydrogen or $C_{1-6}$alkyl.

In preferred compounds of formula I, furthermore,

$R^4$ is $C_{1-6}$alkyl, either unsubstituted or monosubstituted with groups selected from —OH, -halo, —$CO_2R^8$ and —$CONR^6R^7$, wherein

$R^8$ is hydrogen or $C_{1-6}$alkyl, either unsubstituted or substituted with one or more of the groups of formula OR, or —NRCOR, wherein

R is hydrogen or $C_{1-3}$alkyl and $R^6$ and $R^7$ are independently from each other hydrogen or $C_{1-5}$alkyl or $R^6$ and $R^7$ form together with the nitrogen atom to which they are attached a pyrrolidino-, piperidino-, morpholino-, piperazino or N—$C_{1-3}$alkylpiperazino-ring;

—$N(R^{8'})_2$, wherein $R^{8'}$ is $C_{1-3}$alkyl, $C_{2-5}$alkenyl, $C_{6-10}$carbocyclic aryl, —$CO_2R^8$, wherein $R^8$ is hydrogen or $C_{1-6}$alkyl, either unsubstituted or substituted with one or more of OR, or —NRCOR, wherein R is hydrogen or $C_{1-3}$alkyl, or 5 or 6-membered heterocycle selected from imidazo, thiazolo, oxazolo, piperidino, piperazino, pyridino and pyrazino.

In the most preferred compounds of formula I $R^1$ and $R^2$ are hydrogen, $R^3$ is methyl, $R^4$ is $C_{1-6}$alkyl, hydroxypropyl, hydroxyethyl, dimethylamino, $C_{1-3}$ alkoxycarbonylethyl, or dimethylaminocarbonylmethyl, and $R^5$ is hydrogen.

The inventive compounds of formula I are depicted herein as having a 12bα,2β-configuration

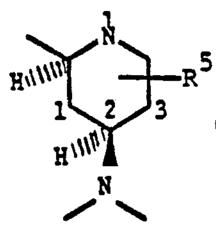 (racemic)

and it is the more preferred isomer for $\alpha_2$-adrenoceptor blockade activity. However, the 12bα,2α isomers are also active $\alpha_2$-adrenoceptor blockers and are considered to be within the scope of this invention. Each of the 12bα,2α and 12bα,2β-configurational isomers are racemates capable of being resolved into dextrorotatory and levorotatory enantiomers. This invention includes these pure enantiomers as well as all mixtures thereof, especially the racemates.

A further subject of the present invention is a process for the preparation of the compounds of formula I

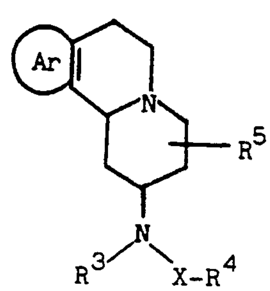

I

or pharmaceutically acceptable salts of the compounds of formula I, wherein Ar, $R^1$, $R^2$, $R^3$, X, $R^4$ and $R^5$ are as defined above. Said process is characterized in that the compound of formula IIa

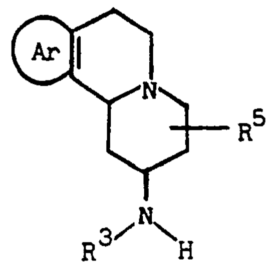

IIa ,

wherein Ar, $R^1$, $R^2$, $R^3$ and $R^5$ are as defined in formula I is acylated with an acylating reagent which introduces the acyl group of formula $—X—R^4$, wherein $R^4$ and X are as defined in formula I and which acylating agent is an acid halide, an activated ester, an acid anhydride or a mixed acid anhydride and in which process the compounds of formula I are isolated in the free form or as pharmaceutically acceptable salts thereof.

Preferred acylating agents used in said process have the following formula III $R^4$—X-halo wherein halo is chloro, bromo or iodo, preferably chloro, and

$$X \text{ is } -\overset{O}{\overset{\|}{C}}-, \quad -\overset{S}{\overset{\|}{C}}- \text{ or } -\underset{\underset{O}{\|}}{\overset{\overset{O}{\|}}{S}}-.$$

The acylation reaction is preferably conducted in an inert solvent in the presence of an acid acceptor. Examples for inert solvents are chlorinated hydrocarbons like methylene chloride, chloroform or 1,2-dichloroethane and examples for acid acceptors are triethylamine, pyridine, alkali metal carbonates, or basic anion exchange resins.

The reaction usually proceeds readily at about room temperature but any temperature from about 0°C to the boiling point of the reaction mixture is reasonable depending on the reactivity of the particular acyl halide. Reaction times of about half an hour to about 48 hours are required, and in most cases about one to 18 hours suffices.

A further object of the present invention is a process for the preparation of compounds of structural formula Ia

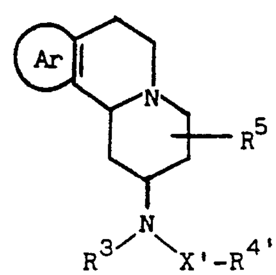

Ia

or pharmaceutically acceptable salts thereof, wherein Ar, $R^1$, $R^2$, $R^3$ and $R^5$ are as defined in formula I and $—X'—R^{4'}$ is a group having the structure

$$-\overset{O}{\overset{\|}{C}}-\overset{H}{\overset{|}{N}}-R^8 \quad \text{or} \quad -\overset{S}{\overset{\|}{C}}-\overset{H}{\overset{|}{N}}-R^8$$

wherein $R^8$ is hydrogen or $C_{1-6}$alkyl, either unsubstituted or substituted with one or more groups of formula OR, or —NRCOR, wherein R is hydrogen or $C_{1-3}$alkyl. Said process is characterized in that a compound of formula IIa

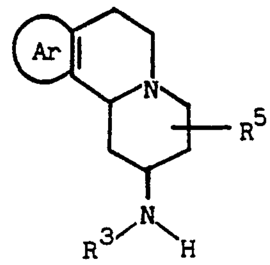

IIa ,

wherein Ar, $R^1$, $R^2$, $R^3$ and $R^5$ are as defined in formula Ia, is treated with an isocyanate of formula

$$O=C=NR^8$$

or an isothiocyanate of formula

$$S=C=NR^8$$

and the resulting compounds of formula Ia or pharmaceutically acceptable salts thereof are isolated.

Preferably said process is performed in an inert organic solvent, for example a lower alkanol like ethanol or propanol, or in 1,2-dimethoxyethane. The reaction is preferably performed at about room temperature or a temperature of up to 100°C for about 5 minutes to about 2 hours.

Preferred compounds of formula Ia prepared according to said process are those, wherein $R^8$ is an ethyl group.

A further object of the present invention is a process for the preparation of compounds of formula Ib

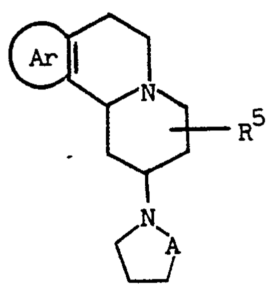

Ib

or pharmaceutically acceptable salts of the compounds of formula Ib wherein Ar, $R^1$, $R^2$ and $R^5$ are as defined in formula I and A is

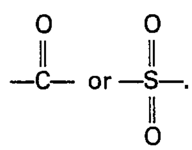

or .

Said process is characterized in that a compound of formula

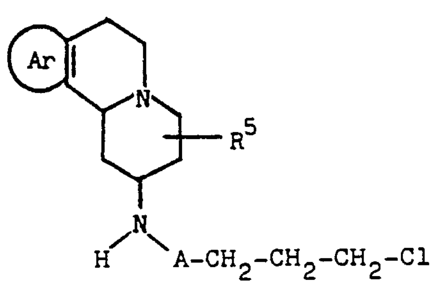

,

wherein Ar, $R^1$, $R^2$, $R^5$ and A are as defined in formula Ib is cyclisized in the presence of a strong base and the resulting compounds of formula Ib or pharmaceutically acceptable salts thereof are isolated.

Examples for strong bases with which said cyclization reaction can be performed are potassium t-butoxide, n-butyl lithium, or sodium hydride. Preferably the cyclization reaction is performed in an ethereal solvent such as 1,2-dimethoxyethane, diglyme, or THF at about 20°C to 60°C for about one to 5 hours.

The present invention, furthermore, concerns a process for the preparation of compounds of formula Ic

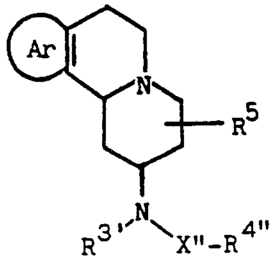

Ic

or pharmaceutically acceptable salts of the compounds of formula Ic, wherein Ar, $R^1$, $R^2$ and $R^5$ are as defined in formula I and $R^{3'}$ and the group —X″—$R^{4''}$ form together with the nitrogen atom to which they are attached a heterocyclic structure selected from 2-oxazolidinon-1-yl, or succinimidoyl, i.e. a heterocyclic structure having the following formulae

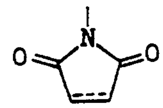 or 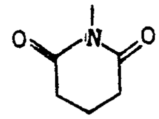

According to said process a compound of formula IIb

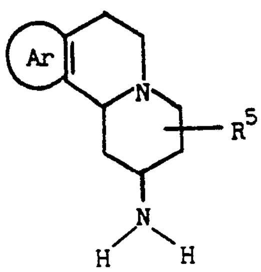

IIb ,

wherein Ar, $R^1$, $R^2$ and $R^5$ are as defined in formula Ic, is acylated with a cyclic dicarboxylic anhydride having the formula

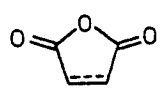 or 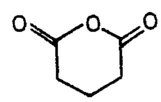

and the resulting compounds of formula Ic are isolated in the free form or as pharmaceutically acceptable salts of said compounds of formula Ic.

Preferably said acylation is either performed in an inert solvent, for example toluene, or without a solvent at a temperature of about 100 to 150°C for about 2 to 5 hours.

A further object of the present invention is a process for the preparation of compounds of structural formula Id

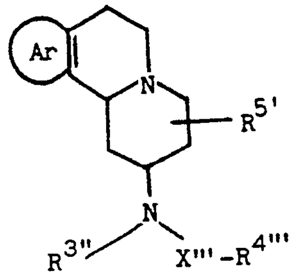

Id

or pharmaceutically acceptable salts of the compounds of formula Id, wherein Ar, $R^1$ and $R^2$ are as defined in formula I and $R^{5'}$ is in the position 1 or in the position 3 and said radical $R^{5'}$ forms together with the radical $R^{3''}$ and with the nitrogen atom to which it is attached, or together with the group

$—X'''—R^{4'''}$

and the nitrogen to which said group is attached, a 5- or 6-membered ring which is a cyclic carbamate, cyclic urea or cyclic sulfamide. Said process is performed by treating a starting material having a corresponding substituent in the position 1 or the position 3 with carbonyldiimidazole or sulphurylchloride. According to said process, accordingly, compounds of formula Id are prepared, which have in the 2-position a cyclic carbamate, a cyclic urea or a cyclic sulfamide corresponding to the following formulae

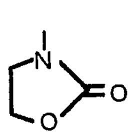 or 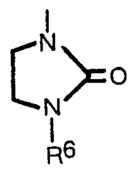 or 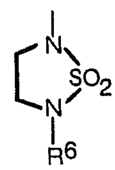

According to said process, for instance a corresponding starting material of formula II comprising a group of formula

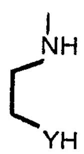

wherein Y is —O— or $NR^6$ can be treated with carbonyl diimidazole or sulfuryl chloride, preferably in an inert solvent such as dimethoxyethane or methylene chloride. The reaction is preferably performed in the presence of an acid acceptor like triethylamine or di(isopropyl)ethylamine at a temperature of about 20 to 60°C for about 5 to 18 hours.

A further object of the present invention is a pharmaceutical composition having $\alpha_2$-adrenoceptor antagonist activity which is characterized in that it comprises as pharmaceutically active ingredient an inventive compound of structural formula I or a pharmaceutically acceptable salt of the compound of formula I.

The inventive pharmaceutical compositions optionally furthermore comprise a pharmaceutically acceptable carrier.

In order to antagonize the $\alpha_2$-adrenergic receptors in a patient, the inventive pharmaceutical compositions are usually administered in such an amount, that the patient receives 0,01 to 20 mg of the compound of formula I or the pharmaceutically acceptable salts thereof per kg of body weight per day. Preferred amounts are in the range of about 0,1 mg to about 10 mg of the active ingredient per kg of body weight per day and the administration can be performed in a single dose or in 2 to 4 divided doses per day.

The inventive pharmaceutical compositions can contain inventive compounds of formula I or the pharmaceutically acceptable salts of the compounds of formula I as sole active ingredient or they can contain additionally further pharmaceutically active ingredients selected from antidepressants such as amitriptyline, imipramine or other norepinephrine or serotonin reuptake inhibitors or monoamine oxidase inhibitors.

If the inventive pharmaceutical compositions are administered in the above named dosages, they are useful for treating depression, diabetes, hypertension and abnormal platelet aggregation.

The inventive pharmaceutical compositions can be formulated so that they are suited to be administered orally, intraperitoneally, subcutaneously, intramuscularly, transdermally or intravenously.

Preferred inventive pharmaceutical compositions are such compositions which can be administered orally and they are for example formulated in the form of tablets, troches, capsules, elixirs, suspensions, syrups, wafers, or chewing gum.

The invention will be further illustrated with examples.

Example 1

N-(1,3,4,6,7,12bα-Hexahydro-2H-benzo[b]furo[2,3-a]quinolizin-2β-yl)-N,N',N'-trimethylsulfamide

Step A: Preparation of 3-Cyanomethylbenzo[b]furan

To a suspension of 2.64 gms (0.11 mole) of oil free sodium hydride in 200 ml of tetrahydrofuran (THF) was added dropwise a solution of 19.47 gms (0.11 mole) of diethylcyanomethylphosphonate in 75 ml of THF. After the $H_2$ evolution had ceased, a solution of 13.4 g (0.1 mole) of 3-(2H)-benzo[b]furanone in 100 ml of THF was added. The solution was heated at 70°C for 1.5 hrs, cooled, and poured into 500 ml of 5% HCl, and washed with ether. The ether phase was washed with brine, dried ($MgSO_4$), filtered and concentrated to give 15.4 g of a dark oil. The product was distilled at a temperature of 96—110°C at a pressure of 9,99915 Pa (0,075 mm Hg) to give 10.85 g of a yellow oil which crystallized upon standing.

Step B: Preparation of 2-(3-benzo[b]furanyl)ethylamine

A solution of 3.97 g (0.025 mole) of 3-cyanomethylbenzo[b]furan in 200 mL of diethyl ether was slowly added to a refluxing suspension of 3.84 g (0.1 mole) of lithium aluminum hydride in 400 mL of ether. The reaction was heated 3 hrs., cooled and water was slowly added. The suspension was filtered through a pad of filter aid and the filtrate was evaporated to give 3.2 g of oily product. The hydrochloride salt has m.p. 183—185°C.

Step C: Preparation of 3-(2-Formamidoethyl)benzo[b]furan

A solution of 2.35 g (0.015 mole) of 2-(3-benzo[b]furanyl)ethylamine and 5 mL of ethyl formate was heated at 60°C for 3 hours, poured into 2*N* HCl and washed with methylene chloride which in turn was washed with 5% sodium hydroxide (w/v), dried ($MgSO_4$), filtered and concentrated to give 2.70 g of product.

Step D: Preparation of 3,4-dihydrobenzo[b]furo[2,3-c]pyridine

2.28 Grams (0.012 mole) of 3-(2-formamidoethyl)benzo[b]furan was added to 28 g of polyphosphoric acid which was preheated to 100°C. After 1—1.5 hours, the reaction mixture was poured onto ice and the residues were washed with water. The polyphosphoric acid was dissolved in water, filtered through a pad of Celite® and made basic with concentrated ammonia. A precipitate was collected and dried to give 1.45 g of product, m.p. 170—171°C.

Step E: Preparation of 1,3,4,6,7,12bα-Hexahydrobenzo[b]furo[2,3-a]quinolizin-2-one

To a solution of 12 g (0.070 mol) of 3,4-dihydrobenzo[b]furo[2,3-c]pyridine dissolved in 500 mL of acetonitrile at 60°C was added 20 g (0.140 mol) of 2-trimethylsiloxy-1,3-butadiene followed by 13.6 g (0.10 mol) of anhydrous zinc chloride. The mixture was heated at 60°C for 1.5 hour, cooled to 25°C, and 30 mL of 5% HCl was added and stirred 10 minutes. 40% Sodium hydroxide was added until the reaction was basic; 200 mL of water was added; and the acetonitrile layer was separated. The aqueous layer was filtered through diatomaceous earth (Celite®) and washed with ether. The combined organic layers were dried ($Na_2SO_4$), filtered, and concentrated to a brown residue which was chromatographed (silica, ethyl acetate/hexane (1:1)) to give 8.2 g of product, m.p. 108—9°C.

Employing the procedures substantially as described in Example 1, Steps A through E, or in some cases, Steps C through E but substituting for the 3-benzofuranone used in Step A thereof the ketones described in Table I, or for the ethylamines used in Step C thereof, the corresponding ethylamines described in Table I, or for the butadienes used in Step E thereof, the corresponding substituted butadienes described in Table I, there are prepared the Ar[2,3-a]quinolizin-2-ones, also described in Table I by the following reactions:

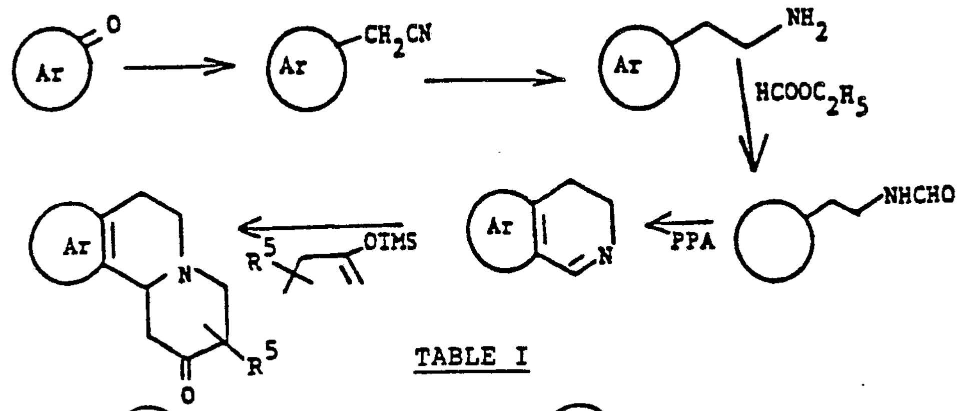


TABLE I

| 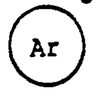 | $R^5$ | 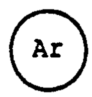 | $R^5$ |
|---|---|---|---|
| 10-chlorobenzo-[b]furo- | H | benzo[b]thieno- | 1-$CH_3$ |
| thieno- | H | 10-methylbenzo-[b]thieno- | 4-$COOCH_3$ |
| furo- | 3-$CH_3$ | 9-methoxybenzo-[b]thieno- | H |
| 11-hydroxy-benzo[b]furo | H | 11-fluorobenzo-[b]furo- | H |
| 10,11-dimethyl-benzo[b]furo- | H | 9-bromobenzo-[b]furo- | 1-$CON(CH_3)_2$ |
| | | 11-methoxybenzo-[b]furo- | H |

Step F: Preparation of N-(1,3,4,6,7,12bα-hexahydro-2H-benzo[b]furo[2,3-a]quinolizin-2β-yl)-N-methylamine

To a solution of 2.41 g (0.010 mol) of 1,3,4,6,7,12bα-hexahydrobenzo[b]furo[2,3-a]quinolizin-2-one dissolved in 60 mL of ether and 40 mL of benzene cooled to 0°C was added 5 mL of methylamine followed by a solution of 0.56 mL (0.052 mol) of titanium tetrachloride in 3 mL of benzene. The reaction was stirred at 0°C for 30 minutes, warmed to 25°C and stirred 2 hours. The mixture was filtered through a pad of diatomaceous earth (Celite®), and the salts were washed with benzene/ether (2:1). The filtrate was evaporated, giving 2.55 g of an oil. The oil was dissolved in 80 mL of ethanol and 0.38 g (0.010 mol) of sodium borohydride was added. The solution was stirred 18 hours, and 100 mL of water was added. Stirring was continued for 30 minutes; the ethanol was evaporated *in vacuo* and the aqueous phase was extracted with methylene chloride which was dried ($Na_2SO_4$), filtered, and concentrated, giving 2.56 g of product. The product was purified by chromatography (silica gel, chloroform saturated with $NH_3$) to yield 1.77 g of product. The dihydrochloride salt obtained from ethanolic HCl has m.p. 300°C.

Employing the procedure substantially as described in Example 1, Step F but substituting for the intermediates and reagents used therein, the Ar[2,3-a]quinolizin-2-ones and the amines of structure $R^3$ $NH_2$, described in Table II, there are prepared the N-(Ar[2,3-a]quinolizin-2β-yl)-N—$R^3$-amines, also described in Table II by the following reaction.

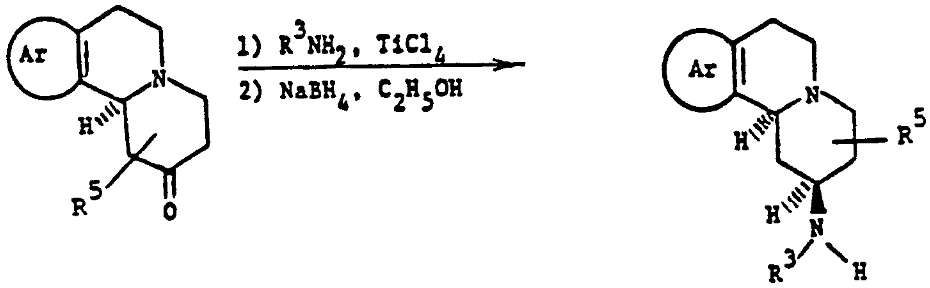


TABLE II

| Ar | $R^3$ | $R^5$ |
|---|---|---|
| 11-fluorobenzo[b]furo- | $CH_3$- | H |
| thieno- | n-$C_3H_7$- | H |
| furo- | $(CH_3)_2NCOCH_2$- | 1-$CH_3$ |
| 11-hydroxybenzo[b]furo- | $CH_3$- | H |
| 10,11-dimethylbenzo[b]-furo- | $CH_3NHC_2H_4$- | 3-$COOCH_3$ |
| benzo[b]thieno- | $CH_3OCH_2CH_2$- | H |
| 10-methylbenzo[b]thieno- | $CH_3$- | H |
| 9-methoxybenzo[b]thieno- | $C_2H_5$- | 4-CON(Me)$_2$ |
| 10-chlorobenzo[b]thieno- | $C_2H_5O_2CCH_2$- | H |
| 9-bromobenzo[b]furo- | H- | 4-$CH_3$ |
| 11-methoxybenzo[b]furo- | $C_2H_5$- | H |
| benzo[b]furo- | $HOC_2H_4$- | 3-$CH_3$ |
| benzo[b]furo- | n-$C_3H_7$- | H |
| benzo[b]furo- | $H_2NOCH_2C$- | H |
| benzo[b]furo- | $CH_3CO$- | 1-$COOCH_3$ |
| benzo[b]furo- | $ClCH_2CH_2CH_2$- | H |

Step G: Preparation of N-(1,3,4,6,7,12bα-hexahydro-2H-benzo[b]furo[2,3-a]quinolizin-2β-yl)-N,N',N'-trimethylsulfamide HCl

To a solution of 2.54 g (0.01 mol) of amine from Step F in 50 mL of methylene chloride was added 2.00 g (0.020 mol) of triethylamine followed by 2.80 g (0.02 mol) of dimethylsulfamoyl chloride. The mixture was stirred for 36—48 hours and then poured into 100 mL of 5% (w/v) NaOH which was then extracted with methylene chloride. The organic layer was dried ($Na_2SO_4$), filtered and concentrated to give 3.75 g of an oil which was chromatographed (silica/2% $CH_3OH/CHCl_3$) to give 2.92 g of product. The free base was acidified with ethanolic HCl. Addition of ether afforded the hydrochloride, m.p. 256—257°C.

Employing the procedure substantially as described in Example 1, Step G, but substituting for the dimethylsulfamoyl chloride and the amine used therein comparable amounts of the compounds $R^4$—X—Cl and the amines described in Table III there are produced the $(R^3)(R^4X)$ amines, also described in Table III, in accordance with the following reaction:

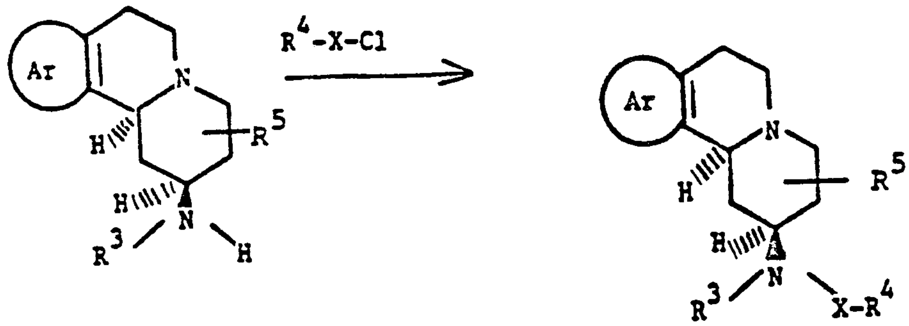


TABLE III

| Ar | $R^3$ | X | $R^4$ | $R^5$ | Reaction time (Hrs) | Salt mp (°C) |
|---|---|---|---|---|---|---|
| benzo[b]furo | $-CH_3$ | $-SO_2-$ | $-CH_2CH(CH_3)_2$ | H | 3 | HCl, 237-239 |
| benzo[b]furo | $-CH_3$ | -CO- | $-CH_2CH_3$ | H | 1 | HCl, 1.5 $H_2O$ 165-170 |
| benzo[b]furo | $-CH_3$ | -CO- | $-CH_2CH(CH_3)_2$ | H | 1 | HCl, 0.5 $H_2O$ 223-226 |
| benzo[b]furo | $-CH_3$ | -CO- | 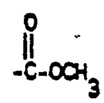 | H | 1 | HCl, 0.25 $H_2O$ 204-206 |
| benzo[b]furo | $-CH_3$ | -CO- | $-N(CH_3)_2$ | H | 48 | HCl, 0.75 $H_2O$ 174-177 |
| benzo[b]furo | $-CH_3$ | -CO- | $-OC_2H_5$ | H | 0.5 | HCl, 0.75 $H_2O$ 240-243 |
| benzo[b]furo | $-CH_3$ | -CO- | 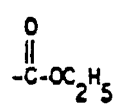 | H | 18 | HCl, 225 |
| benzo[b]furo | $-CH_3$ | -CO- | 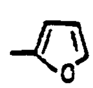 | H | 1.5 | HCl, 0.5 $H_2O$ 275-280 |
| benzo[b]furo | $-CH_3$ | $-SO_2-$ | 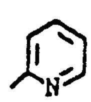 | H | 18 | HCl, 250 |
| benzo[b]furo | $-CH_3$ | -CO- | 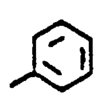 | H | 18 | HCl, 0.5 $H_2O$ 250 |

TABLE III (cont'd)

| | | | | | |
|---|---|---|---|---|---|
| benzo[b]furo | $-CH_3$ | -CO- | 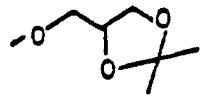 | H | |
| benzo[b]furo | $-CH_3$ | $-SO_2-$ | $CH_2CH_2OH$ | H | HCl, 250 |
| benzo[b]furo | $-CH_3$ | -CO- | 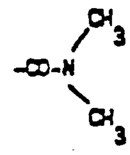 | H | HCl, 325 |
| benzo[b]furo | $HOCH_2CH_2-$ | $-SO_2$ | 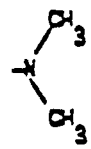 | H | HCl, 0.5 $H_2O$ |
| benzo[b]furo | $-CH_3$ | $-SO_2$ | 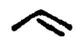 | H | HCl, 0.5 $H_2O$ 248 |
| benzo[b]furo | $-CH_3$ | $-SO_2-$ | $C_2H_5-$ | H | HCl, 257-260 |
| benzo[b]furo | n-$C_3H_7$ | $-SO_2$ | 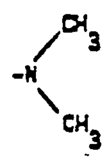 | H | HCl, 160 |
| benzo[b]furo | $-CH_3$ | $-SO_2$ | $CH_2CH_2COOCH_3$ | H | HCl, 227-28 |
| benzo[b]furo | $-CH_3$ | -CO- | $CH_2COOC_2H_5$ | H | HCl, 202-04 |
| benzo[b]furo | $-CH_3$ | -CO- | $CH_2OCH_3$ | H | HCl, 227-28 |
| benzo[b]furo | $-CH_2CONH_2$ | $-SO_2-$ | 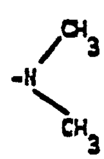 | H | HCl, 1.5 $H_2O$; 225-227 |
| benzo[b]furo | $CH_3-$ | $-SO_2-$ | $CH_2CH_2CH_2Cl$ | H | HCl, 234-236 |
| benzo[b]furo | $CH_3-$ | -CO- | 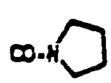 | H | HCl, 240-243 |
| benzo[b]furo | $CH_3-$ | $-SO_2-$ | 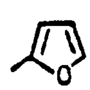 | H | HCl, 270-275 |

TABLE III (cont'd)

| | | | | | | |
|---|---|---|---|---|---|---|
| benzo[b]thieno | $CH_3$- | -$SO_2$- | $C_2H_5$ | H | | HCl, 240 |
| benzo[b]furo | $CH_3$- | -$SO_2$- | 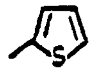 | H | | HCl, 270 |
| benzo[b]furo | $CH_3$- | -$SO_2$- | $CH_2COOC_2H_5$ | H | | HCl, 0.5 $H_2O$; 147-150 |
| benzo[b]furo | $CH_3$- | -$SO_2$- | 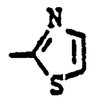 | H | | HCl; 260 |
| 11-fluorobenzo-[b]furo | $CH_3$- | -$SO_2$ | 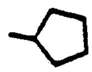 | 3-$CH_3$ | | |
| 11-methoxybenzo-[b]furo | $C_2H_5$- | -$SO_2$- | 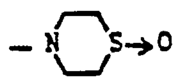 | 4-$CH_3$ | | |
| benzo[b]thieno | $CH_3$- | -CO- | 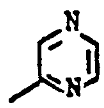 | H | | |
| benzo[b]furo | $CH_3$- | -$SO_2$- | 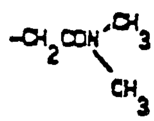 | H | | HCl, 0.5 $H_2O$ 151-153 |
| benzo[b]furo | -$CH_3$ | -CO- | 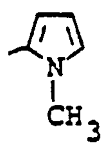 | H | 12 | 2HCl, 280-284 |
| benzo[b]furo | -$CH_3$ | -$SO_2$- | 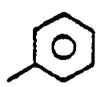 | H | 16 | HCl, 290-294 |
| 10-chlorobenzo-[b]furo | -$CH_3$ | -$SO_2$- | -$C_2H_5$- | H | 14 | HCl, 262-264 |
| benzo[b]furo | -$CH_3$ | -CO- | | H | 3 | HCl, 305-310 |
| benzo[b]furo | -$CH_3$ | -$SO_2$- | -$CH_3$ | H | 4 | HCl, 255(dec) |

TABLE III (cont'd)

| | | | | | | |
|---|---|---|---|---|---|---|
| (2R, 12bS)benzo-[b]furo | $-CH_3$ | $-SO_2-$ | 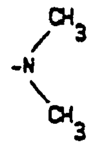 | H | 18 | HCl, 263-264 |
| benzo[b]furo | $-CH_3$ | $-SO_2-$ | $-CH_2Ph$ | H | 8 | HCl, 255-265 |
| 11-methoxybenzo-[b]furo | $-CH_3$ | $-SO_2-$ | -Et | H | 10 | HCl, 244-247 |
| (2S, 12bR)benzo-[b]furo | $-CH_3$ | $-SO_2-$ | 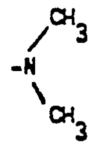 | H | 18 | HCl, 263-264 |
| benzo[b]thieno | $-CH_3$ | $-SO_2-$ | $-CH_2CH_2OH$ | H | 2 | HCl, $0.5H_2O$ 164-167 |
| benzo[b]thieno | $-CH_3$ | $-SO_2-$ | $-CH_3$ | H | 4 | HCl, 250 |
| 11-chlorobenzo-[b]furo | $-CH_3$ | $-SO_2-$ | $-C_2H_5-$ | H | 3 | HCl, $0.5H_2O$, 256-259 |
| benzo[b]thieno | $-CH_3$ | $-SO_2-$ | 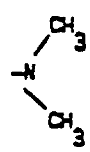 | H | 18 | HCl, 265-268 |
| 9-chlorobenzo-[b]furo | $-CH_3$ | $-SO_2-$ | $-C_2H_5-$ | H | 4 | HCl, 280 |
| (2S, 12bR)benzo-[b]furo | $-CH_3$ | $-SO_2-$ | $-CH_2CH_2OH$ | H | 1 | HCl, 265(dec) |
| (2R, 12bS)benzo-[b]furo | $-CH_3$ | $-SO_2-$ | $-CH_2CH_2OH$ | H | 1 | HCl, 265(dec) |
| (2R, 12bS)benzo-[b]furo | $-CH_3$ | $-SO_2-$ | $-CH_3$ | H | 1 | HCl, 280-284 |

TABLE III (cont'd)

| | | | | | | |
|---|---|---|---|---|---|---|
| benzo[b]furo | $-CH_3$ | $-SO_2-$ | 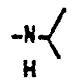 | H | 18 | HCl, 225-227 |
| benzo[b]furo | $-CH_3$ | $-SO_2-$ | 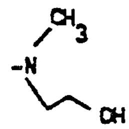 | H | 24 | HCl, $0.5H_2O$ 214-216 |
| 9-methoxybenzo-[b]furo | $-CH_3$ | $-SO_2-$ | $-C_2H_5-$ | H | 5 | HCl, 231-234 |
| 10-methoxybenzo-[b]furo | $-CH_3$ | $-SO_2-$ | $-C_2H_5$ | H | 4 | HCl, $H_2O$, 240-242 |
| 9-methoxybenzo-[b]furo | $-CH_3$ | $-SO_2-$ | $-N(CH_3)_2$ | H | 18 | HCl, $.75H_2O$, 234-236 |
| 10-methoxybenzo-[b]furo | $-CH_3$ | $-SO_2-$ | $-N(CH_3)_2$ | H | 18 | HCl, 247-248 |
| thieno | $-CH_3$ | $-SO_2-$ | $-N(CH_3)_2$ | H | 18 | HCl, $0.25H_2O$, 243-245(dec) |
| benzo[b]furo | $-CH_3$ | $-SO_2-$ | $-N(CH_2CH_2OH)_2$ | H | 24 | 0.5 $H_2O$, 140-142 |
| 10-chlorobenzo-[b]furo | $-CH_3$ | $-SO_2-$ | $-N(CH_3)_2$ | H | 16 | HCl, 0.25 $H_2O$ |
| 10-methylbenzo-[b]thieno | $-CH_3$ | $-SO_2-$ | $-C_2H_5$ | H | 6 | HCl, 280 |
| 11-chlorobenzo-[b]thieno | $-CH_3$ | $-SO_2-$ | $-C_2H_5$ | H | 4 | HCl, 278-281 |
| 11-chlorobenzo-[b]thieno | $-CH_3$ | $-SO_2-$ | $-N(CH_3)_2$ | H | 18 | HCl, 246-248 |

TABLE III (cont'd)

| | | | | | | |
|---|---|---|---|---|---|---|
| 9-hydroxybenzo-[b]furo | $-CH_3$ | $-SO_2-$ | $-C_2H_5$ | H | 3 | HCl, 301-304 |
| 11-Isopropyl-benzo[b]thieno | $-CH_3$ | $-SO_2-$ | $-C_2H_5$ | H | 6 | HCl, 228-230 |
| benzo[b]furo | $-CH_3$ | $-SO_2-$ | $-CH_3$ | 1α-$CH_3$ | 2 | HCl, 0.25$H_2$O; 270-273 |
| benzo[b]furo | $-CH_3$ | $-SO_2-$ | $-CH_3$ | 3α-$CH_3$ | 2 | HCl, 0.25$H_2$O; 262-264 |
| benzo[b]furo | $-CH_3$ | $-SO_2-$ | $-CH_3$ | 1β-$CH_3$ | 2 | HCl, 0.5$H_2$O; 250-252 |
| benzo[b]furo | $CH_3-$ | $-SO_2$ | 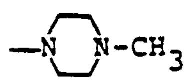 | 1-$CH_3$ | | |
| 9-methoxybenzo-[b]thieno | $C_2H_5$ | -CO- | $-CH_2CH_2CH_2-NH_2$ | 3-$C_2H_5$ | | |
| thieno- | n-$C_3H_7$ | $-SO_2-$ | 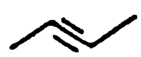 | 1-$C_3H_7$ | | |
| furo- | $(CH_3)NCOCH_2-$ | $-SO_2-$ | 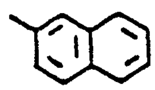 | H | | |
| 11-hydroxybenzo-[b]furo | $CH_3-$ | $-SO_2-$ | 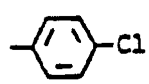 | 4-$COOCH_3$ | | |
| 10,11-dimethyl-benzo[b]furo- | $CH_3NHC_2H_4$ | $-SO_2-$ | 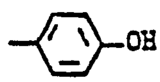 | H | | |
| 10-methylbenzo-[b]thieno- | $CH_3-$ | $-SO_2-$ | 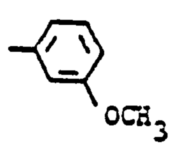 | 1-$COOCH_3$ | | |
| 10-chlorobenzo-[b]thieno- | $-C_2H_5CO_2CH_3$ | $-SO_2-$ | $-CH_3$ | H | | |

TABLE III (cont'd)

| | | | | |
|---|---|---|---|---|
| 9-bromobenzo-[b]furo- | H- | $-SO_2-$ | $-C_2H_5$ | H |
| benzo[b]furo | $CH_3CO-$ | $-SO_2-$ | $-CH_2\overset{O}{\overset{\|}{C}}N(CH_3)_2$ | $3-CH_2Ph$ |
| benzo[b]furo | $Cl(CH_2)_3-$ | $-SO_2-$ | $n-C_3H_7$ | H |

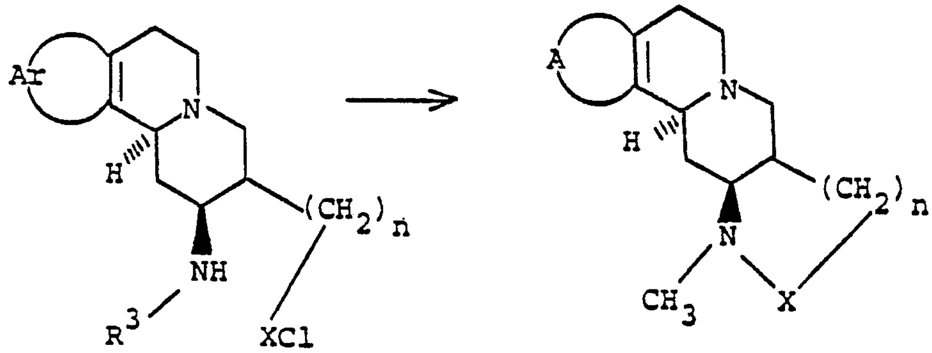

| | | | |
|---|---|---|---|
| benzo[b]furo | $-CH_3$ | $-CO-$ | $-CH_2-CH_2-$ |
| benzo[b]furo | $-CH_3$ | $-SO_2-$ | $-CH_2$ |

Example 2

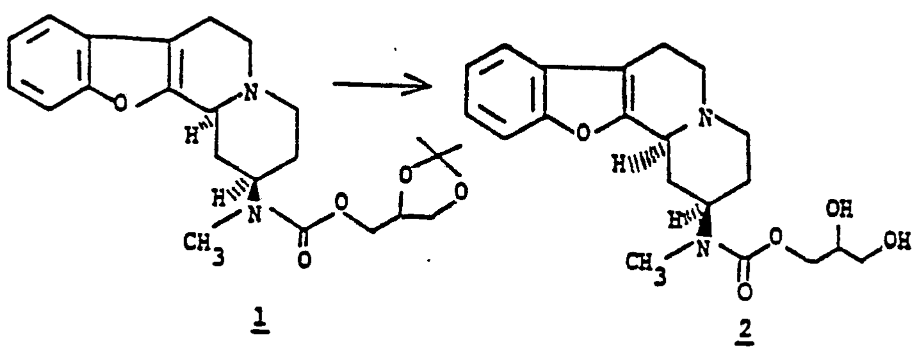

1 2

1-(2,3-Dihydroxypropyl)-N-(1,3,4,6,7,12bα-hexahydro-2H-benzo[b]furo[2,3-a]quinolizin-2β-yl)-N-methylcarbamate oxalate

The carbamate, 1 (0.324 g, 0.723 mmole) was dissolved in 5 mL of acetone and 5 mL of 3*N* HCl and was stirred at room temperature for 30 minutes; made basic with 40% NaOH; and was extracted with methylene chloride. The extract was dried, filtered and concentrated. The crude oil obtained was purified by spinning plate chromatography ($NH_3$ sat'd $CHCl_3$) to give 0.185 g of product (63%). The monoxalate salt has m.p. 83—86°C.

Example 3

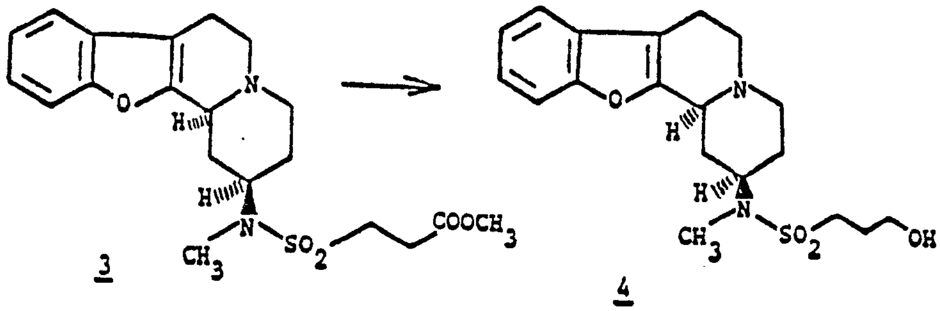

3 4

N-(1,3,4,6,7,12bα-Hexahydro-2H-benzo[b]furo[2,3-a]quinolizin-2β-yl)-N-methyl-3-hydroxypropanesulfonamide hydrochloride, 4

To the ester, 3, (0.130 g, 0.32 mmole) dissolved in 10 mL of ether at 0°C was added 0.016 g (0.42 mmole) of lithium aluminum hydride. After 30 minutes, the reaction was poured into dilute HCl and made basic with 40% NaOH. The aqueous solution was extracted with methylene chloride and the extract was dried, filtered and concentrated to an oil. The oil was chromatographed on a spinning plate (2% acetone/ethyl acetate) to give 0.100 g of product (82%). The HCl salt has m.p. 239—241°C.

Example 4

Preparation of 2-[N'-(1,3,4,6,7,12bα-Hexahydro-2H-benzo[b]furo[2,3-a]quinolizin-2β-yl)-N'-methylaminosulfonyl]N,N-dimethylacetamide hydrochloride hemihydrate

Dimethylamine hydrochloride (0.222 g, 2.72 mmole) was slurried in 20 mL of dry benzene and cooled to 0°C. To this was added 1.36 mL (2.72 mmol) of trimethyl aluminum in toluene. After stirring at room temperature for 1.5 hours, 0.275 g (0.68 mmol) of ethyl 2-([N-(1,3,4,6,7,12bα-hexahydro-2H-benzo[b]furo-[2,3-a]quinolizin-2β-yl)-N-methylaminosulfonyl]acetate was added dropwise in 2 mL of benzene. After refluxing 18 hour, the reaction was cooled and 1*N* HCl was added until gas evolution ceased. The mixture was made alkaline with solid $Na_2CO_3$, filtered through a filter pad, and washed with 25 ml of ethyl acetate. The layers were separated and the aqueous layer was extracted with 3 × 10 mL of ethyl acetate. The organic fractions were combined, washed with water and saturated sodium chloride, dried ($Na_2SO_4$) and evaporated to dryness. Medium pressure column chromatography over silica gel, eluting with ethyl acetate gave 0.06 g (0.15 mmol) of starting ester. Continued elution with 5% (v/v) $CH_3OH/CHCl_3$ afforded 0.136 g (0.33 mmol) of the dimethyl acetamide in 62.2% yield based on ester consumed. This was dissolved in ether and ethanolic HCl was added dropwise to give a white solid, m.p., 151—153°C (acetone/hexane).

Example 5

N-(1,3,4,6,7,12bα-Hexahydro-2H-benzo[b]furo[2,3-a]quinolizin-2β-yl)-N-methyl-N'-ethyl urea.HCl.$H_2O$

0.100 Grams (0.39 mmol) of N-(1,3,4,6,7,12bα-hexahydro-2H-benzo[b]furo[2,3-a]quinolizin-2β-yl)-N-methylamine was dissolved in 3 mL of ethanol and 0.5 mL of ethyl isocyanate. The reactants were heated to 60°C for 10 minutes and then evaporated to dryness to give 0.110 g of product. The hydrochloride salt monohydrate melts at m.p. 199—202°C.

Employing the procedure substantially as described in Example 5 but substituting for the amine and the ethyl isocyanate used therein, the amines and isocyanates described in Table IV there are produced the ureas, also described in Table IV, by the following reaction:

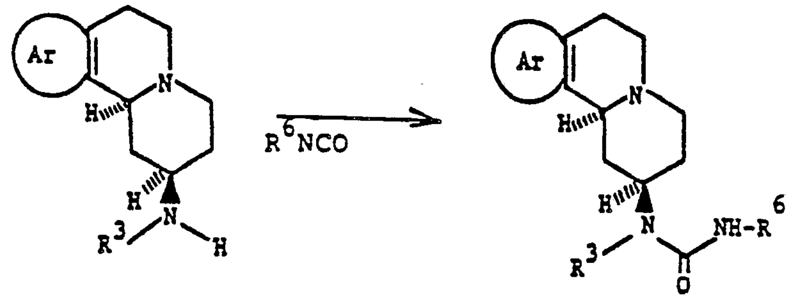


TABLE IV

| 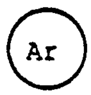 | $R^3$ | $R^6$ |
|---|---|---|
| benzo[b]furo- | $-CH_3$ | t-$C_4H_9$- |
| 11-methoxybenzo[b]furo- | $-C_2H_5$ | $CH_3$- |
| benzo[b]thieno- | $-CH_3$ | *iso*-$C_2H_7$- |
| 11-chlorobenzo[b]furo- | $-CH_3$ | $C_2H_5$- |

Example 6

N-(1,3,4,6,7,12bα-Hexahydro-2H-benzo[b]furo[2,3-a]quinolizin-2β-yl)-N-methyl-N'-ethylthiourea.HCl.0.5 $H_2O$

0.100 Grams (0.39 mmol) of amine from Example 1, Step F is dissolved in 3 mL of dimethoxyethane and 0.5 mL of ethyl isothiocyanate. After 20 minutes, the reaction is evaporated to give the product (0.110 g). The hydrochloride salt hemihydrate melts at 199—201°C.

Employing the procedure substantially as described in Example 6 but substituting for the amine and the ethylisothiocyanate used therein, the amines and isocyanates described in Table V there are produced the thioureas, also described in Table V, by the following reaction:

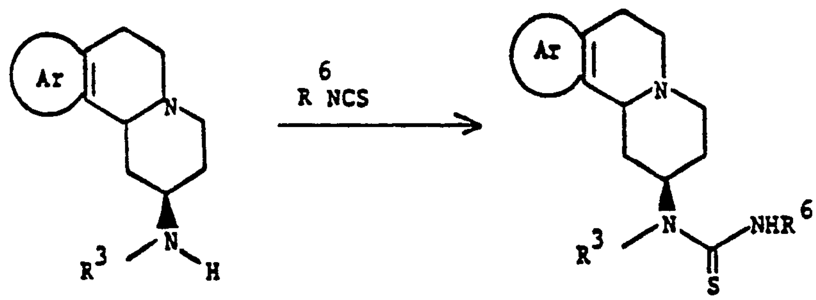



TABLE V

| Ar | $R^3$ | $R^6$ |
|---|---|---|
| benzo[b]furo- | $CH_3$- | t-$C_4H_9$- |
| benzo[b]furo- | n-$C_3H_7$- | $C_2H_5$- |
| benzo[b]thieno- | $CH_3$- | $CH_3$- |
| 10-chlorobenzo[b]thieno- | $C_2H_5O_2CCH_2$- | *iso*-$C_3H_7$- |
| furo- | $(CH_3)_2NCOCH_2$- | $CH_3$- |

Example 7

N-(1,3,4,6,7,12bα-Hexahydro-2H-benzo[b]furo[2,3-a]quinolizin-2β-yl)-2-methylpropane sulfonamide.HCl

Step A: Preparation of N-(1,3,4,6,7,12bα-hexahydro-2H-benzo[b]furo[2,3-a]quinolizin-2-yl)-amine

To a solution of 1,3,4,6,7,12bα-hexahydrobenzo[b]furo[2,3-a]quinolizin-2-one (0.10 g, 0.41 mmol) in 5 mL of methanol was added 0.224 g (2.9 mmol) of ammonium acetate and 0.027 g (0.41 mmol) of sodium cyanoborohydride. The reaction was stirred at 25°C for 24 hours; the methanol was evaporated; the residue was stirred in 6N HCl for 30 minutes, diluted with 30 mL of water and extracted with methylene chloride. The aqueous layer was made basic and extracted with methylene chloride and the extract was dried ($Na_2SO_4$), filtered and concentrated to 0.065 g of product as a 68/28 ratio of β/α amines.

Step B: Preparation of N-(1,3,4,6,7,12bα-hexahydro-2H-benzo[b]furo[2,3-a]quinolizin-2β-yl)-2-methylpropane sulfonamide

To 0.080 g (0.33 mmol) of the above amine mixture in 3 mL of methylene chloride was added 0.050 g (0.50 mmol) of triethylamine and 0.078 g (0.50 mmol) of isobutylsulfonylchloride. The mixture was stirred 2 hours, poured into 5% NaOH and washed with methylene chloride which was dried ($Na_2SO_4$), filtered and concentrated to an oil. Chromatography gave the pure β-isomer which after concentration of the rich fractions was taken up in a minimum of ethanol, treated with ethanolic HCl, and ether was added to incipient cloudiness. After crystallization was complete there was collected 0.047 g of product with m.p. 266—269°C.

Employing the procedure substantially as described in Example 7, but substituting for the quinolizine-2-one, ammonium acetate and sulfonyl chloride used therein, the Ar[2,3-a]quinolizin-2-ones, $R^3$-ammonium acetates and $R^4$-sulfonyl chlorides described in Table VI, there are prepared the N-(Ar[2,3-a]quinolizin-2β-yl)amines, also described in Table VI by the following reaction:

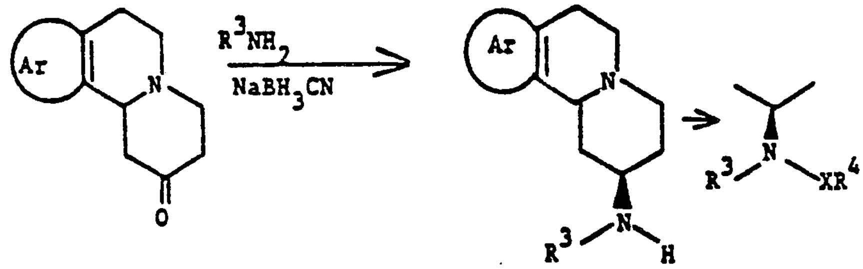


TABLE VI

| 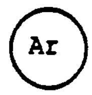 | $R^3$ | $XR^4$ |
|---|---|---|
| benzo[b]furo- | $HOCH_2CH_2$- | $-SO_2CH_2CH(CH_3)_2$ |
| benzo[b]furo- | $H_2N\overset{O}{\overset{\|}{C}}-CH_2-$ | $-SO_2(CH_2)_3CH_3$ |
| 11-chlorobenzo-[b]furo- | $CH_3\overset{O}{\overset{\|}{C}}NH(CH_2)_2-$ | $-SO_2(CH_2)_2CH_3$ |
| 10,11-dimethyl-benzo[b]furo- | $HOOCCH_2$- | $-SO_2CH_2CH_3$ |
| benzo[b]thieno- | $(CH_3)_2N(CH_2)_2$- | $-SO_2CH_2CH(CH_3)_2$ |

Example 8

N-Acetyl-N-(1,3,4,6,7,12bα-hexahydro-2H-benzo[b]furo[2,3-a]quinolizin-2β-yl)-2-methylpropane sulfonamide

To a solution of N-(1,3,4,6,7,12bα-hexahydro-2H-benzo[b]furo[2,3-a]quinolizin-2β-yl)-2-methylpropane sulfonamide (362 mg; 1.0 mmol) in 20 mL of dry THF is added NaH (26 mg; 1.1 mmol). After the reaction is stirred 30 minutes at room temperature, a solution of acetyl chloride (86 mg; 1.1 mmol) in 5 mL of dry THF is added dropwise at 0°C. The reaction is warmed to room temperature and evaporated to dryness. The residue is extracted with ethyl acetate to which is then added ethanolic HCl, causing the hydrochloride salt of the product to crystallize.

By substituting for the acetyl chloride used in Example 8, approximately equimolar amounts of methyl formate, butanoyl chloride and heptanoyl chloride, there are produced, respectively the corresponding: N-formyl, N-butanoyl and N-heptanoyl-N-(1,3,4,6,7,12bα-hexahydro-2H-benzo[b]furo[2,3-a]quinolizin-2β-yl)-2-methylpropane sulfonamide.

Example 9

N-(1,3,4,6,7,12bα-Hexahydro-2H-benzo[b]furo[2,3-a]quinolizin-2β-yl)-1,3-propanesultam

Step A: Preparation of N-(1,3,4,6,7,12bα-hexahydro-2H-benzo[b]furo[2,3-a]quinolizin-2β-yl)-3-chloropropanesulfonamide.HCl

To 0.300 g (1.24 mmol) of N-(1,3,4,6,7,12bα-hexahydro-2H-benzo[b]furo[2,3-a]quinolizin-2β- and 2α-yl)-amine dissolved in 3 ml of methylene chloride and cooled to 0°C was added 0.150 g (1.49 mmol) of triethylamine and 0.242 g (1.37 mmol) of 3-chloropropanesulfonylchloride. The reaction was stirred 3 hours at 25°C, poured into 5% NaOH and washed with methylene chloride which was dried ($Na_2SO_4$), filtered and concentrated. The oil obtained was chromatographed (silica/10% MeOH/$CHCl_3$) giving 0.100 g of pure β-isomer.

Step B: Preparation of N-(1,3,4,6,7,12bα-hexahydro-2H-benzo[b]furo[2,3-a]quinolizin-2β-yl)-1,3-propane-sultam.HCl

The above sulfonamide (0.092 g, 0.24 mmol) was dissolved in 3 mL of dimethoxyethane (DME) and 0.055 g (0.48 mmol) of potassium *t*-butoxide was added. The reactants were heated at 60°C for 2 hours and then poured into 5% NaOH and extracted with ether. The ether was washed with brine, dried ($MgSO_4$), filtered and concentrated to yield a crude product. Chromatography (silica; 5% MeOH/$CHCl_3$) gave 0.062 g of product. The product was taken up in ethanol, treated with ethanolic HCl and ether to incipient cloudiness. When crystallization was complete the product was collected and dried, m.p 207—210°C (dec).

Example 10

N-(1,3,4,6,7,12bα-Hexahydro-2H-benzo[b]furo[2,3-a]quinolizin-2β-yl)oxazolin-2-one hydrochloride 0.25 hydrate

Step A: Preparation of N-(1,3,4,6,7,12bα-Hexahydro-2H-benzo[b]furo[2,3-a]quinolizin-2-β-yl)-N-(2-hydroxyethyl)amine

Ethanolamine (0.366 g, 6 mmol) and 0.241 g (1 mmol) of 1,3,4,6,7,12bα-hexahydro-2H-benzo[b]furo-[2,3-a]quinolizin-2-one were dissolved in 20 mL dry methanol, and ethanolic HCl added until the pH was 6.5. To this was added 0.038 g (0.6 mmol) of sodium cyanoborohydride and 3A molecular sieves. After stirring 18 hours, $NH_3$ saturated $CHCl_3$ was added until basic, and the solvent removed *in vacuo.* The residue was stirred in ethyl acetate, filtered and the solvent removed *in vacuo.* Purification by spinning disc chromatography (silica; $NH_3$ saturated $CHCl_3$) afforded 0.063 g (36%) of α-isomer and 0.112 g (64%) of β-isomer in 61% overall yield. The desired β-isomer was recrystallized from ether/pet. ether to yield white needles with m.p. 131—132°C.

Step B: Preparation of N-(1,3,4,6,7,12bα-hexahydro-2H-benzo[b]furo[2,3-a]quinolizin-2-β-yl)oxazolin-2-one hydrochloride 0.25 hydrate

N - (1,3,4,6,7,12bα - Hexahydro - 2H - benzo[b]furo[2,3-a]-quinolizin - 2-β - yl) - N - (2 - hydroxyethyl)amine (0.71 g, .25 mmol) was placed in 5 ml dry toluene, and enough THF was added to make it homogeneous. To this was added dropwise 0.205 g (1.25 mmol) of carbonyl diimidazole in 2 ml dry toluene, and the reaction was refluxed 18 hours, after which time it was cooled and the solvent evaporated. Purification by spinning disc chromatography (silica; 5% (v/v) MeOH/$CHCl_3$) gave 0.062 g (0.2 mmol) of N-(1,3,4,6,7,12bα-Hexahydro-2H-benzo[b]furo[2,3-a]quinolizin-2-β-yl)oxazolin-2-one in 79% yield. This was dissolved in ethyl acetate and ethanolic HCl was added dropwise to yield the hydrochloride 0.25 hydrate salt as a yellow solid, with m.p. 230°C (dec).

Example 11

N-(1,3,4,6,7,12bα-Hexahydro-2H-benzo[b]furo[2,3-a]quinolizin-2β-yl)succinimide.

A mixture of (1,3,4,6,7,12bα-Hexahydro-2H-benzo[b]furo[2,3-a]quinolizin-2β-yl)amine (80 mg; 0.33 mmol) and succinic anhydride (33 mg; 0.33 mmol) is heated under nitrogen at 130°C for 2 hours. The residue is extracted into ethyl acetate which is washed with saturated $NaHCO_3$ solution. The organic phase is dried ($Na_2SO_4$), filtered and acidified with ethanolic HCl, causing the hydrochloric salt of the product to crystallize.

Employing maleic anhydride and glutaric anhydride in place of the succinic anhydride in Example 11, affords; respectively N-(1,3,4,6,7,12bα-hexahydro-2H-benzo[b]furo[2,3-a]quinolizin-2β-yl)maleimide; and N-(1,3,4,6,7,12bα-Hexahydro-2H-benzo[b]furo[2,3-a]quinolizin-2β-yl)glutarimide.

Example 12

N-(1,3,4,6,7,10bα-Hexahydro-2H-thiazolo[4,5-a]quinolizin-2-β-yl)-N,N′,N′-trimethylsulfamide, hydrochloride

Step A: Preparation of Ethyl 4-oxo-pipecolinate, ethylene ketal.

A solution of ethyl 4-oxo-pipecolinate (17.1 g; 0.1 mole), ethylene glycol (6.8 g; 0.11 mole), and p-toluenesulfonic acid (0.5 g) in 250 mL of dry benzene is refluxed under Dean-Stark conditions for 18 hours. The benzene solution is washed with saturated aqueous $NaHCO_3$ solution, dried ($Na_2SO_4$), and evaporated to afford the desired product.

Step B: Preparation of Ethyl N-(3-Ethoxycarbonylpropyl)-4-oxopipecolinate, ethylene ketal.

A mixture of ethyl 4-oxopipecolinate, ethylene ketal (12.9 g; 60 mmol), ethyl 4-bromobutyrate (12.9 g; 66 mmol), and $K_2CO_3$ (12.0 g; 86 mmol) in 250 mL of toluene is heated at 80°C for 4 hours. The solid is then filtered off, and the filtrate is concentrated. Distillation of the residue affords the product.

Step C: Preparation of 1,3,4,6,7,8,9,9a-Octahydro-2H-quinolizin-1,8-dione-2-carboxylic acid, ethyl ester, 8-ethylene ketal.

Ethyl N-(3-ethoxycarbonylpropyl)-4-oxopipecolinate, ethylene ketal (3.29 g; 10 mmol) is added to a stirred suspension of NaH (0.58 g of a 50% dispersion in oil; 12 mmol) in 5 mL of dry toluene. The reaction is then refluxed for 2 hours. Water is added, followed by acetic acid until the reaction is neutral. The organic fraction is separated, dried ($Na_2SO_4$), and concentrated to yield the crude product.

Step D: Preparation of 1,3,4,6,7,8,9,9a-Octahydro-2H-quinolizin-1,8-dione, 8-ethylene ketal.

A mixture of 1,3,4,6,7,8,9,9a-octahydro-2H-quinolizin-1,8-dione-2-carboxylic acid, ethyl ester, 8-ethylene ketal (2.83 g; 10 mmol), LiCl (0.84 g; 20 mmol), and water (0.36 g; 20 mmol) in 25 mL of DMSO is heated to 180°C for 2 hours. After the reaction is cooled to room temperature, it is partitioned between ethyl acetate and water. The organic extracts are separated, dried ($Na_2SO_4$), and concentrated. The residue is chromatographed over silica gel to give the product.

Step E: Preparation of 2-Bromo-1,3,4,6,7,8,9,9a-Octahydro-2H-quinolizin-1,8-dione, 8-ethylene ketal.

To a solution of 1,3,4,6,7,8,9,9a-octahydro-2H-quinolizin-1,8-dione-1,8-dione, 8-ethylene ketal (2.1 g; 10 mmol) in 20 mL of dry methylene chloride is added dropwise a solution of benzyltrimethylammonium bromide perbromide (3.9 g; 10 mmol) in 10 mL of methylene chloride at 0°C. After 2 hours, the reaction mixture is washed three times with water. The organic phase is dried ($Na_2SO_4$) and concentrated *in vacuo* to yield the crude product which is used without further purification.

Step F: Preparation of 1,3,4,6,7,10b-Hexahydro-2H-thiazolo[4,5-a]quinolizin-2-one, ethylene ketal.

A solution of 2-bromo-1,3,4,6,7,8,9,9a-octahydro-2H-quinolizin-1,8-dione, 8-ethylene ketal (4.3 g; 15 mmol) and thioformamide (1.8 g; 30 mmol) in 10 mL of DMF is stirred with 2.0 g of 3A-molecular sieves at 50°C for 8 hours. After the solid is filtered, the filtrate is poured into water, causing the product to crystallize.

Step G: Preparation of 1,3,4,6,7,10b-Hexahydro-2H-thiazolo[4,5-a]quinolizin-2-one

1,3,4,6,7,10b-Hexahydro-2H-thiazolo[4,5-a]quinolizin-2-one, ethylene ketal (1.0 g) is dissolved in 25 mL of acetone. 6N HCl (2.0 mL) is added, and the reaction is stirred at room temperature for 4 hours. The acetone is removed *in vacuo*, and the aqueous fraction is made basic with $K_2CO_3$. This mixture is extracted with methylene chloride (3 × 10 mL). The organic extracts are then dried ($Na_2SO_4$) and concentrated to afford the product.

Following the procedures substantially as described in Example 1, Steps F and G but substituting for the quinolizin-2-one used therein, an equimolar amount of quinolizin-2-one from Step G of this Example 11, there are produced in sequence:

N-(1,3,4,6,7,10bα-hexahydro-2H-thiazolo[4,5-a]quinolizin-2β-yl)-N-methylamine; and

N-(1,3,4,6,7,10bα-hexahydro-2H-thiazolo[4,5-a]quinolizin-2β-yl)-N,N',N'-trimethylsulfamide, hydrochloride.

Similarly prepared are those compounds wherein Ar is thiazolo and —X—$R^4$ are —$SO_2CH_2CH_2OH$, —$SO_2CH_2CH_3$, and —$SO_2(CH_2)_3OH$.

Example 13

N-(1,3,4,6,7,10bα-Hexahydro-2H-imidazolo[4,5-a]quinolizin-2β-yl)-N,N',N'-trimethylsulfamide, hydrochloride

Step A: Preparation of 1,3,4,6,7,10b-hexahydro-2H-imidazo[4,5-a]quinolizin-2-one, ethylene ketal.

Formamidine acetate (3.1 g; 30 mmol) is added to a solution of 2-bromo-1,3,4,6,7,8,9,9a-octahydro-2H-quinolizin-1,8-dione, 8-ethylene ketal (4.3 g; 15 mmol) in 15 mL of ethanol. The reaction is refluxed for 3 hours and then concentrated. The residue is partitioned between ethyl acetate and saturated $NaHCO_3$. The organic fraction is dried ($Na_2SO_4$) and evaporated to yield the desired product, after chromatography over silica gel.

Step B: Preparation of 1,3,4,7,10b-Hexahydro-2H-imidazo[4,5-a]quinolizin-2-one.

1,3,4,6,7,10b-Hexahydro-2H-imidazo[4,5-a]quinolizin-2-one, ethylene ketal (2.0 g) is dissolved in 25 mL of a mixture of acetone-6N HCl (10:1) and stirred at room temperature for 6 hours. The acetone is removed *in vacuo*, and the aqueous fraction is made basic with $K_2CO_3$. This mixture is extracted with methylene chloride. The organic extracts are then dried ($Na_2SO_4$) and concentrated to afford the product.

Following the procedures substantially as described in Example 1, Steps F and G but substituting for the quinolizin-2-one used therein, an equimolar amount of the quinolizin-2-one from Step B of this Example 13 there are produced in sequence:

N-(1,3,4,6,7,10bα-hexahydro-2H-imidazolo[4,5-a]quinolizin-2β-yl)-N-methylamine; and

N-(1,3,4,6,7,10bα-hexahydro-2H-imidazolo[4,5-a]quinolizin-2β-yl)-N,N',N'-trimethylsulfamide, hydrochloride.

Similarly prepared are those compounds wherein Ar is imidazo and —X—$R^4$ are —$SO_2(CH_2)_2OH$—, $SO_2(CH_2)_3OH$, and $SO_2CH_2CH_3$.

Example 14

N-(1,3,4,6,7,10bα-Hexahydro-2H-pyrazolo[3,4-a]quinolizin-2β-yl)-N,N',N'-trimethylsulfamide, hydrochloride

Step A: Preparation of 1,3,4,6,7,10-Hexahydro-2H-pyrazolo[3,4-a]quinolizin-2-one, ethylene ketal.

1,3,4,6,7,8,9,9a-Octahydro-2H-quinolizin-1,8-dione, 8-ethylene ketal (4.2 g; 20 mmol) and DMF-dimethyl acetal (2.86 g; 24 mmol) are heated at 100°C under nitrogen for 16 hours. The dark residue is then dissolved in 5 mL of ethanol and treated with anhydrous hydrazine (1.28 g; 40 mmol). The reaction is stirred

at room temperature for 18 hours. The solvent is evaporated, and the residue is chromatographed over silica gel, eluting with 5% $MeOH/CHCl_3$ saturated with ammonia to yield the product.

Step B: Preparation of 1,2,3,4,6,7,10-Hexahydro-2H-pyrazolo[3,4-a]quinolizin-2-one

1,2,3,4,6,7,10-Hexahydro-2H-pyrazolo[3,4-a]quinolizin-2-one, ethylene ketal (1.0 g) is dissolved in 25 mL of a mixture of acetone-6N HCl (10:1) and stirred at room temperature for 5 hours. The acetone is removed *in vacuo*, and the aqueous fraction is made basic with $K_2CO_3$. This mixture is extracted with methylene chloride. The organic extracts are then dried ($Na_2SO_4$) and concentrated to afford the product.

Following the procedure substantially as described in Example 1, Steps F and G but substituting for the quinolizin-2-one used therein, an equimolecular amount of the quinolizin-2-one from Step B of this Example 14, there are produced in sequence:

N-(1,3,4,6,7,10bα-hexahydro-2H-pyrazolo[3,4-a]quinolizin-2β-yl)-N-methylamine; and

N-(1,3,4,6,7,10bα-hexahydro-2H-pyrazolo[3,4-a]quinolizin-2β-yl)-N,N',N'-trimethylsulfonamide.

Similarly prepared are those compounds wherein Ar is pyrazolo and $—XR^4$ are $—SO_2(CH_2)_2OH$, $—SO_2(CH_2)_3OH$ and $—SO_2CH_2CH_3$.

Example 15

N-(1,3,4,6,7,11bα-Hexahydro-2H-pyrido[2,3-a]quinolizin-2β-yl)-N,N',N'-trimethylsulfamide, hydrochloride

Step A: Preparation of 2-(2-(1,3-Dioxolan-2-yl)ethyl)-1,3,4,6,7,8,9,9a-Octahydro-2H-quinolizin-1,8-dione-2-carboxylic acid, ethyl ester, 8-ethylene ketal.

1,3,4,6,7,8,9,9a-Octahydro-2H-quinolizin-1,8-dione-2-carboxylic acid, ethyl ester, 8-ethylene ketal (5.7 g; 20 mmol) is added in small portions to a stirred suspension of NaH (0.48 g; 20 mmol) in 50 mL of toluene/DMF (1:1). After 15 minutes 2-(2-bromoethyl)-1,3-dioxolane is added in one portion, and the reaction is refluxed for 4 hours. The mixture is cooled and partitioned between water and ethyl acetate. The organic layer is washed well with water, dried ($Na_2SO_4$), and concentrated. The residue is chromatographed over silica gel to afford the product.

Step B: Preparation of 2-(2-(1,3-Dioxolan-2-yl)ethyl)-1,3,4,6,7,8,9,9a-octahydro-2H-quinolizin-1,8-dione, 8-ethylene ketal.

A mixture of 2-(2-(1,3-dioxolan-2-yl)ethyl-1,3,4,6,7,8,9,9a-octahydro-2H-quinolizin-1,8-dione-2-carboxylic acid, ethyl ester, 8-ethylene ketal (1.9 g; 5 mmol), LiCl (0.42 g; 10 mmol), water (0.18 g; 10 mmol), and 20 mL DMSO is heated at 180°C for 2 hours. The reaction is then poured into 100 mL of water and extracted with ethyl acetate. The organic fraction is washed well with water, dried ($Na_2SO_4$), and concentrated. The residue is chromatographed over silica gel to yield the desired product.

Step C: Preparation of 1,3,4,6,7,11b-Hexahydro-2H-pyrido[2,3-a]quinolizin-2-one, ethylene ketal.

A solution of 2-(2-(1,3-dioxolan-2-yl)ethyl)-1,3,4,6,7,8,9,9a-octahydro-2H-quinolizin-1,8-dione, 8-ethylene ketal (1.5 g; 5 mmol) and hydroxylamine hydrochloride (0.7 g; 10 mmol) in 25 mL absolute ethanol is refluxed for 2 hours. The solvent is evaporated, and the residue is chromatographed over silica gel to give the product.

Step D: Preparation of 1,3,4,6,7,11b-Hexahydro-2H-pyrido[2,3-a]quinolizin-2-one.

A solution of 1,3,4,6,7,11b-hexahydro-2H-pyrido[2,3-a]quinolizin-2-one, ethylene ketal (2.0 g) in 50 mL of acetone/6N HCl (10:1) is stirred at room temperature for 3 hours. The acetone is removed *in vacuo*, and the aqueous fraction is made basic with $K_2CO_3$. The resulting mixture is extracted with methylene chloride, which is then dried ($Na_2SO_4$) and concentrated to afford the product.

Employing the procedures substantially as described in Example 1, Steps F and G but substituting for the quinolizin-2-one used therein, an equimolar amount of the quinolizin-2-one from Step D of this Example 15, there are produced in sequence:

N-(1,3,4,6,7,11bα-hexahydro-2H-pyrido[2,3-a]quinolizin-2β-yl)-N-methylamine; and

N-(1,3,4,6,7,11bα-hexahydro-2H-pyrido[2,3-a]quinolizin-2β-yl)-N,N',N'-trimethylsulfamide, hydrochloride.

Similarly prepared are those compounds wherein Ar is pyrido and $XR^4$ is $—SO_2(CH_2)_2OH$, $—SO_2(CH_2)_3OH$ and $SO_2CH_2CH_3$.

Alternate processes for preparing N-(1,3,4,6,7,12bα-hexahydro-2H-benzo[b]furo[2,3-a]quinolizin-2β-yl)-N-methyl-2-hydroxyethanesulfonamide (Compound III) and its pharmaceutically acceptable salts are shown in the Flow Sheets. The compound may be prepared by these methods as a racemic mixture or as either single enantiomer. The enantiomers are obtained by resolving the final compound or any intermediate containing a center of chirality.

Flow Sheet I illustrates the preparation of Compound III by sulfonylating the amine from Example 1 with a 2-hydroxyethanesulfonic acid derivative or with an O-protected form which is hydrolytically or hydrogenolytically labile.

Flow Sheet II shows the synthesis of Compound III via an intermediate imine which in turn is prepared from the ketone of Example 1. Sulfonylation of the imine affords the sulfonamido substituted olefin which is reduced to Compound III.

Flow Sheet III indicates that reduction of the appropriate ester, affords Compound III.

Flow Sheet IV illustrates the process whereby the secondary sulfonamide is obtained from the primary amine from Step A of Example 7. Alkylation of this intermediate yields Compound III. Alternatively, hydroxyethylation followed by reduction yields III. The reaction will also work if the precursor to be hydroxyethylated is unprotected.

Flow Sheet V outlines the homologation of a methanesulfonamide derivative to Compound III via an organometallic species.

Flow Sheet VI illustrates processes employing more complex butadiene components in the cycloaddition reaction. The resulting sulfonamido substituted dihydropyridines are then reduced directly or in a stepwise fashion to Compound III.

Flow Sheet VII depicts the synthesis of Compound III wherein construction of the 7—7a bond completes the tetracyclic skeleton. Subsequent reduction affords the product.

Flow Sheet VIII shows a synthetic route to Compound III wherein construction of the 12a—12b bond by acid catalyzed cyclization completes the tetracyclic skeleton. Subsequent reduction, directly or in steps, yields the product.

Flow Sheet IX shows the synthesis of Compound III which involves alkylating the appropriately substituted piperidine with 3-(2-haloethyl)benzofuran. Oxidation of this intermediate with $Hg(OAc)_2$ or similar oxidant to the iminium salt under controlled conditions causes cyclization to the tetracyclic skeleton of Compound III. Excess oxidant results in the formation of a tetracyclic iminium salt which is reduced to Compound III.

Flow Sheet X shows a synthesis of Compound III in which an appropriately substituted 2-piperidone is treated with base to generate amide anion. Alkylation with 3-(2-haloethyl)benzofuran yields an intermediate which can be cyclized under acidic conditions to the tetracyclic iminium salt. Reduction and deprotonation affords the product. Alternatively, the alkylated 2-piperidone may be reduced to an enamine. Acid catalyzed cyclization affords Compound III.

Flow Sheet XI illustrates a synthesis of Compound III which exploits the known rearrangement of O-phenyloximes to benzofurans. Alkylation of the appropriately substituted piperidine occurs under standard conditions. Oxidation of the amine to the corresponding N-oxide followed by $(CF_3CO)_2O$ mediated dehydration to the iminium intermediate.

Addition of cyanide affords the aminonitrile. Treatment with base and aqueous work-up produces the keto ester which can be decarboxylated under a variety of conditions. The resulting ketone is condensed with O-phenylhydroxylamine to yield the O-phenyloxime. Acid causes rearrangement and cyclization to the benzofuran of Compound III.

FLOW SHEET I

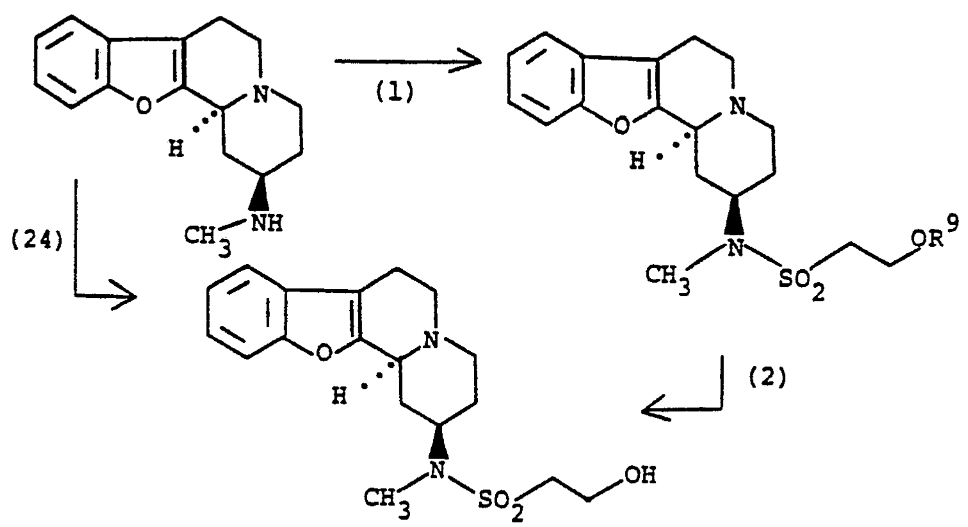


COMPOUND III

FLOW SHEET II

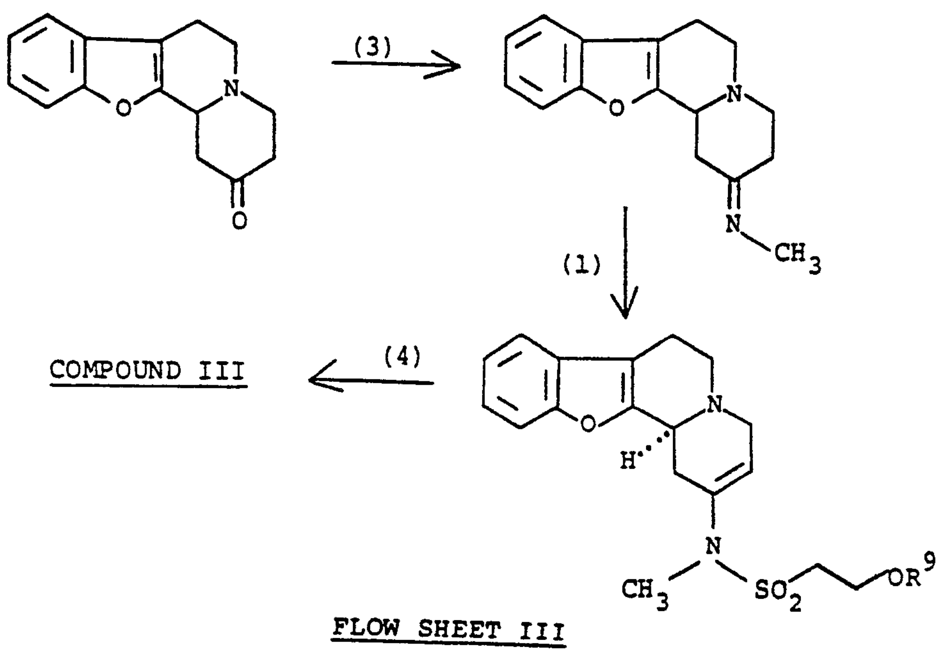


FLOW SHEET III

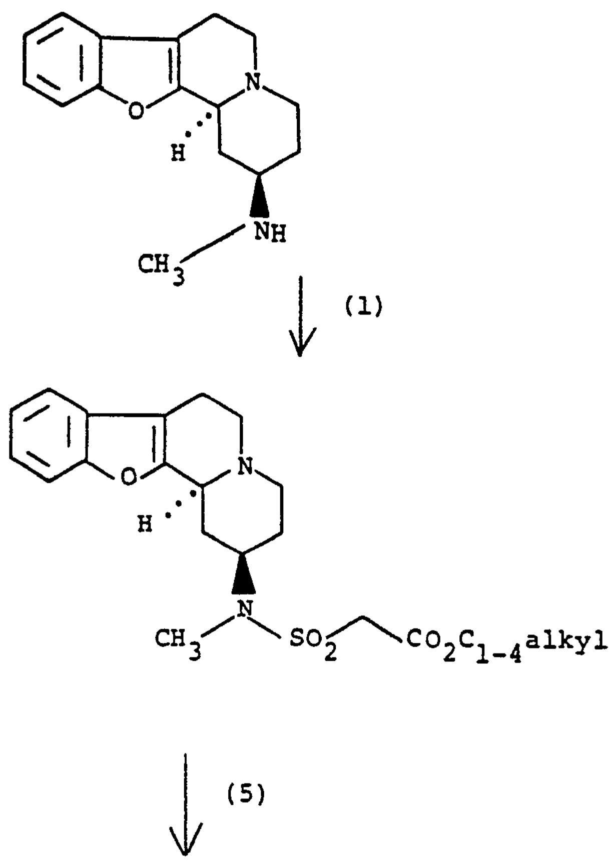


COMPOUND III

FLOW SHEET IV

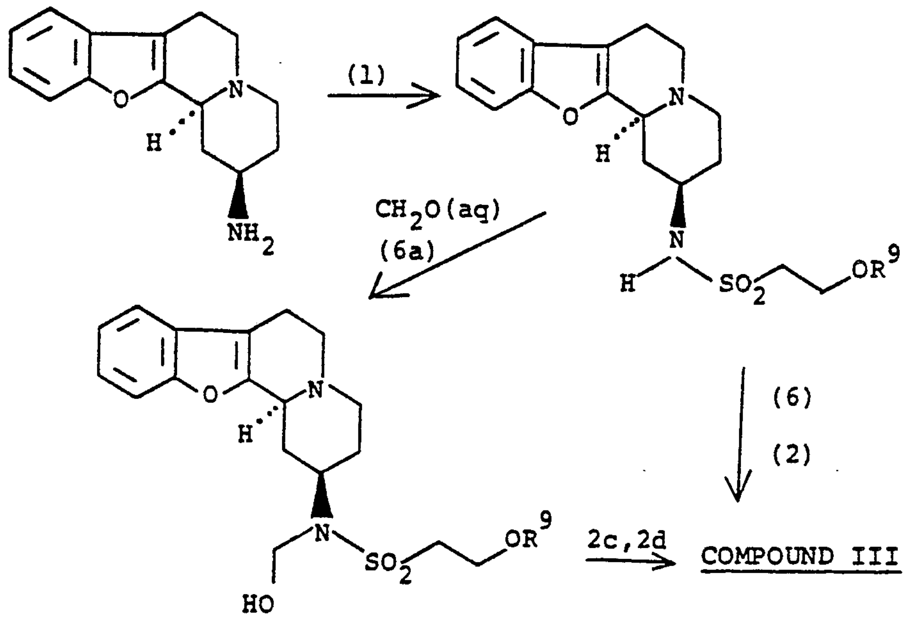


FLOW SHEET V

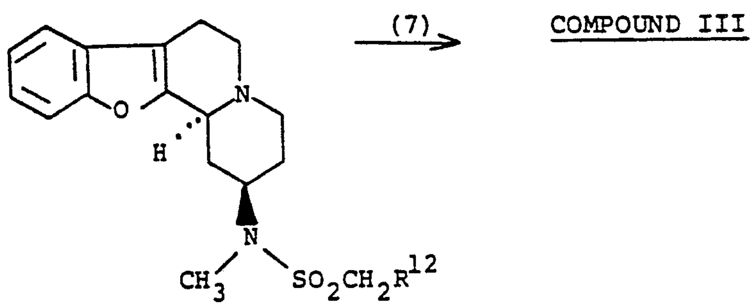


FLOW SHEET VI

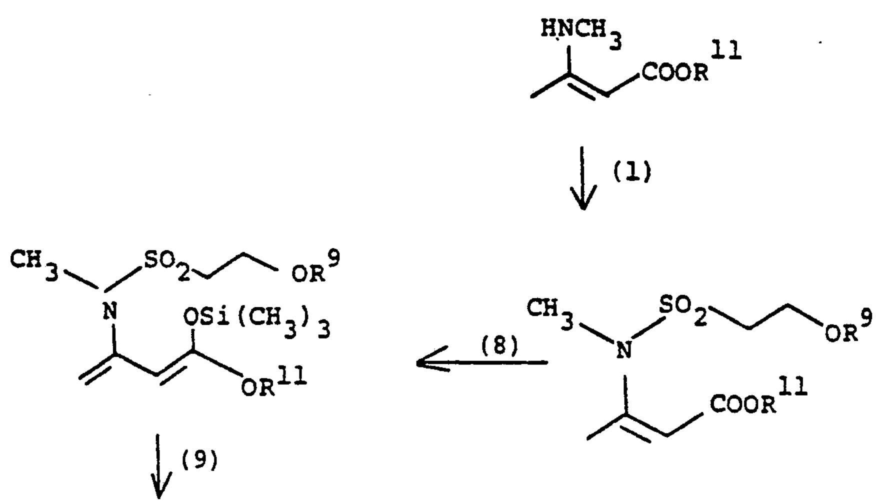

FLOW SHEET VI

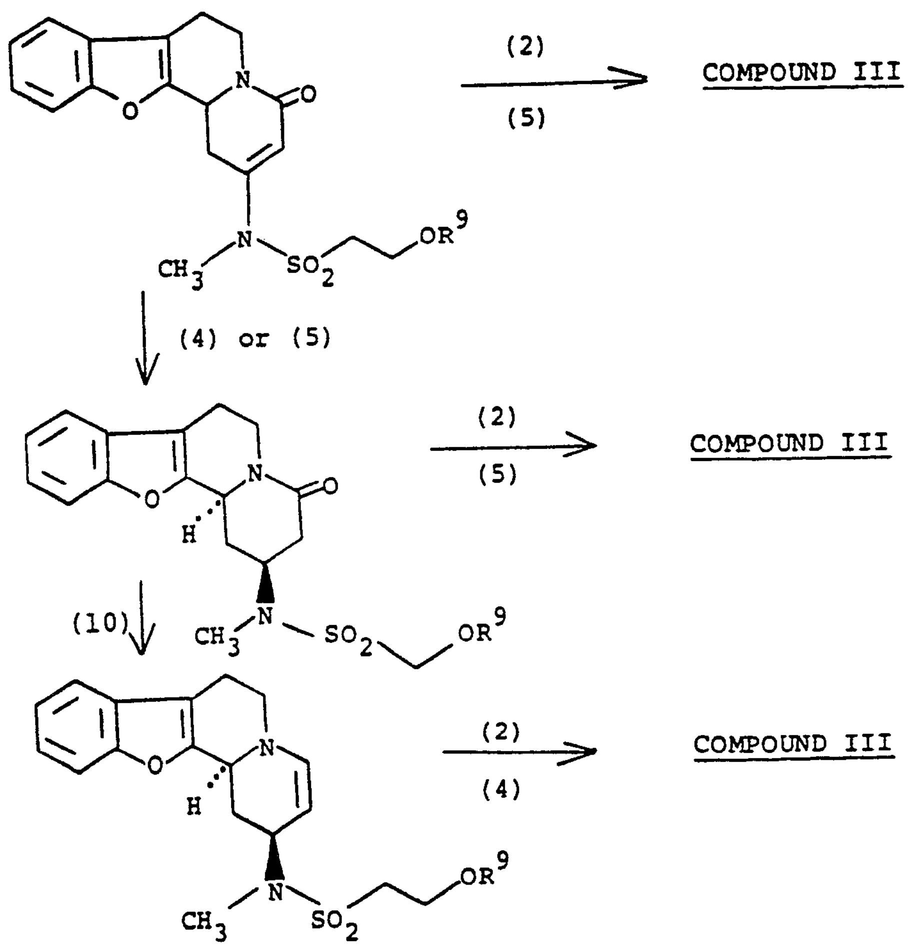


FLOW SHEET VII

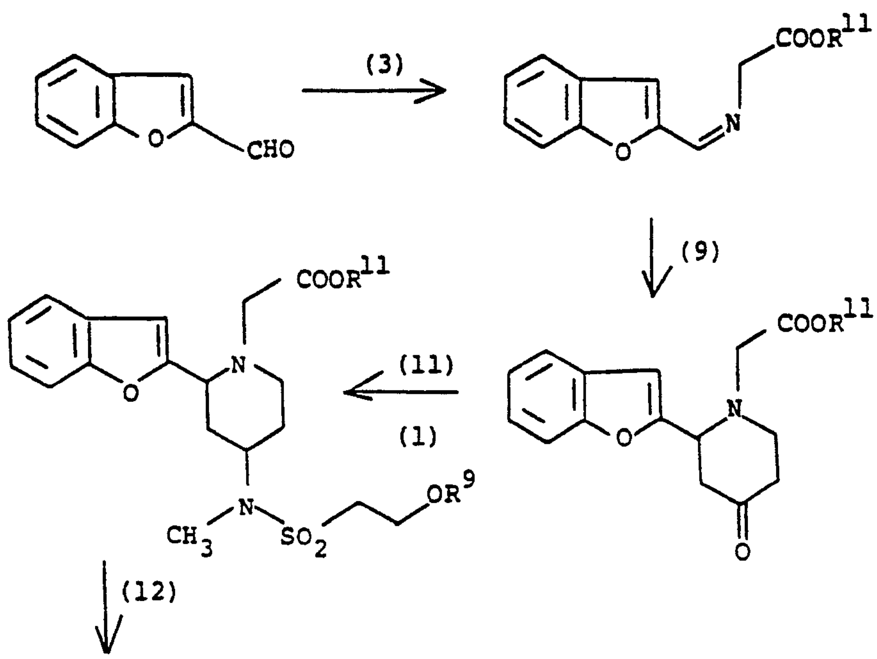

FLOW SHEET VII

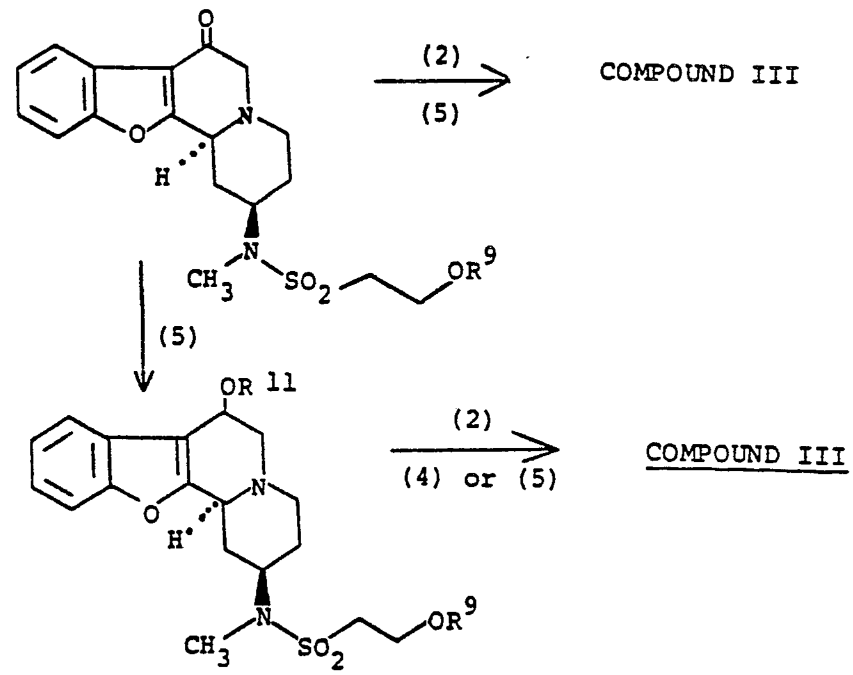


FLOW SHEET VIII

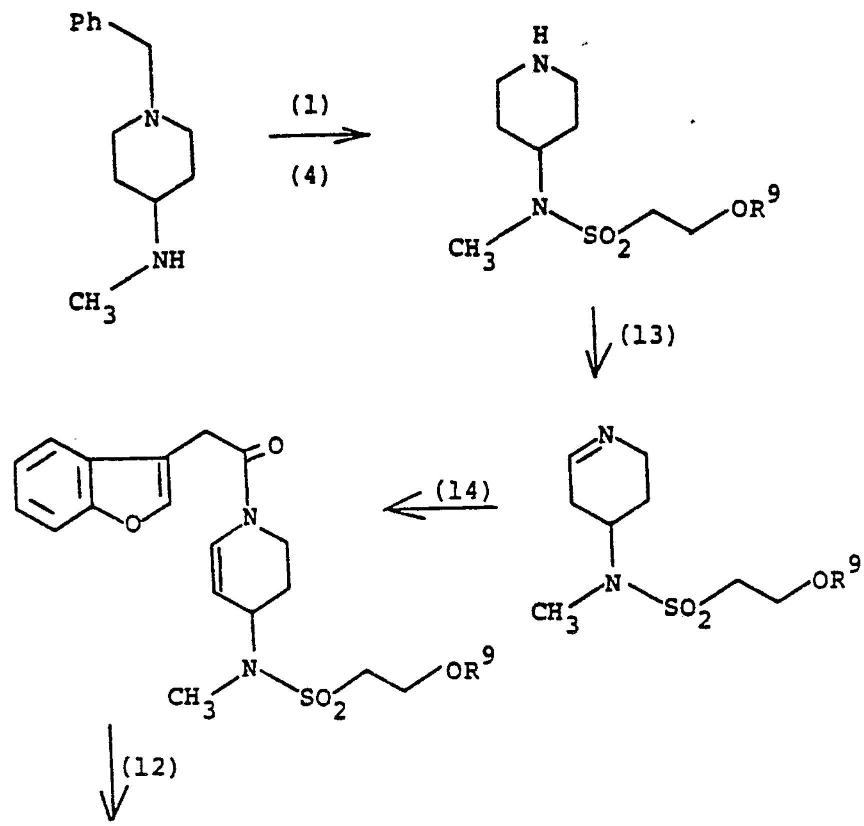

FLOW SHEET VIII

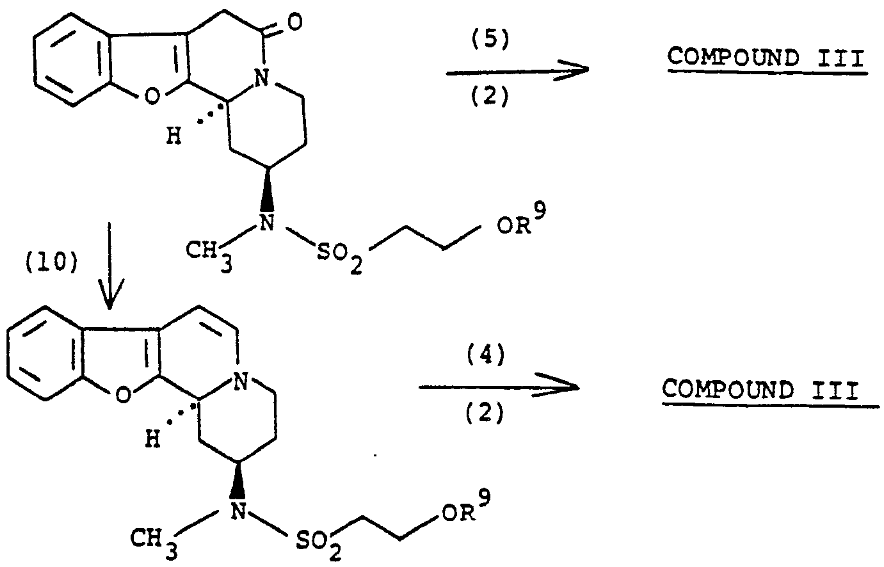


FLOW SHEET IX

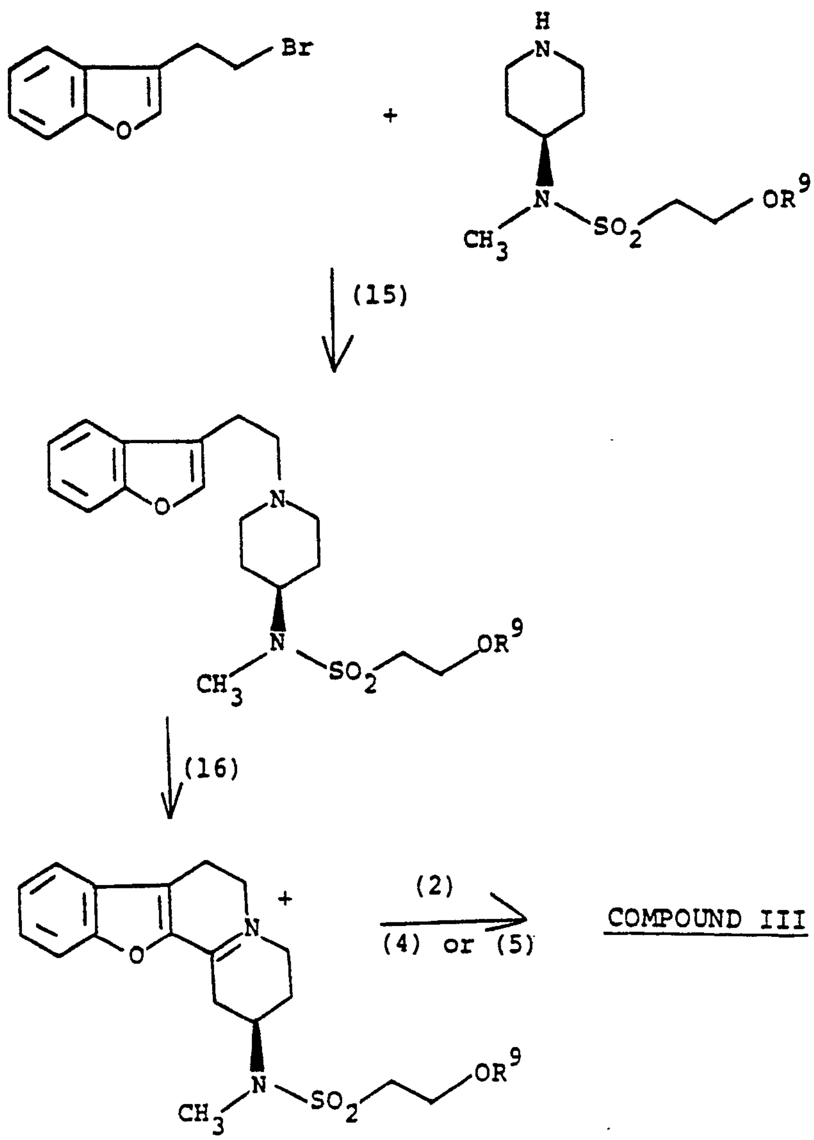

VARIATION OF FLOW SHEET IX

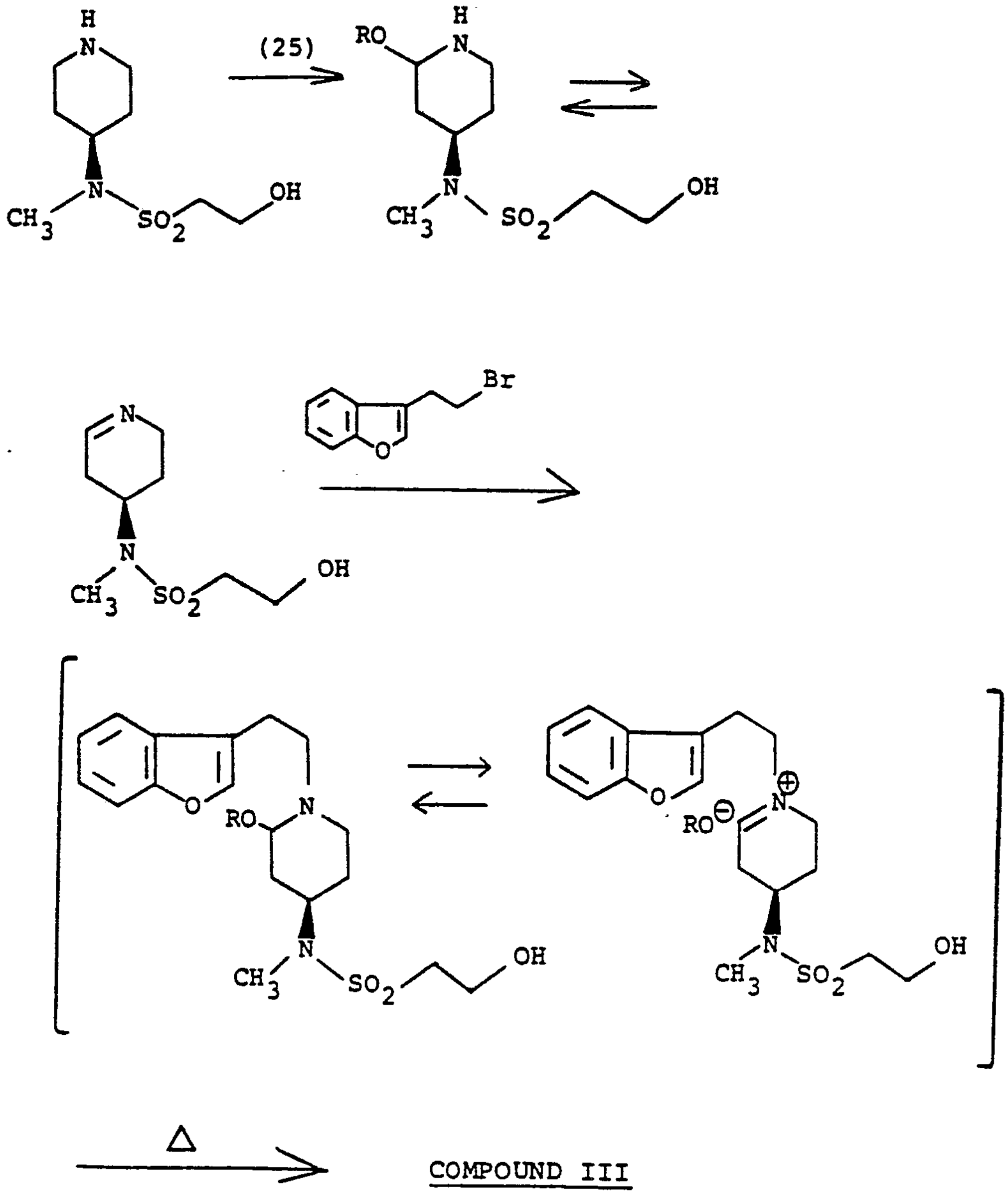


COMPOUND III

FLOW SHEET X

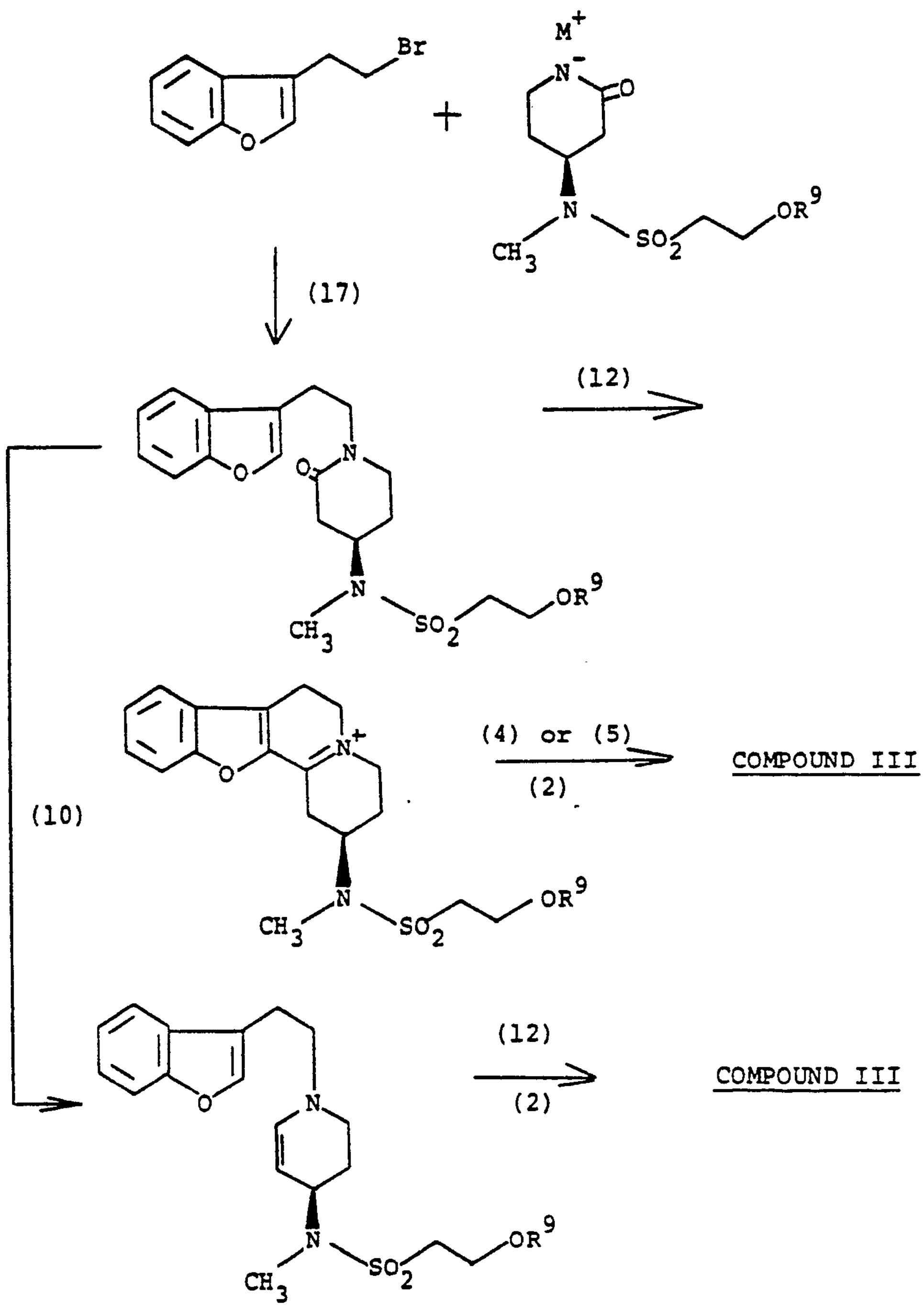

FLOW SHEET XI

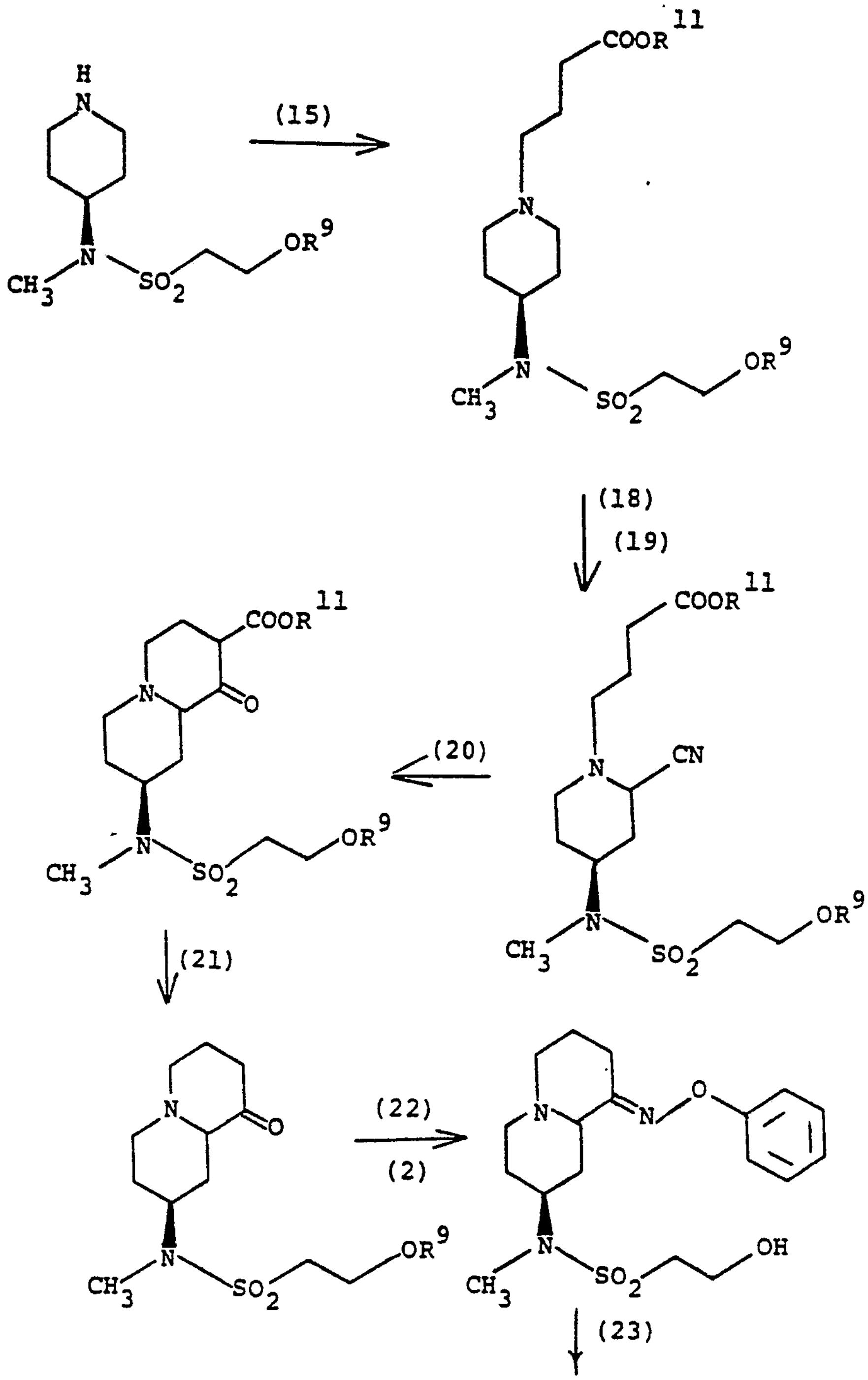


COMPOUND III

FLOW SHEET XII

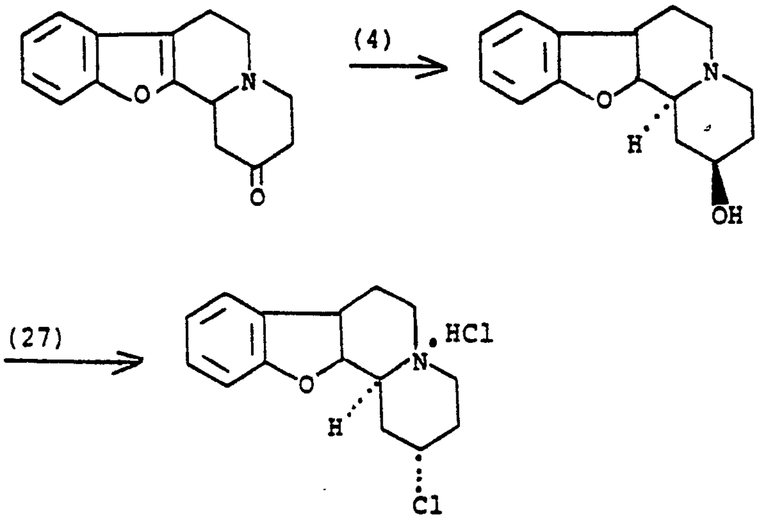


(28)

COMPOUND III

Definitions for Flow Sheets I—XI

$R^9$ is

1) Hydrogen

2)

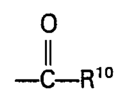

wherein $R^{10}$ is —H, $C_{1-4}$alkyl, phenyl either unsubstituted or substituted with halo, $C_{1-3}$alkyl, $C_{1-3}$alkoxy, or $N(R^8)_2$

3) $—CO_2CH_2Ar$

wherein Ar is phenyl, either unsubstituted or substituted with halo, $C_{1-3}$alkoxy, $C_{1-3}$alkyl, or $—N(R^8)_2$;

4) $—CO_2R^{10}$

5) $—Si(R^{11})_3$

wherein $R^{11}$ is $C_{1-3}$alkyl

$R^{12}$ is

1) hydrogen

2) Cl, Br, I

Reactions in Flow Sheets I—XI

1. Sulfonylation using sulfonyl halides, sulfonyl imidazoles, activated esters of sulfonic acids, or sulfonic acid anhydrides.

2. Removal of the protecting group. This can be done in several ways, depending on $R^9$:

(a) alkaline hydrolysis [1), 2), 3), 4)]

(b) acidic hydrolysis [2), 3), 4), 5)]

(c) hydrogenolysis [3)]

(d) reduction by metal hydrides

3. Treatment with the primary amine under dehydrating conditions.

4. Catalytic hydrogenation.

5. Reduction. This can be done in several ways:

(a) directly in one step, using e.g. $LiAlH_4$

(b) reduction to the aldehyde with $(i\text{-}Bu)_2AlH$ followed by further reduction of the aldehyde with additional metal hydride $[(i\text{-}Bu)_2AlH, LiAlH_4, (NaBH_4)]$.

6. Alkylation. This may be accomplished by sequential treatment with a base such as NaH followed by $CH_3I$.

6a. Hydroxyalkylation with aqueous $CH_2O$ in a water-miscible organic solvent such as THF or dioxane.

7. Homologation. This can be done in several ways:

(a) when $R^{12}$=H, treatment with a strong base such as LiN)i-Pr$)_2$ generates the carbanion. Addition of formaldehyde to this carbanion, followed by neutralization with acid affords Compound III.

(b) When $R^{12}$ = Cl, Br or I, treatment with an active metal such as Li or Mg generates the corresponding organometallic species. Addition of formaldehyde to this organometallic followed by neutralization with acid affords Compound III.

8. Silylation: Treatment with base (e.g. LiN(i-Pr$)_2$) followed by a silylating agent.

9. Cycloaddition following substantially the same procedure as Step E of Example 1.

10. Reduction with (i-Bu$)_2$AlH.

11. Reductive amination: Treatment of the ketone with an amine and $NaCNBH_3$ in MeOH at pH of 4—6.

12. Acid catalyzed cyclization with PPA, $POCl_3$, or HCl.

13. Oxidation with tert-butyl hypochlorite followed by base induced elimination.

14. Acylation with an acid chloride.

15. Alkylation of the amine under standard conditions.

16. Oxidation with Hg(OAc$)_2$.

17. Nitrogen alkylation of a metal salt of an appropriately substituted 2-piperidone.

18. Oxidation of nitrogen to the amine oxide with $CH_3CO_3H$ or m-chloroperbenzoic acid.

19. Treatment of the amine oxide with $(CF_3CO)_2O$ in the presence of KCN.

20. Treatment with NaH in toluene followed by an aqueous work-up.

21. Decarboxylation: This can be done in several ways.

(a) Saponification of the ester with alkali followed by acidification.

(b) Heat with 2,6-lutidine/KI or DABCO (1,4-diazabicyclo[2,2,2]octane).

(c) Heat in DMSO with NaCl.

22. Oxime formation using O-phenylhydroxylamine.

23. Acid catalyzed rearrangement/cyclication of O-phenyloximes to benzofurans.

24. Treatment of the amine with $SO_2$ in a common organic solvent ($CH_3CN$, THF, ethylacetate or toluene); removal of excess $SO_2$ *in vacuo*; addition of a hindered tertiary amine catalyst such as $(n\text{-}C_4H_9)_3N$, followed by an excess of ethylene oxide.

25., 26. The amine on Flow Sheet IX (R=H) is treated in a simple alcoholic solvent ($CH_3OH$, $C_2H_5OH$, i-$C_3H_7OH$, etc) with a mole of t-butylhypochlorite in the presence of a mole of the corresponding alkoxide of $Li^+$, $Na^+$, etc derived from the alcohol to give the alkoxyamine which is in equilibrium with the amine. Subsequent treatment with 3-(2-bromoethyl)benzofuran (Flow Sheet IX) affords the alkylated ethoxyamine which cyclizes on warming to afford III.

27. The reduced ketone is converted to the inverted chloro amino hydrochloride with a standard inverting system such as $SOCl_2$ in pyridine.

28. The chloro compound in a dipolar, aprotic solvent such as DMSO, DMF, NMP, etc. is treated with two moles of the sulfonamide anion, $CH_3N^-SO_2(CH_2)_2OH$, to afford III.

Example 16
Pharmaceutical Formulation

| Ingredient | Mg/Capsule |
|---|---|
| N-(1,3,4,6,7,12bα-Hexahydro-2H-benzo[b]furo[2,3-a]quinolizin-2β-yl)-N,N',N'-trimethylsulfamide.HCl | 6 |
| starch | 87 |
| magnesium stearate | 7 |

The active ingredient, starch and magnesium stearate are blended together. The mixture is used to fill hard shell capsules of a suitable size at a fill weight of 100 mg per capsule.

Example 17

| Ingredient | Mg/Capsule |
|---|---|
| N-(1,3,4,6,7,12bα-Hexahydro-2H-benzo[b]furo[2,3-a]quinolizin-2β-yl)-N-methyl-2-hydroxyethane-sulfonamide.HCl | 6 |
| starch | 87 |
| magnesium stearate | 7 |

**Claims for the Contracting States: BE CH DE FR GB IT LI LU NL SE**

1. Compounds of structural formula I

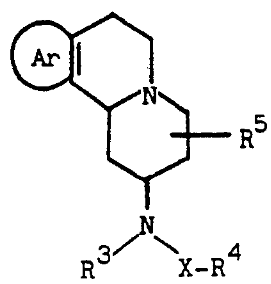

I

or pharmaceutically acceptable salts of the compounds of formula I, wherein
Ar represents an aromatic heterocycle selected from

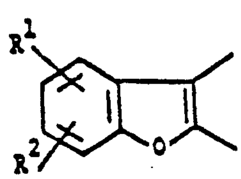

benzofuro-,

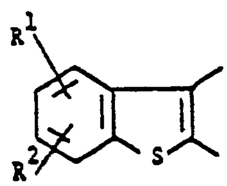

benzothieno-,

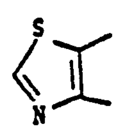

thiazolo-,

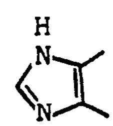

imidazo-,

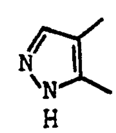

pyrazolo-,

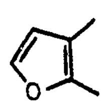

furo-,

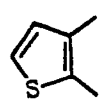

thieno-,

and

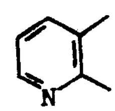

pyridino-;

$R^1$ and $R^2$ are independently:
1) hydrogen,
2) halo,
3) hydroxy,
4) $C_{1-3}$alkoxy, or
5) $C_{1-6}$alkyl;
$R^3$ is
1) hydrogen,
2) $-\overset{O}{\overset{\|}{C}}-R$, wherein R is hydrogen or $C_{1-3}$alkyl,
3) $C_{1-6}$alkyl, either unsubstituted or substituted with one or more of:
a) hydroxy,
b) carboxy,
c) $C_{1-3}$alkoxycarbonyl,
d) halo,

e) $C_{1-3}$alkoxy,

f) —$CONR^6R^7$, wherein $R^6$ and $R^7$ are independently from each other hydrogen or $C_{1-5}$alkyl or $R^6$ and $R^7$ form together with the nitrogen atom to which they are attached a pyrrolidino-, piperidino-, morpholino-, piperazino- or N—$C_{1-3}$ alkylpiperazino-ring or

g) —$NR^6R^7$; wherein $R^6$ and $R^7$ are as defined in 3f);

X is 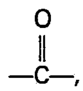, 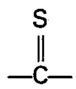 or 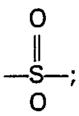; and

$R^4$ is

1) —$OR^8$, wherein $R^8$ is hydrogen or $C_{1-6}$alkyl, either unsubstituted or substituted with one or more groups of formula OR, or —NRCOR, wherein R is hydrogen or $C_{1-3}$alkyl,

2) —$N(R^8)_2$, wherein $R^8$ is as defined in 1),

3) —$CO_2R^8$, wherein $R^8$ is as defined in 1),

4) —$CONR^6R^7$, wherein $R^6$ and $R^7$ are independently from each other hydrogen or $C_{1-5}$alkyl or $R^6$ and $R^7$ form together with the nitrogen atom to which they are attached a pyrrolidino-, piperidino-, morpholino-, piperazino- or N—$C_{1-3}$ alkylpiperazino-ring,

5) $C_{1-6}$alkyl, either unsubstituted or substituted with groups selected from —$OR^8$, —halo, —$CO_2H^8$ and —$CONR^6R^7$, wherein $R^6$ and $R^7$ are as defined in 4) and $R^8$ is as defined in 1),

6) $C_{2-5}$alkenyl,

7) $C_{2-5}$alkynyl,

8) $C_{3-6}$cycloalkyl,

9) 5 or 6-membered heterocycle selected from imidazo, thiazolo, oxazolo, piperidino, piperazino, pyridino and pyrazino,

10) carbocyclic aryl of 6—10 carbon atoms, either unsubstituted or substituted with one or more of the groups selected from halo and OR, wherein R is hydrogen or $C_{1-3}$alkyl or

$R^3$ and the group —X—$R^4$ form together with the nitrogen atom to which they are attached a heterocyclic structure selected from 2-oxazolidinon-1-yl and succinimidoyl and

$R^5$ is

1) hydrogen,

2) $C_{1-6}$alkyl, either unsubstituted or substituted with one or more of the groups of formula —$OR^8$, —$NR^8COR^8$, or —$CO_2R^8$, wherein $R^8$ is hydrogen or $C_{1-6}$alkyl, either unsubstituted or substituted with one or more of the groups of formula OR, or —NRCOR, wherein R is hydrogen or $C_{1-3}$alkyl,

3) —$CO_2R^8$, wherein $R^8$ is as defined in 2),

4) —$CONR^6R^7$, wherein $R^6$ and $R^7$ are independently from each other hydrogen or $C_{1-5}$alkyl or $R^6$ and $R^7$ form together with the nitrogen atom to which they are attached a pyrrolidino-, piperidino-, morpholino-, piperazino- or N—$C_{1-3}$alkylpiperazino-ring or

provided that $R^5$ is in the position 1 or the position 3, the radical $R^5$ can form together with the radical $R^3$ and the nitrogen atom to which it is attached or together with the group —X—$R^4$ and the nitrogen to which said group is attached, a 5- or 6-membered ring and with the provision that if the group —X—$R^4$ is a group having the formula

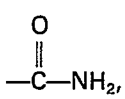, 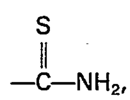, 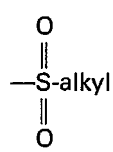 or 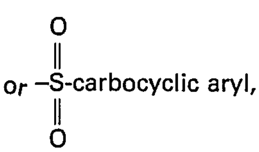,

then the group Ar must not be the aromatic heterocycle thieno or pyridino.

2. Compound of formula I according to claim 1 or pharmaceutically acceptable salts of compounds of formula I wherein Ar is $R^1$,$R^2$-benzo[b]furo- or $R^1$,$R^2$-benzo[b]thieno, wherein

$R^1$ and $R^2$ are hydrogen or halo,

$R^3$ is $C_{1-6}$alkyl,

X is

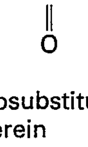

$R^4$ is $C_{1-6}$alkyl, either unsubstituted or monosubstituted with groups selected from —OH, —halo, —$CO_2R^8$ and —$CONR^6R^7$, wherein

$R^8$ is hydrogen or $C_{1-6}$alkyl, either unsubstituted or substituted with one or more of the groups of

formula OR, or —NRCOR, wherein R is hydrogen or $C_{1-3}$alkyl and

$R^6$ and $R^7$ are independently from each other hydrogen or $C_{1-5}$alkyl or

$R^6$ and $R^7$ form together with the nitrogen atom to which they are attached a pyrrolidino-, piperidino-, morpholino-, piperazino- or N—$C_{1-3}$alkylpiperazino-ring;

—$N(R^{8'})_2$, wherein $R^{8'}$ is $C_{1-3}$alkyl,

$C_{2-5}$alkenyl,

$R_{6-10}$carbocyclic aryl,

—$CO_2R^8$, wherein $R^8$ is hydrogen or $C_{1-6}$alkyl, either unsubstituted or substituted with one or more of OR, or —NRCOR, wherein R is hydrogen or $C_{1-3}$alkyl, or 5 or 6-membered heterocycle selected from imidazo, thiazolo, oxazolo, piperidino, piperazino, pyridino and pyrazino, and

$R^5$ is hydrogen or $C_{1-6}$alkyl.

3. Compound of formula I according to claim 2 or a pharmaceutically acceptable salt thereof wherein Ar is $R^1$,$R^2$-benzo[b]furo- or $R^1$,$R^2$-benzo[b]thieno-, wherein

$R^1$ and $R^2$ are hydrogen,

$R^3$ is methyl,

$R^4$ is $C_{1-6}$alkyl, hydroxypropyl, hydroxyethyl, dimethylamino, $C_{1-3}$alkoxycarbonylethyl, or dimethylaminocarbonylmethyl, and

$R^5$ is hydrogen.

4. Compound of formula I according to claim 2 which is

a) N-(1,3,4,6,7,12bα-hexyhydro-2H-benzo[b]furo[2,3a]quinolizin-2β-yl)-N-methyl-2-hydroxyethanesulfonamide;

b) N-(1,3,4,6,7,12bα-hexahydro-2H-benzo[b]furo[2,3a]quinolizin-2β-yl)-N-methyl-3-hydroxypropanesulfonamide;

c) N-(1,3,4,6,7,12bα-hexahydro-2H-benzo[b]furo[2,3a]quinolizin-2β-yl)-N-methyl-methanesulfonamide;

d) (+)-N-(1,3,4,6,7,12bα-hexahydro-2H-benzo[b]furo[2,3a]quinolizin-2β-yl)-N,N',N'-trimethylsulfonamide; or

e) N-(1,3,4,6,7,12bα-hexahydro-2H-benzo[b]thieno[2,3a]quinolizin-2β-yl)-N-methyl-methanesulfonamide;

or a pharmaceutically acceptable salt of the compounds (a) through (e).

5. Process for the preparation of the compounds of formula I

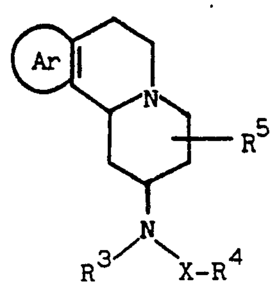

I

according to claim 1 or pharmaceutically acceptable salts of the compounds of formula I, wherein Ar, $R^1$, $R^2$, $R^3$, X, $R^4$ and $R^5$ are as defined in claim 1, characterized in that a compound of formula IIa

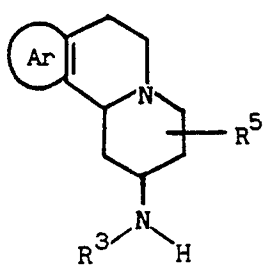

IIa ,

wherein Ar, $R^1$, $R^2$, $R^3$ and $R^5$ are as defined in formula I is acylated with an acylating reagent which introduces the acyl group of formula —X—$R^4$, wherein $R^4$ and X are as defined in formula I and which acylating agent is an acid halide, an activated ester, an acid anhydride or a mixed acid anhydride and the compounds of formula I are isolated in the free form or as pharmaceutically acceptable salts thereof.

6. Process for the preparation of compounds of structural formula Ia

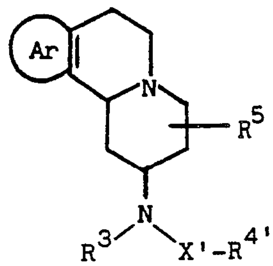

Ia

according to claim 1 or pharmaceutically acceptable salts thereof, wherein Ar, $R^1$, $R^2$, $R^3$ and $R^5$ are as defined in claim 1 and $—X'—R^{4'}$ is a group having the structure

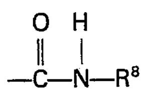 or 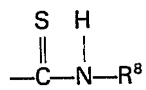

wherein $R^8$ is hydrogen or $C_{1-6}$alkyl, either unsubstituted or substituted with one or more groups of formula

OR, or —NRCOR,

wherein R is hydrogen or $C_{1-3}$alkyl, characterized in that a compound of formula IIa

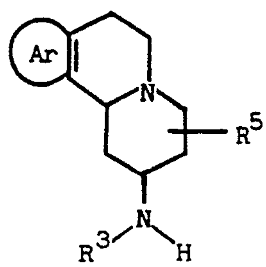

IIa ,

wherein Ar, $R^1$, $R^2$, $R^3$ and $R^5$ are as defined in formula Ia, is treated with an isocyanate of formula

$$O{=}C{=}NR^8$$

or an isothiocyanate of formula

$$S{=}C{=}NR^8$$

and that the resulting compounds of formula Ia or pharmaceutically acceptable salts thereof are isolated.

7. Process for the preparation of compounds of formula Ib

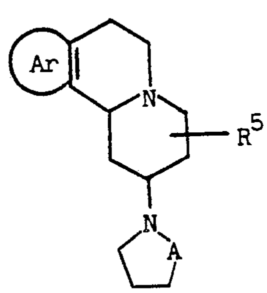

Ib

according to claim 1 or pharmaceutically acceptable salts of the compounds of formula Ib wherein Ar, $R^1$, $R^2$ and $R^5$ are as defined in claim 1 and A is

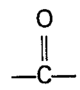 or 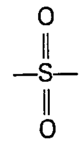

characterized in that a compound of formula

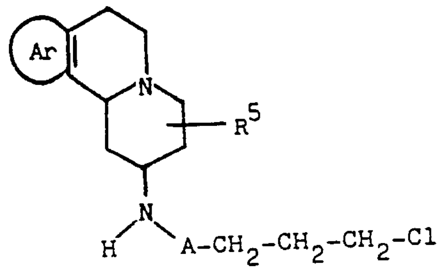 ,

wherein Ar, $R^1$, $R^2$, $R^5$ and A are as defined in formula Ib is cyclisized in the presence of a strong base and the resulting compounds of formula Ib or pharmaceutically acceptable salts thereof are isolated.

8. Process for the preparation of compounds of formula Ic

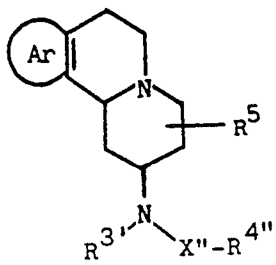

Ic

according to claim 1 or pharmaceutically acceptable salts of the compounds of formula Ic, wherein Ar, $R^1$, $R^2$ and $R^5$ are as defined in claim 1 and $R^{3'}$ and the group —X''—$R^{4''}$ form together with the nitrogen atom to which they are attached a heterocyclic structure selected from 2-oxazolidinon-1-yl and succinimidoyl characterized in that a compound of formula IIb

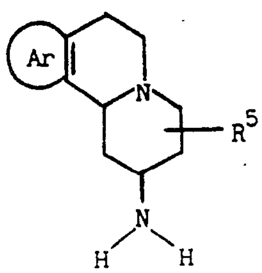

IIb ,

wherein Ar, $R^1$, $R^2$ and $R^5$ are as defined in formula Ic, is acylated with a cyclic dicarboxylic anhydride having the formula

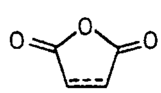 or 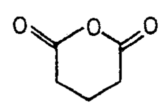

and that the resulting compounds of formula Ic are isolated in the free form or as pharmaceutically acceptable salts of said compounds of formula Ic.

9. Process for the preparation of compounds of structural formula Id

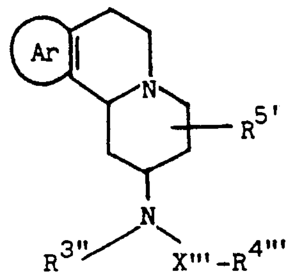

Id

according to claim 1 or pharmaceutically acceptable salts of the compounds of formula Id, wherein Ar, $R^1$ and $R^2$ are as defined in claim 1 and $R^{5'}$ is in the position 1 or in the position 3 and said radical $R^{5'}$ forms together with the radical $R^{3''}$ and with the nitrogen atom to which it is attached, or together with the group

$$—X'''—R^{4'''}$$

and the nitrogen to which said group is attached, a 5- or 6-membered ring which is a cyclic carbamate, cyclic urea or cyclic sulfamide, by treating a starting material having a corresponding substituent in the position 1 or the position 3 with carbonyldiimidazole or sulphuryl chloride.

10. Pharmaceutical composition having $\alpha_2$-adrenoceptor antagonist activity characterized in that it comprises as pharmaceutically active ingredient a compound of structural formula I according to claim 1 or a pharmaceutically acceptable salt of the compound of formula I.

11. Pharmaceutical composition according to claim 10 characterized in that it comprises as pharmaceutically active ingredient a compound according to one of the claims 2—4 or a pharmaceutically acceptable salt of said compound and optionally furthermore a pharmaceutically acceptable carrier.

**Claims for the Contracting State: AT**

1. Process for the preparation of compounds of structural formula I

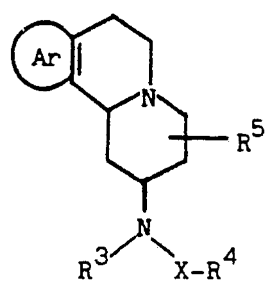

I

or pharmaceutically acceptable salts of the compounds of formula I, wherein

Ar represents an aromatic heterocycle selected from

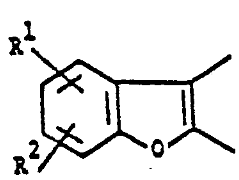

benzofuro-,

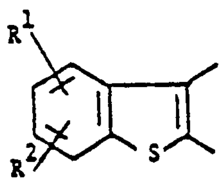

benzothieno-,

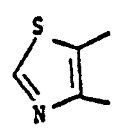

thiazolo-,

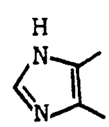

imidazo-,

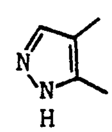

pyrazolo-,

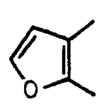

furo-,

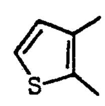

thieno-, and

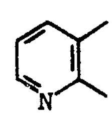

pyridino-;

$R^1$ and $R^2$ are independently:
1) hydrogen,
2) halo,
3) hydroxy,
4) $C_{1-3}$alkoxy, or
5) $C_{1-6}$alkyl;
$R^3$ is
1) hydrogen,
2) $-\overset{\overset{O}{\|}}{C}-R$, wherein R is hydrogen or $C_{1-3}$alkyl,
3) $C_{1-6}$alkyl, either unsubstituted or substituted with one or more of:
a) hydroxy,
b) carboxy,
c) $C_{1-3}$alkoxycarbonyl,
d) halo,
e) $C_{1-3}$alkoxy,
f) $-CONR^6R^7$, wherein $R^6$ and $R^7$ are independently from each other hydrogen or $C_{1-5}$alkyl or $R^6$ and $R^7$ form together with the nitrogen atom to which they are attached a pyrrolidino-, piperidino-, morpholino-, piperazino- or N—$C_{1-3}$ alkylpiperazino-ring or
g) $-NR^6R^7$; wherein $R^6$ and $R^7$ are as defined in (3f);

X is $-\overset{\overset{O}{\|}}{C}-$, $-\overset{\overset{S}{\|}}{C}-$ or $-\underset{\underset{O}{\|}}{\overset{\overset{O}{\|}}{S}}-$; and

$R^4$ is
1) $-OR^8$, wherein $R^8$ is hydrogen or $C_{1-6}$alkyl, either unsubstituted or substituted with one or more groups of formula OR, or —NRCOR, wherein R is hydrogen or $C_{1-3}$alkyl,
2) $-N(R^8)_2$, wherein $R^8$ is as defined in (1),
3) $-CO_2R^8$, wherein $R^8$ is as defined in (1),
4) $-CONR^6R^7$, wherein $R^6$ and $R^7$ are independently from each other hydrogen or $C_{1-5}$alkyl or $R^6$ and $R^7$ form together with the nitrogen atom to which they are attached a pyrrolidino-, piperidino-, morpholino-, piperazino- or N—$C_{1-3}$ alkylpiperazino-ring,
5) $C_{1-6}$alkyl, either unsubstituted or substituted with groups selected from $-OR^8$, —halo, $-CO_2H^8$ and $-CONR^6R^7$, wherein $R^6$ and $R^7$ are as defined in (4) and $R^8$ is as defined in (1),
6) $C_{2-5}$alkenyl,
7) $C_{2-5}$alkynyl,
8) $C_{3-6}$cycloalkyl,
9) 5 or 6-membered heterocycle selected from imidazo, thiazolo, oxazolo, piperidino, piperazine, pyridino and pyrazino, or
10) carbocyclic aryl of 6—10 carbon atoms, either unsubstituted or substituted with one or more of the groups selected from halo and OR, wherein R is hydrogen or $C_{1-3}$alkyl or
$R^3$ and the group $-X-R^4$ form together with the nitrogen atom to which they are attached a heterocyclic structure selected from 2-oxazolidinon-1-yl and succinimidoyl and
$R^5$ is
1) hydrogen,
2) $C_{1-6}$alkyl, either unsubstituted or substituted with one or more of the groups of formula $-OR^8$, $-NR^8COR^8$, or $-CO_2R^8$, wherein $R^8$ is hydrogen or $C_{1-6}$alkyl, either unsubstituted or substituted with one or more of the groups of formula OR, or —NRCOR, wherein R is hydrogen or $C_{1-3}$alkyl,
3) $-CO_2R^8$, wherein $R^8$ is as defined in (2),
4) $-CONR^6R^7$, wherein $R^6$ and $R^7$ are independently from each other hydrogen or $C_{1-5}$alkyl or $R^6$ and $R^7$ form together with the nitrogen atom to which they are attached a pyrrolidino-, piperidino-, morpholino-, piperazino- or N—$C_{1-3}$alkylpiperazino-ring or
provided that $R^5$ is in the position 1 or the position 3, the radical $R^5$ can form together with the radical $R^3$ and the nitrogen atom to which it is attached or together with the group $-X-R^4$ and the nitrogen to which said group is attached, a 5- or 6-membered ring and with the provision that if the group $-X-R^4$ is a group having the formula

$-\overset{\overset{O}{\|}}{C}-NH_2$, $-\overset{\overset{S}{\|}}{C}-NH_2$, $-\underset{\underset{O}{\|}}{\overset{\overset{O}{\|}}{S}}$-alkyl or $-\underset{\underset{O}{\|}}{\overset{\overset{O}{\|}}{S}}$-carbocyclic aryl,

then the group Ar must not be the aromatic heterocycle thieno or pyridino, characterized in that a compound of formula IIa

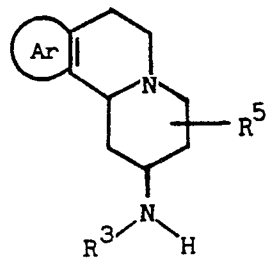

IIa ,

wherein Ar, $R^1$, $R^2$, $R^3$ and $R^5$ are as defined in formula I is acylated with an acylating reagent, which introduces an acyl group of formula —X—$R^4$ wherein $R^4$ and X are as defined in formula I and which acylating agent is an acid halide, an activated ester, an acid anhydride or a mixed acid anhydride and wherein the compounds of formula I are isolated in the free form or as pharmaceutically acceptable salts thereof.

2. Process for the preparation of compounds of structural formula Ia

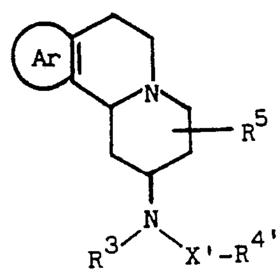

Ia

or pharmaceutically acceptable salts thereof, wherein Ar, $R^1$, $R^2$, $R^3$ and $R^5$ are as defined in claim 1 and —X'—$R^{4'}$ is a group having the structure

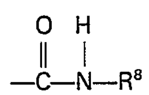

or

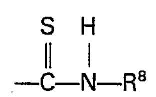

wherein $R^8$ is hydrogen or $C_{1-6}$alkyl, either unsubstituted or substituted with one or more groups of formula

OR, or —NRCOR,

wherein R is hydrogen or $C_{1-3}$alkyl, characterized in that a compound of formula IIa

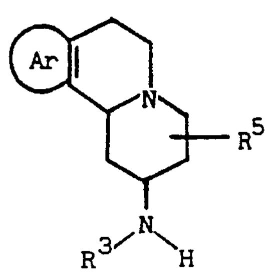

IIa ,

wherein Ar, $R^1$, $R^2$, $R^3$ and $R^5$ are as defined in formula Ia, is treated with an isocyanate of formula

$O{=}C{=}NR^8$

or an isothiocyanate of formula

$S=C=NR^8$

and that the resulting compounds of formula Ia or pharmaceutically acceptable salts thereof are isolated.

3. Process for the preparation of compounds of formula Ib

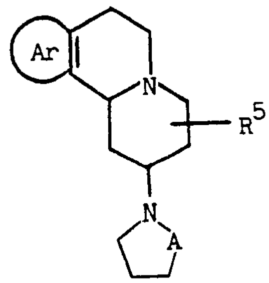

Ib

or pharmaceutically acceptable salts of the compounds of formula Ib wherein Ar, $R^1$, $R^2$ and $R^5$ are as defined in formula 1 and A is

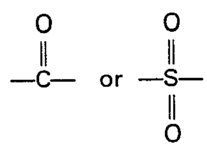

characterized in that a compound of formula

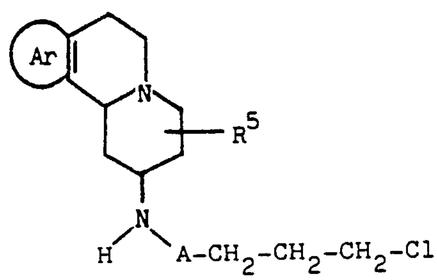

,

wherein Ar, $R^1$, $R^2$, $R^5$ and A are as defined in formula Ib is cyclisized in the presence of a strong base and the resulting compounds of formula Ib or pharmaceutically acceptable salts thereof are isolated.

4. Process for the preparation of compounds of formula Ic

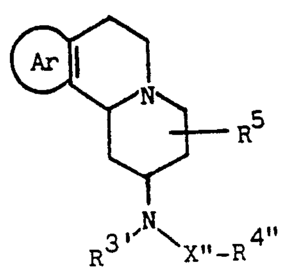

Ic

or pharmaceutically acceptable salts of the compounds of formula Ic, wherein Ar, $R^1$, $R^2$ and $R^5$ are as defined in formula 1 and $R^{3'}$ and the group —X''—$R^{4''}$ form together with the nitrogen atom to which they are attached a heterocyclic structure selected from 2-oxazolidinon-1-yl and succinimidoyl characterized in that a compound of formula IIb

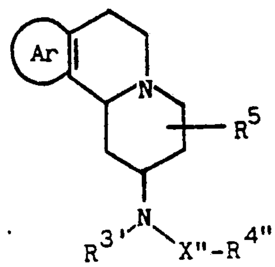

Ic

wherein Ar, $R^1$, $R^2$ and $R^5$ are as defined in formula Ic, is acylated with a cyclic dicarboxylic anhydride having the formula

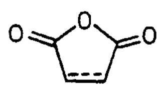 oder 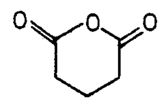

and that the resulting compounds of formula Ic are isolated in the free form or as pharmaceutically acceptable salts of said compounds of formula Ic.

5. Process for the preparation of compounds of structural formula Id

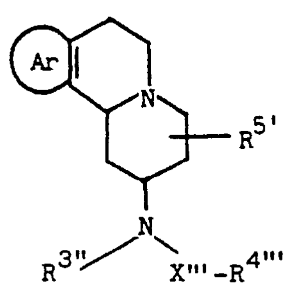

Id

or pharmaceutically acceptable salts of the compounds of formula Id wherein Ar, $R^1$ and $R^2$ are as defined in formula I and $R^{5'}$ is in the position 1 or in the position 3 and said radical $R^{5'}$ forms together with the radical $R^{3''}$ and with the nitrogen atom to which it is attached, or together with the group

$$—X'''—R^{4'''}$$

and the nitrogen to which said group is attached, a 5- or 6-membered ring which is a cyclic carbamate, cyclic urea or cyclic sulfamide, by treating a starting material having a corresponding substituent in the position 1 or the position 3 with carbonyldiimidazole or sulphuryl chloride.

6. Process according to one of the claims 1—5, characterized in that a compound of formula I is prepared wherein Ar is $R^1$, $R^2$-benzo[b]furo- or $R^1$, $R^2$-benzo[b]thieno, wherein

$R^1$ and $R^2$ are hydrogen or halo,

$R^3$ is $C_{1-6}$alkyl,

X is

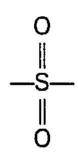

$R^4$ is $C_{1-6}$alkyl, either unsubstituted or monosubstituted with groups selected from —OH, —halo, —$CO_2R^8$ and —$CONR^6R^7$, wherein

$R^8$ is hydrogen or $C_{1-6}$alkyl, either unsubstituted or substituted with one or more groups of formula OR, or —NRCOR, wherein R is hydrogen or $C_{1-3}$alkyl and

$R^6$ and $R^7$ are independently from each other hydrogen or $C_{1-5}$alkyl or

$R^6$ and $R^7$ form together with the nitrogen atom to which they are attached a pyrrolidino-, piperidino-, morpholino-, piperazino- or N—$C_{1-3}$alkylpiperazino-ring;

—$N(R^{8'})_2$, wherein $R^{8'}$ is $C_{1-3}$alkyl,

$C_{2-5}$alkenyl,

$R_{6-10}$carbocyclic aryl,

—$CO_2R^8$, wherein $R^8$ is hydrogen or $C_{1-6}$alkyl, either unsubstituted or substituted with one or more of OR, or —NRCOR, wherein R is hydrogen or $C_{1-3}$alkyl, or 5 or 6-membered heterocycle selected from imidazo, thiazolo, oxazolo, piperidino, piperazino, pyridino and pyrazino, and

$R^5$ is hydrogen or $C_{1-6}$alkyl, or that pharmaceutically acceptable salts of said compound of formula I are prepared.

7. Process according to one of the claims 1—6, characterized in that a compound of formula I is prepared wherein Ar is $R^1$, $R^2$-benzo[b]furo- or $R^1$, $R^2$-benzo[b]thieno-, wherein

$R^1$ and $R^2$ are hydrogen,

$R^3$ is methyl,

$R^4$ is $C_{1-6}$alkyl, hydroxypropyl, hydroxyethyl, dimethylamino, $C_{1-3}$alkoxycarbonylethyl, or dimethylaminocarbonylmethyl, and

$R^5$ is hydrogen,

or that pharmaceutically acceptable salts of said compound of formula I are prepared.

8. Process according to one of the claims 1—6, characterized in that

a) N-(1,3,4,6,7,12bα-hexyhydro-2H-benzo[b]furo[2,3a]quinolizin-2β-yl)-N-methyl-2-hydroxyethane-sulfonamide;

b) N-(1,3,4,6,7,12bα-hexahydro-2H-benzo[b]furo[2,3a]quinolizin-2β-yl)-N-methyl-3-hydroxypropane-sulfonamide;

c) N-(1,3,4,6,7,12bα-hexahydro-2H-benzo[b]furo[2,3a]quinolizin-2β-yl)-N-methyl-methanesulfonamide;

d) (+)-N-(1,3,4,6,7,12bα-hexahydro-2H-benzo[b]furo[2,3a]quinolizin-2β-yl)-N,N',N'-trimethylsulfonamide; or

e) N-(1,3,4,6,7,12bα-hexahydro-2H-benzo[b]thieno[2,3a]quinolizin-2β-yl)-N-methyl-methanesulfonamide;

or a pharmaceutically acceptable salt of the compounds (a) through (e) is prepared.

9. Process for the preparation of a pharmaceutical composition having $\alpha_2$-adrenoceptor antagonist activity, characterized in that compounds of formula I or pharmaceutically acceptable salts of said compounds of formula I prepared according to the process of claim 1 are formulated with pharmaceutically acceptable carriers or further selected from antidepressants, serotinin reuptake inhibitors and monoamine oxidase inhibitors to form the pharmaceutical composition.

10. Process according to claim 9, characterized in that the active ingredients prepared according to the processes of claims 2 through 8 are used as active ingredients for formulating said pharmaceutical compositions.

**Patentansprüche für die Vertragsstaaten: BE CH DE FR GB IT LI LU NL SE**

1. Verbindungen der Strukturformel I

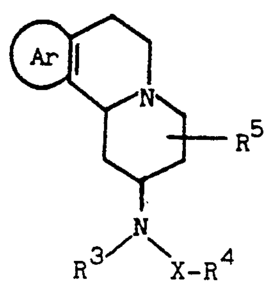

I

oder pharmazeutisch annehmbare Salze dieser Verbindungen der Formel I, worin

Ar einen aromatischen Heterocyclus darstellt, ausgewählt aus

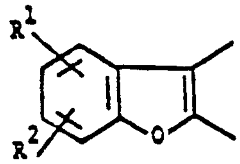
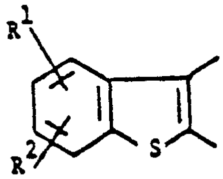
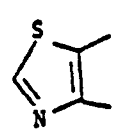
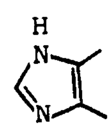
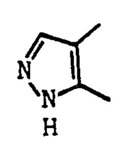

Benzofuro-, Benzothieno-, Thiazolo-, Imidazo-, Pyrazolo-,

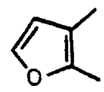 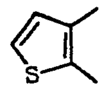 und 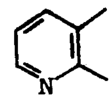

Furo-, Thieno- Pyridino-;

$R^1$ und $R^2$ unabhängig
1) Wasserstoff,
2) Halogen,
3) Hydroxy,
4) $C_{1-3}$-Alkoxy, oder
5) $C^1{}_{-6}$-Alkyl sind;
1) Wasserstoff,
2) $\overset{O}{\overset{\|}{-C-}}R$, worin R Wasserstoff oder $C_{1-3}$-Alkyl ist,
3) $C_{1-6}$-Alkyl, entweder unsubstituiert oder substituiert mit einem oder mehreren aus
a) Hydroxy,
b) Carboxy,
c) $C_{1-3}$-Alkoxycarbonyl,
d) Halogen,
e) $C_{1-3}$-Alkoxy,
f) $-CONR^6R^7$, worin $R^6$ und $R^7$ unabhängig voneinander Wasserstoff oder $C_{1-5}$-Alkyl sind, oder $R^6$ und $R^7$ zusammen mit dem Stickstoffatom, an das sie gebunden sind, einen Pyrrolidino-, Piperidino-, Morpholino-, Piperazino- oder N—$C_{1-3}$-Alkylpiperazinoring bilden, oder
g) $-NR^6R^7$; worin $R^6$ und $R^7$ wie in 3f) definiert sind; ist

X $\overset{O}{\overset{\|}{-C-}}$, $\overset{S}{\overset{\|}{-C-}}$ oder $\underset{O}{\underset{\|}{\overset{O}{\overset{\|}{-S-}}}}$ ist und

$R^4$
1) $-OR^8$, worin $R^8$ Wasserstoff oder $C_{1-6}$-Alkyl ist, entweder unsubstituiert oder mit einer oder mehreren Gruppen der Formel OR oder —NRCOR substituiert, worin R Wasserstoff oder $C_{1-3}$-Alkyl ist,
2) $-N(R^8)_2$, worin $R^8$ wie in 1) definiert ist,
3) $-CO_2R^8$, worin $R^8$ wie in 1) definiert ist,
4) $-CONR^6R^7$, worin $R^6$ und $R^7$ unabhängig voneinander Wasserstoff oder $C_{1-5}$-Alkyl sind, oder $R^6$ und $R^7$ zusammen mit dem Stickstoffatom, an das sie gebunden sind, einen Pyrrolidino-, Piperidino-, Morpholino-, Piperazino- oder N—$C_{1-3}$-Alkylpiperazinoring bilden,
5) $C_{1-6}$-Alkyl, entweder unsubstituiert oder substituiert mit aus $-OR^8$, -Halogen, $-CO_2R^8$ und $-CONR^6R^7$ ausgewählten Gruppen, worin $R^6$ und $R^7$ wie in 4) definiert sind und $R^8$ wie in 1) definiert ist,
6) $C_{2-5}$-Alkenyl,
7) $C_{2-5}$-Alkinyl,
8) $C_{3-6}$-Cycloalkyl,
9) ein 5- oder 6-gliedriger Heterocyclus, ausgewählt aus Imidazo, Thiazolo, Oxazolo, Piperidino, Piperazino, Pyridino und Pyrazino,
10) carbocyclisches Aryl mit 6 bis 10 Kohlen-stoffatomen, entweder unsubstituiert oder mit einer oder mehreren Gruppen, ausgewählt aus Halogen und OR, substituiert, worin R Wasserstoff oder $C_{1-3}$-Alkyl ist, ist oder
$R^3$ und die Gruppe —X—$R^4$ zusammen mit dem Stickstoffatom, as das sie gebunden sind, eine aus 2-Oxazolidinon-1-yl und Succinimidoyl ausgewählte heterocyclische Struktur bilden und $R^5$
1) Wasserstoff,
2) $C_{1-6}$-Alkyl, entweder unsubstituiert oder mit einer oder mehreren Gruppen der Formel $-OR^8$, $-NR^8COR^8$ oder $-CO_2R^8$ substituiert, worin $R^8$ Wasserstoff oder $C_{1-6}$-Alkyl ist, entweder unsubstituiert oder mit einer oder mehreren Gruppen der formel OR oder —NRCOR substituiert worin R Wasserstoff oder $C_{1-3}$-Alkyl ist,
3) $-CO_2R^8$, worin $R^8$ wie in 2) definiert ist,
4) $-CONR^6R^7$, worin $R^6$ und $R^7$ unabhängig von einander Wasserstoff oder $C_{1-5}$-Alkyl sind oder $R^6$ und $R^7$ zusammen mit dem Stickstoffatom, an das sie gebunden sind, einen Pyrrolidino-, Piperidino-, Morpholino-, Piperazino- oder N—$C_{1-3}$-Alkylpiperazinoring bilden, ist, wobei unter der Voraussetzung, daß $R^5$ in der 1-Stellung oder der 3-Stellung vorliegt, der Rest $R^5$ zusammen mit dem Rest $R^3$ und dem Stickstoffatom, an das er gebunden ist, oder zusammen mit der Gruppe —X—$R^4$ und dem Stickstoff, an das diese Gruppe gebunden ist, einen 5- oder 6-gliedrigen Ring bilden kann, und mit der Maßgabe, daß wenn die Gruppe —X—$R^4$ eine Gruppe der Formel

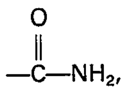, 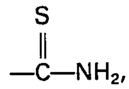, 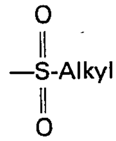 oder 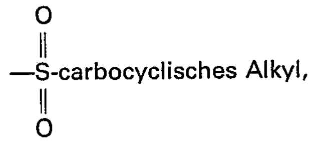,

ist, die Gruppe Ar nicht der aromatische Heterocyclus Thieno oder Pyridino sein darf.

2. Verbindung der Formel I nach Anspruch 1 oder pharmazeutisch annehmbare Salz der Verbindungen der Formel I, worin Ar $R^1$, $R^2$-Benzo[b]furo- oder $R^1$, $R^2$-Benzo[b]thieno ist, worin $R^1$ und $R^2$

$R^1$ und $R^2$ Wasserstoff oder Halogen sind,

$R^3$ $C_{1-6}$-Alkyl ist,

X 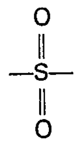 ist,

$R^4$ $C_{1-6}$-Alkyl, entweder unsubstituiert oder mit aus —OH, -Halogen, —$CO_2R^8$ und —$CONR^6R^7$ ausgewählten Gruppen, ist, worin

$R^8$ Wasserstoff oder $C_{1-6}$-Alkyl, entweder unsubstituiert oder mit einer oder mehreren Gruppen der Formel OR oder —NRCOR substituiert, worin R Wasserstoff oder $C_{1-3}$-Alkyl ist, ist und

$R^6$ und $R^7$ unabhängig voneinander Wasserstoff oder $C_{1-5}$-Alkyl sind oder

$R^6$ und $R^7$ zusammen mit dem Stickstoffatom, an das sie gebunden sind, einen Pyrrolidino-, Piperidino, Morpholino-, Piperazino- oder N—$C_{1-3}$-Alkylpiperazinoring bilden;

—$N(R^{8'})_2$, worin $R^{8'}$ $C_{1-3}$-Alkyl ist, $C_{1-5}$-Alkenyl, $C_{6-10}$-carbocyclisches Aryl, —$CO_2R^8$, worin $R^8$ Wasserstoff oder $C_{1-6}$-Alkyl, entweder unsubstituiert oder mit einer oder mehreren OR oder —NRCROR substituiert, worin R Wasserstoff oder $C_{1-3}$-Alkyl ist, ist, oder ein 5- oder 6-gliedriger Heterocyclus, ausgewählt aus Imidazo, Thiazolo, Oxazolo, Piperidino, Piperazino, Pyridino und Pyrazino ist, und

$R^5$ Wasserstoff oder $C_{1-6}$-Alkyl ist.

3. Verbindung der Formel I nach Anspruch 2 oder ein pharmazeutisch annehmbares Salz davon, worin Ar $R^1$, $R^2$-Benzo[b]furo- oder $R^1$, $R^2$-Benzo[b]thieno ist, worin $R^1$ und $R^2$ Wasserstoff sind

$R^3$ Methyl ist,

$R^4$ $C_{1-6}$-Alkyl, Hydroxypropyl, Hydroxyethyl, Dimethylamino, $C_{1-3}$-Alkoxycarbonylethyl oder Dimethylaminocarbonylmethyl ist, und

$R^5$ Wasserstoff ist.

4. Verbindung der Formel I nach Anspruch 2, welche

(a) N-(1,3,4,6,7,12bα-Hexahydro-2H-benzo[b]furo[2,3a]-chinolizin-2β-yl)-N-methyl-2-hydroxyethansulfonamid;

(b) N-(1,3,4,6,7,12bα-Hexahydro-2H-benzo[b]furo[2,3a]-chinolizin-2β-yl)-N-methyl-2-hydroxypropansulfonamid;

(c) N-(1,3,4,6,7,12bα-Hexahydro-2H-benzo[b]furo[2,3a]-chinolizin-2β-yl)-N-methyl-methansulfonamid;

(d) (+)-N-(1,3,4,6,7,12bα-Hexahydro-2H-benzo[b]furo[2,3a]-chinolizin-2β-yl)-N,N',N'-trimethylsulfamid; oder

(e) N-(1,3,4,6,7,12bα-Hexahydro-2H-benzo[b]thieno-[2,3a]-chinolizin-2β-yl)-N-methyl-methansulfonamid;

oder ein pharmazeutisch annehmbares Salz der Verbindungen (a) bis (e) ist.

5. Verfahren zur Herstellung der Verbindungen der Formel I

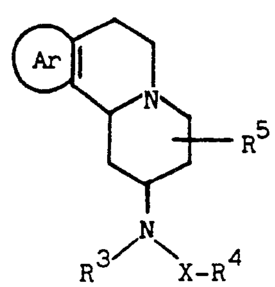

I

nach Anspruch 1 oder pharmazeutisch annehmbarer Salze der Verbindungen der Formel I, worin Ar, $R^1$, $R^2$, $R^3$, X, $R^4$ und $R^6$ wie in Anspruch 1 definiert sind, dadurch gekennzeichnet, daß eine Verbindung der Formel IIa

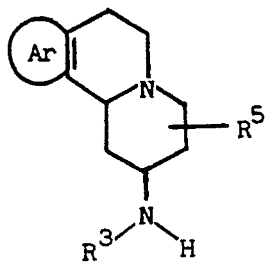

IIa ,

worin Ar, $R^1$, $R^2$, $R^3$ und $R^5$ wie in Formel I definiert sind, mit einem Acylierungsmittel acyliert wird, welches die Acylgruppe der Formel

$$—X—R^4,$$

worin $R^4$ und X wie in Formel I definiert sind, einführt und welches Acylierungsmittel ein Säurehalogenid, ein aktivierter Ester, ein Säureanhydrid oder ein gemischtes Säureanhydrid ist, und die Verbindungen der Formel I in freier Form oder als ihre pharmazeutisch annehmbaren Salze isoliert werden.

6. Verfahren zur Herstellung der Verbindungen der Strukturformel Ia

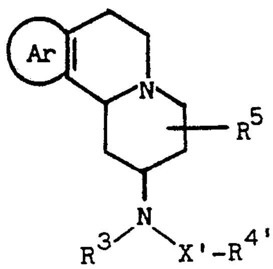

Ia

nach Anspruch 1 oder pharmazeutisch annehmbarer Salze davon, worin Ar, $R^1$, $R^2$, $R^3$ und $R^5$ wie in Anspruch 1 definiert sind und

$$—X'—R^{4'}$$

ein Gruppe der Struktur

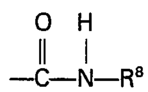 oder 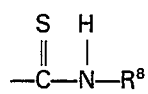

ist, worin $R^8$ Wasserstoff oder $C_{1-6}$-Alkyl ist, entweder unsubstituiert oder mit einer oder mehreren Gruppen der Formel OR oder —NRCOR substituiert, worin R Wasserstoff oder $C_{1-3}$-Alkyl ist, dadurch gekennzeichnet, daß eine Verbindung der Formel IIa

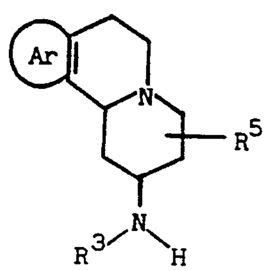

IIa ,

worin Ar, $R^1$, $R^2$, $R^3$ und $R^5$ wie in Formel Ia definiert sind, mit einem Isocyanat der Formel

$$O=C=NR^8$$

oder einem Isothiocyanat der Formel

$$S=C=NR^8$$

behandelt wird, und die resultierenden Verbindungen der Formel Ia oder ihre pharmazeutisch annehmbaren Salze isoliert werden.

7. Verfahren zur Herstellung von Verbindungen der Formel Ib

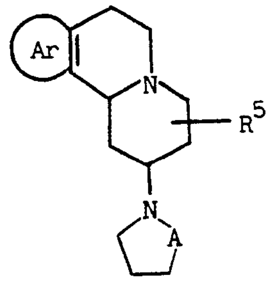

Ib

nach Anspruch 1 oder pharmazeutisch annehmbarer Salze der Verbindungen der Formel Ib, worin Ar, $R^1$, $R^2$ und $R^5$ wie in Anspruch 1 definiert sind und A

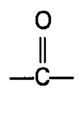 oder 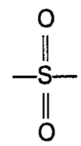

ist, dadurch gekennzeichnet, daß eine Verbindung der Formel

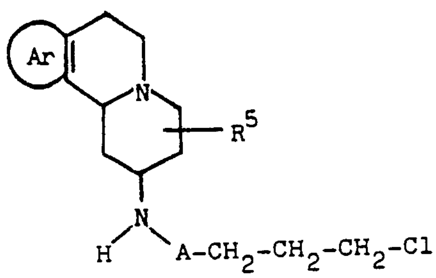 ,

worin Ar, $R^1$, $R^2$, $R^5$ und A wie in Formel Ib definiert sind, in Gegenwart einer starken Base cyclisiert wird und die resultierenden Verbindungen der Formel Ib oder ihre pharmazeutisch annehmbaren Salze isoliert werden.

8. Verfahren zur Herstellung von Verbindungen der Formel Ic

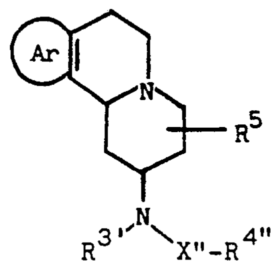

Ic

nach Anspruch 1 oder pharmazeutisch annehmbarer Salze der Verbindungen der Formel Ic, worin Ar, $R^1$, $R^2$ und $R^5$ wie in Anspruch 1 definiert sind und $R^{3'}$ und die Gruppe $—X''—R^{4''}$ zusammen mit dem Stickstoffatom, an das sie gebunden sind, eine aus 2-Oxazolidinon-1-yl und Succinimidoyl ausgewählte heterocyclische Struktur bilden, dadurch gekennzeichnet, daß eine Verbindung der Formel IIb

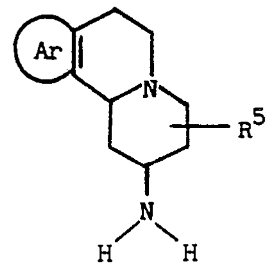

IIb ,

worin Ar, $R^1$, $R^2$ und $R^5$ wie in Formel Ic definiert sind, mit einem cyclischen Dicarbonsäureanhydrid der Formel

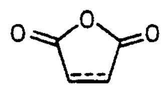 oder 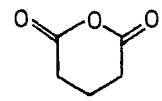

acyliert wird und die resultierenden Verbindungen der Formel Ic in freier Form oder als pharmazeutisch annehmbare Salze der Verbindungen der Formel Ic isoliert werden.

9. Verfahren zur Herstellung von Verbindungen der Strukturformel Id

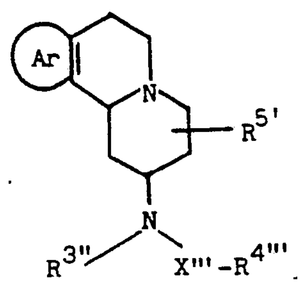

Id

gemäß Anspruch 1 oder pharmazeutisch annehmbarer Salze der Verbindungen der Formel Id, worin Ar, $R^1$ und $R^2$ wie in Anspruch 1 definiert sind und $R^{5'}$ in der 1-Stellung oder in der 3-Stellung ist und der Rest $R^{5'}$ zusammen mit dem Rest $R^{3''}$ und mit dem Stickstoffatom, an welches er gebunden ist, oder zusammen mit der Gruppe

$$—X'''—R^{4'''}$$

und dem Stickstoffatom, an das diese Gruppe gebunden ist, einen 5- oder 6-gliedrigen Ring bildet, der ein cyclisches Carbamat, ein cyclischer Harnstoff oder ein cyclisches Sulfonamid ist, durch Behandlung eines Ausgangsmaterials mit einem entsprechenden Substituenten in der 1-Stellung oder der 3-Stellung mit Carbonyldiimidazol oder Sulfurylchlorid.

10. Pharmazeutische Zusammensetzung mit $\alpha_2$-Adrenozeptor-Antagonistwirksamkeit, dadurch gekennzeichnet, daß sie als pharmazeutisch wirksamen Bestandteil eine Verbindung der Strukturformel I nach Anspruch 1 oder ein pharmazeutisch annehmbares Salz der Verbindung der Formel I umfaßt.

11. Pharmazeutische Zusammensetzung nach Anspruch 10, dadurch gekennzeichnet, daß sie als pharmazeutisch wirksamen Bestandteil eine Verbindung nach einem der Ansprüche 2 bis 4 oder ein pharmazeutisch annehmbares Salz dieser Verbindung und gegebenenfalls weiterhin einen pharmazeutisch annehmbaren Träger umfaßt.

**Patentansprüche für den Vertragsstaat: AT**

1. Verfahren zur Herstellung von Verbindungen der Strukturformel I

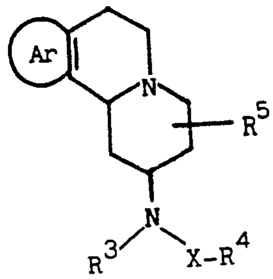

I

oder pharmazeutisch annehmbaren Salzen der Verbindungen der Formel I, worin

Ar einen aromatischen Heterocyclus bedeutet, der aus

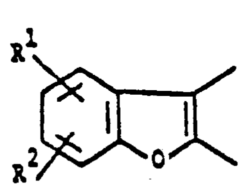 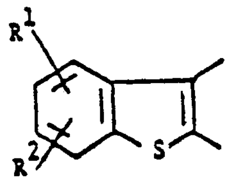 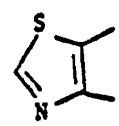 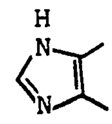 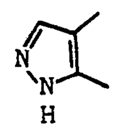

Benzofuro-, Benzothieno-, Thiazolo-, Imidazo-, Pyrazolo-,

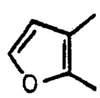 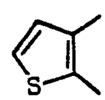 und 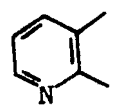

Furo-, Thieno- Pyridino-

ausgewählt ist;

$R^1$ und $R^2$ unabhängig voneinander:

1) Wasserstoff,
2) Halogen,
3) Hydroxy,
4) $C_{1-3}$-Alkoxy, oder
5) $C_{1-6}$-Alkyl sind;

$R^3$

1) Wasserstoff,

O
||
2) —C—R, wobei R Wasserstoff oder $C_{1-3}$-Alkyl ist, oder

3) $C_{1-6}$-Alkyl ist, das entweder unsubstituiert oder durch einen oder mehreren der folgenden Reste substituiert ist:

a) Hydroxy,
b) Carboxy,
c) $C_{1-3}$-Alkoxycarbonyl,
d) Halogen,
e) $C_{1-3}$-Alkoxy,
f) —CONCR$^6$R$^7$, wobei $R^6$ und $R^7$ unabhängig voneinander Wasserstoff oder $C_{1-5}$-Alkyl sind, oder $R^6$ und $R^7$ gemeinsam mit dem Stickstoffatom, an das sie gebunden sind, einen Pyrrolidino-, Piperidino-, Morpholino-, Piperazino- oder N—$C_{1-3}$-Alkylpiperazino-Ring bilden, oder
g) —NR$^6$R$^7$; wobei $R^6$ und $R^7$ wie unter (3f) angegeben definiert sind;

O S O
|| || ||
X —C—, —C— oder —S— ist; und
||
O

$R^4$

1) —$OR^8$, wobei $R^8$ Wasserstoff oder entweder unsubstituiertes oder durch eine oder mehrere Gruppen der Formel OR oder —NRCOR substituiertes $C_{1-6}$-Alkyl ist, wobei R Wasserstoff oder $C_{1-3}$-Alkyl ist,

2) —$N(R^8)_2$, wobei $R^8$ wie unter (1) angegeben definiert ist,

3) —$CO_2R^8$, wobei $R^8$ wie unter (1) angegeben definiert ist,

4) —$CONR^6R^7$, worin $R^6$ und $R^7$ unabhängig voneinander Wasserstoff oder $C_{1-5}$-Alkyl sind, oder $R^6$ und $R^7$ gemeinsam mit dem Stickstoffatom, an das sie gebunden sind, einen Pyrrolidino-, Piperidino-, Morpholino-, Piperazino- oder N—$C_{1-3}$-Alkylpiperazino-Ring bilden,

5) $C_{1-6}$-Alkyl, das entweder unsubstituiert oder durch Gruppen substituiert ist, die aus —$OR^8$, Halogen, —$CO_2R^8$ und —$CONR^6R^7$ ausgewählt sind, wobei $R^6$ und $R^7$ wie unter (4) angegeben definiert sind, und $R^8$ wie unter (1) angegeben definiert ist,

6) $C_{2-5}$-Alkenyl,

7) $C_{2-5}$-Alkinyl,

8) $C_{3-6}$-Cycloalkyl,

9) ein 5- oder 6-gliedriger Heterocyclus, ausgewählt aus Imidazo, Thiazolo, Oxazolo, Piperidino, Piperazino, Pyridino und Pyrazino, oder

10) carbocyclisches Aryl mit 6—10 Kohlenstoffatomen ist, das entweder unsubstituiert oder durch eine oder mehrere aus Halogen und OR ausgewählte Gruppen substituiert ist, wobei R Wasserstoff oder $C_{1-3}$-Alkyl ist, oder

$R^3$ und die Gruppe —X—$R^4$ gemeinsam mit dem Stickstoffatom, as das sie gebunden sind, eine heterocyclische Struktur bilden, die aus 2-Oxazolidinon-1-yl und Succinimidoyl ausgewähalt ist, und $R^5$

1) Wasserstoff,

2) unsubstituiertes oder durch eine oder mehrere Gruppen der Formel $OR^8$, —$NR^8COR^8$ oder —$CO_2R^8$ substituiertes $C_{1-6}$-Alkyl, wobei $R^8$ Wasserstoff oder entweder ubsubstituiertes oder durch eine oder mehrere Gruppen der Formel OR oder —NRCOR substituiertes $C_{1-6}$-Alkyl ist, wobei R Wasserstoff oder $C_{1-3}$-Alkyl ist,

3) —$CO_2R^8$, wobei $R^8$ wie unter (2) angegeben definiert ist, oder

4) —$CONR^6R^7$ ist, worin $R^6$ und $R^7$ unabhängig voneinander Wasserstoff oder $C_{1-5}$-Alkyl sind bedeuten, oder $R^6$ und $R^7$ zusammen mit dem Stickstoffatom, an das sie gebunden sind, einen Pyrrolidino-, Piperidino-, Morpholino-, Piperazino- oder N—$C_{1-3}$-Alkylpiperazino-Ring bilden, oder vorausgesetzt, daß $R^5$ in der Stellung 1 oder in der Stellung 3 vorliegt, der Rest $R^5$ gemeinsam mit dem Rest $R^3$ und dem Stickstoffatom, an das dieser gebunden ist, oder zusammen mit der Gruppe —X—$R^4$ und dem Stickstoff, an das die genannte Gruppe gebunden ist, einen 5- oder 6-gliedrigen Ring bilden kann, und mit der Maßgabe, daß, falls die Gruppe

—X—$R^4$

eine Gruppe mit der Formel

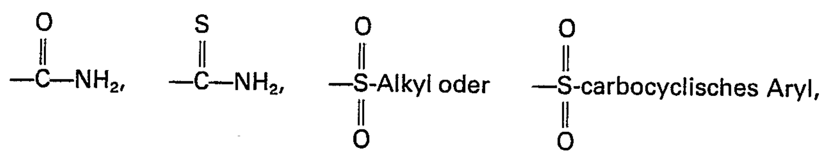

ist, die Gruppe Ar nicht der aromatische Heterocyclus Thieno oder Pyridino sein darf, dadurch gekennzeichnet, daß eine Verbindung der Formel IIa

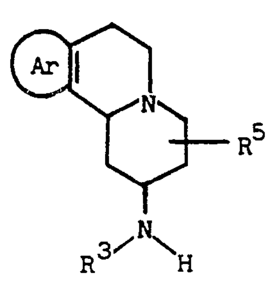

IIa ,

worin Ar, $R^1$, $R^2$, $R^3$ und $R^5$ wie in Formel I definiert sind, mit einem Acylierungsreagens, welches eine Acylgruppe der Formel

—X—$R^4$ einführt,

worin $R^4$ und X wie in Formel I definiert sind, und welches Acylierungsmittel ein Säurehalogenid, ein aktivierter Ester, ein Säureanhydrid oder ein gemischtes Säureanhydrid ist, acyliert wird, und wobei die Verbindungen der Formel I in freien Form oder als pharmazeutisch annehmbare Salze davon isoliert werden.

2. Verfahren zur Herstellung der Verbindungen der Strukturformel Ia

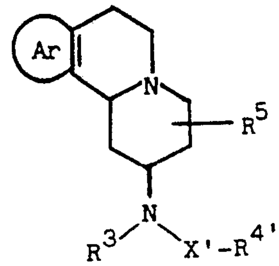

Ia

oder von pharmazeutisch annehmbaren Salzen davon, wobei Ar, $R^1$, $R^2$, $R^3$ und $R^5$ wie in Formel I definiert sind, und

$$-X'-R^{4'}$$

eine Gruppe mit der Struktur

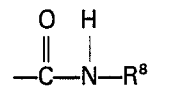 oder 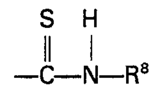

ist, wobei $R^8$ Wasserstoff oder entweder unsubstituiertes oder durch einer oder mehreren Gruppen der Formel OR oder —NRCOR substituiertes $C_{1-6}$-Alkyl ist, wobei R Wasserstoff oder $C_{1-3}$-Alkyl ist, dadurch gekennzeichnet, daß eine Verbindung der Formel IIa

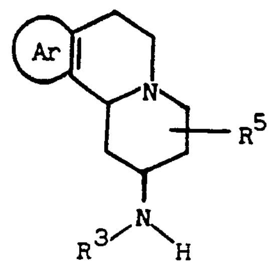

IIa ,

worin Ar, $R^1$, $R^2$, $R^3$ und $R^5$ wie in Formel Ia definiert sind, mit einem Isocyanat der Formel

$$O{=}C{=}NR^8$$

oder einem Isothiocyanat der Formel

$$S{=}C{=}NR^8$$

behandelt wird, und daß die entstandenen Verbindungen der Formel Ia oder pharmazeutisch annehmbare Salze davon isoliert werden.

3. Verfahren zur Herstellung von Verbindungen der Formel Ib

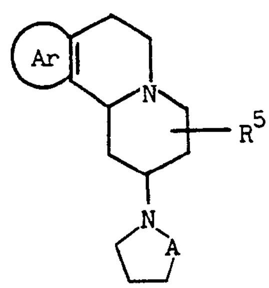

Ib

oder von pharmazeutisch annehmbaren Salzen der Verbindungen der Formel Ib, worin Ar, $R^1$, $R^2$ und $R^5$ wie in Formel I definiert sind, und A

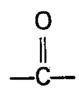 oder 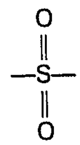

ist, dadurch gekennzeichnet, daß eine Verbindung der Formel

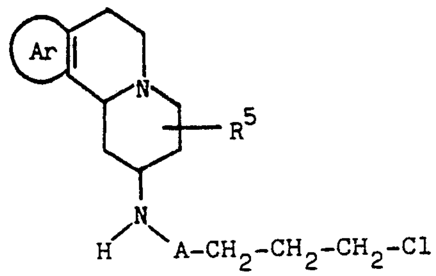 ,

worin Ar, $R^1$, $R^2$, $R^5$ und A wie in Formel Ib definiert sind, in Gegenwart einer starken Base cyclisiert wird, und die entstandenen Verbindungen der Formel Ib oder pharmazeutisch annehmbare Salze davon isoliert werden.

4. Verfahren zur Herstellung von Verbindungen der Formel Ic

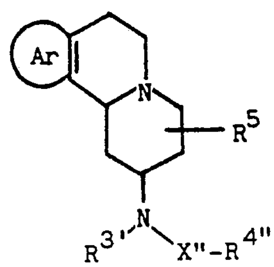

Ic

oder von pharmazeutisch annehmbaren Salzen der Verbindungen der Formel Ic, worin Ar, $R^1$, $R^2$ und $R^5$ wie in Formel I definiert sind, und $R^{3'}$ und die Gruppe —X″—$R^{4''}$ gemeinsam mit dem Stickstoffatom, an das sie gebunden sind, eine heterocyclische, aus 2-Oxazolidinon-1-yl und Succinimidoyl ausgewählte Struktur bilden, dadurch gekennzeichnet, daß eine Verbindung der Formel IIb

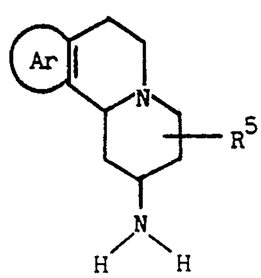

IIb ,

worin Ar, $R^1$, $R^2$ und $R^5$ wie in Formel Ic definiert sind, mit einem cyclischen Dicarbonsäureanhydrid der Formel

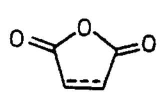 oder 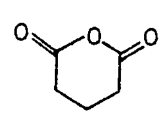

acyliert wird, und daß die entstandenen Verbindungen der Formel Ic in der freien Form oder als pharmazeutisch annehmbare Salze der genannten Verbindungen der Formel Ic isoliert werden.

5. Verfahren zur Herstellung von Verbindungen der Strukturformel Id

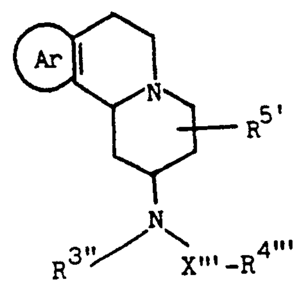

Id

oder von pharmazeutisch annehmbaren Salzen der Verbindungen der Formel Id, worin Ar, $R^1$ und $R^2$ wie in Formel I definiert sind, und $R^{5'}$ in der Stellung 1 oder in der Stellung 3 vorliegt, und der genannte Rest 5' zusammen mit dem Rest $R^{3'}$ und mit dem Stickstoffatom, an das diesergebunden ist, oder zusammen mit der Gruppe

$$—X'''—R^{4'''}$$

und dem Stickstoffatom, an das die genannte Gruppe gebunden ist, einen 5- oder 6-gliedrigen Ring bildet, welcher ein cyclisches Carbamat, ein cyclischer Harnstoff oder ein cyclisches Sulfonamid ist, durch Behandlung eines Ausgangsmaterials, welches einen entsprechenden Substituenten in der Stellung 1 oder in der Stellung-3 aufweist, mit Carbonyldiimidazol oder Sulfurylchlorid.

6. Verfahren nach einem der Ansprüche 1 bis 5, dadurch gekennzeichnet, daß ein Verbindung der Formel I hergestellt wird, worin Ar $R^1$, $R^2$-Benzo[b]furo- oder $R^1$, $R^2$-Benzo[b]thieno ist, wobei $R^1$ und $R^2$ Wasserstoff oder Halogen sind,

$R^3$ $C_{1-6}$-Alkyl ist,

X $—\overset{\overset{O}{\|}}{\underset{\underset{O}{\|}}{S}}—$ ist,

$R^4$ $C_{1-6}$-Alkyl, das entweder unsubstituiert oder durch Gruppen monosubstituiert ist, welche aus —OH, -Halogen, —$CO_2R^8$ und —$CONR^6R^7$ ausgewählt sind, wobei $R^8$ Wasserstoff oder entweder unsubstituiertes oder durch eine oder mehrere Gruppen der Formel OR oder —NRCOR substituiertes $C_{1-6}$-Alkyl ist, wobei R Wasserstoff oder $C_{1-3}$-Alkyl ist, und

$R^6$ und $R^7$ unabhängig voneinander Wasserstoff oder $C_{1-5}$-Alkyl sind, oder

$R^6$ oder $R^7$ gemeinsam mit dem Stickstoffatom, an das sie gebunden sind, einen Pyrrolidino-, Piperidino-, Morpholino-, Piperazino- oder N—$C_{1-3}$-Alkylpiperazino-Ring bilden;

—$N(R^{8'})_2$, wobei $R^{8'}$ $C_{1-3}$-Alkyl, $C_{2-5}$-Alkenyl, $C_{6-10}$-carbocyclisches Aryl oder —$CO_2R^8$ ist, wobei $R^8$ Wasserstoff oder entweder unsubstituiertes oder durch einen oder mehrere Reste OR oder —NRCOR substituiertes $C_{1-6}$-Alkyl ist, wobei R Wasserstoff oder $C_{1-3}$-Alkyl ist; oder ein 5- oder 6-gliedriger Heterocyclus, ausgewählt aus Imidazo, Thiazolo, Oxazolo, Piperidino, Piperazino, Pyridino und Pyrazino, ist, und

$R^5$ Wasserstoff oder $C_{1-6}$-Alkyl ist, oder daß pharmazeutisch annehmbare Salze der genannten Verbindung der Formel I hergestellt werden.

7. Verfahren nach einem der Anspruche 1 bis 6, dadurch gekennzeichnet, daß eine Verbindung der Formel I hergestellt wird, worin

Ar $R^1$, $R^2$-Benzo[b]furo- oder $R^1$, $R^2$-Benzo[b]thieno ist, wobei

$R^1$ und $R^2$ Wasserstoff sind,

$R^3$ Methyl ist,

$R^4$ $C_{1-6}$-Alkyl, Hydroxypropyl, Hydroxyethyl, Dimethylamino, $C_{1-3}$-Alkoxycarbonylethyl oder Dimethylaminocarbonylmethyl ist, und

$R^5$ Wasserstoff ist, oder daß pharmazeutisch annehmbare Salze der genannten Verbindung der Formel I hergestellt werden.

8. Verfahren nach einem der Ansprüche 1 bis 6, dadurch gekennzeichnet, daß

a) N-(1,3,4,6,7,12bα-Hexahydro-2H-benzo[b]furo[2,3a]-chinolizin-2β-yl)-N-methyl-2-hydroxyethansulfonamid;

b) N-(1,3,4,6,7,12bα-Hexahydro-2H-benzo[b]furo[2,3a]-chinolizin-2β-yl)-N-methyl-3-hydroxypropansulfonamid;

c) N-(1,3,4,6,7,12bα-Hexahydro-2H-benzo[b]furo[2,3a]-chinolizin-2β-yl)-N-methyl-methansulfonamid;

d) (+)-N-(1,3,4,6,7,12bα-Hexahydro-2H-benzo[b]furo[2,3a]-chinolizin-2β-yl)-N,N',N'-trimethylsulfamid; oder

e) N-(1,3,4,6,7,12bα-Hexahydro-2H-benzo[b]thieno-[2,3a]-chinolizin-2β-yl)-N-methyl-methan-sulfonamid;

oder ein pharmazeutisch annehmbares Salz der Verbindungen (a) bis (e) hergestellt wird.

9. Verfahren zur Herstellung einer pharmazeutischen Zusammensetzung mit $\alpha_2$-Adrenozeptor-Antagonistenaktivität, dadurch gekennzeichnet, daß Verbindungen der Formel I, oder pharmazeutisch annehmbare Salze der genannten Verbindungen der Formel I, die nach dem Verfahren von Anspruch 1 hergestellt sind, mit pharmazeutisch annehmbaren Trägern oder weiteren pharmazeutisch wirksamen Bestandteilen, ausgewählt aus Antidepressiva, Serotininwiederaufnahmeinhibitoren und Monoaminooxidaseinhibitoren, unter Bildung der pharmazeutischen Zusammensetzung formuliert werden.

10. Verfahren nach Anspruch 9, dadurch gekennzeichnet, daß die Wirkbestandteile, hergetellt nach den Verfahren der Ansprüche 2 bis 8, als Wirkbestandteile zur Formulierung der genannten pharmazeutischen Zusammensetzungen verwendet werden.

**Revendications pour les Etats contractants: BE CH DE FR GB IT LI LU NL SE**

1. Composés de formule développée I:

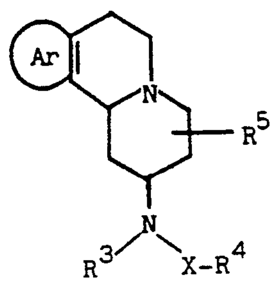

I

ou les sels pharmaceutiquement acceptables des composés de formule I, dans laquelle:

Ar représente un groupe hétérocyclique aromatique choisi parmi:

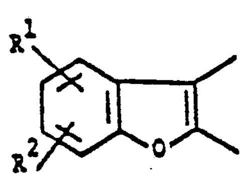

benzofuro-,

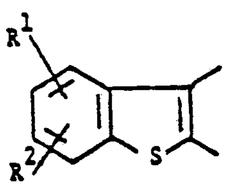

benzothiéno-,

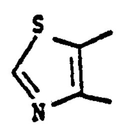

thiazolo-,

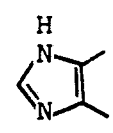

imidazo-,

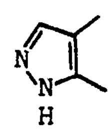

pyrazolo,

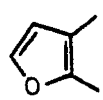

furo-,

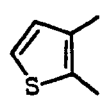

thiéno-,

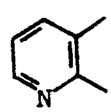

pyridino-;

$R^1$ et $R^2$ représentent indépendamment:
1) un atome d'hydrogène,
2) un atome d'halogène,
3) un groupe hydroxy,
4) un groupe alcoxy en $C_1$—$C_3$, ou
5) un groupe alkyle en $C_1$—$C_6$;

$R^3$ représente:
1) un atome d'hydrogène,
2) un groupe

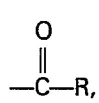

dans lequel R représente un atome d'hydrogène ou un groupe alkyle en $C_1$—$C_3$,

3) un groupe alkyle en $C_1$—$C_6$, non-substitué ou substitué avec au moins un groupe choisi parmi:
a) un groupe hydroxy,

b) un groupe carboxy,

c) un groupe (alcoxy en $C_1$—$C_3$) carbonyle,

d) un atome d'halogène,

e) un groupe alcoxy en $C_1$—$C_3$,

f) un groupe —$CONR^6R^7$, dans lequel $R^6$ et $R^7$ représentent indépendamment l'un de l'autre, un atome d'hydrogène ou un groupe alkyle en $C_1$—$C_5$, ou $R^6$ et $R^7$ forment ensemble avec l'atome d'azote auquel ils sont liés, un cycle pyrrolidino-, pipéridino-, morpholino-, pipérazino- ou N-(alkyle en $C_1$—$C_3$)pipérazino, ou

g) un groupe —$NR^6R^7$, dans lequel $R^6$ et $R^7$ sont tels que définis en 3f);

X représente un groupe

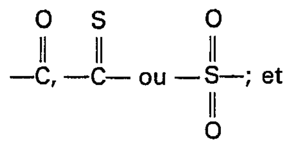

$R^4$ représente:

1) un groupe —$OR^8$ dans lequel $R^8$ représente un atome d'hydrogène ou un groupe alkyle en $C_1$—$C_6$, non-substitué ou substitué avec au moins un groupe de formule OR ou —NRCOR dans lequel R représente un atome d'hydrogène ou un groupe alkyle en $C_1$—$C_3$,

2) un groupe —$N(R^8)_2$, dans lequel $R^8$ est tel que défini en 1),

3) un groupe —$CO_2R^8$, dans lequel $R^8$ est tel que défini en 1),

4) un groupe —$CONR^6R^7$, dans lequel $R^6$ et $R^7$ représentent indépendamment l'un de l'autre, un atome d'hydrogène ou un groupe alkyle en $C_1$—$C_5$, ou $R^6$ et $R^7$ forment ensemble avec l'atome d'azote auquel ils sont liés, un cycle pyrrolidino-, pipéridino-, morpholino-, pipérazino- ou N-(alkyle en $C_1$—$C_3$)pipérazino,

5) un groupe alkyle en $C_1$—$C_6$ non-substitué ou substitué avec des groupes choisis parmi les groupes —$OR^8$, -halo, —$CO_2R^8$ et —$CONR^6R^7$ dans lesquels $R^6$ et $R^7$ sont tels que définis en 4) et $R^8$ est tel que défini en 1),

6) un groupe alkényle en $C_2$—$C_5$,

7) un groupe alkynyle en $C_2$—$C_5$,

8) un groupe cycloalkyle en $C_3$—$C_6$,

9) un groupe hétérocyclique à 5 ou 6 chaînons choisis parmi un groupe imidazo, thiazolo, oxazolo, pipéridino, pipérazino, pyridino et pyrazino,

10) un groupe (carbocyclique)aryle de 6 à 10 atomes de carbone non-substitué ou substitué avec au moins un des groupes choisis parmi un groupe halo et OR dans lequel R représente un atome d'hydrogène ou un groupe alkyle en $C_1$—$C_3$; ou

$R^3$ ainsi que le groupe —X—$R^4$ forment ensemble avec l'atome d'azote auquel ils sont liés, un groupe hétérocyclique choisi parmi un groupe 2-oxazolidinon-1-yle et succinimidoyle; et

$R^5$ représente:

1) un atome d'hydrogène,

2) un groupe alkyle en $C_1$—$C_6$ non-substitué ou substitué avec au moins un groupe de formule —$OR^8$, —$NR^8COR^8$ ou —$CO_2R^8$, dans lequel $R^8$ représente un atome d'hydrogène ou un groupe alkyle en $C_1$—$C_6$, non-substitué ou substitué avec au moins un groupe de formule OR ou —NRCOR dans lequel R représente un atome d'hydrogène ou un groupe alkyle en $C_1$—$C_3$,

3) un groupe —$CO_2R^8$ dans lequel $R^8$ est tel que défini en 2), ou

4) un groupe —$CONR^6R^7$ dans lequel $R^6$ et $R^7$ représentent indépendamment l'un de l'autre, un atome d'hydrogène ou un groupe alkyle en $C_1$—$C_5$, ou $R^6$ et $R^7$ forment ensemble avec l'atome d'azote auquel ils sont liés, un cycle pyrrolidino-, pipéridino-, morpholino-, pipérazino- ou N-(alkyle en $C_1$—$C_3$)pipérazino; ou

à condition que $R^5$ soit en position 1 ou en position 3, le groupe $R^5$ puisse former avec le groupe $R^3$ et l'atome d'azote auquel il est lié, ou avec le groupe —X—$R^4$ et l'atome d'azote auquel ce groupe est lié, un cycle à 5 ou 6 chaînons; et

à condition que si le groupe —X—$R^4$ est un groupe de formule:

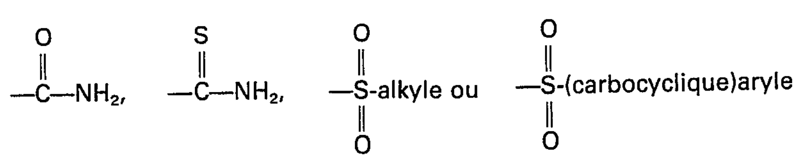

le groupe Ar ne puisse alors pas être le groupe hétérocyclique aromatique thiéno ou pyridino.

2. Composé de formule selon la revendication 1, ou les sels pharmaceutiquement acceptables de composés de formule I dans laquelle

Ar représente un groupe $R^1$,$R^2$-benzo[b]furo- ou $R^1$,$R^2$-benzo[b]thiéno dans lequel $R^1$ et $R^2$ représentent un atome d'hydrogène ou un groupe halo,

$R^3$ représente un groupe alkyle en $C_1$—$C_6$,

X représente un groupe

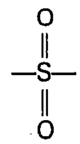

$R^4$ représente un groupe alkyle en $C_1—C_6$ non-substitué ou monosubstitué avec des groupes choisis parmi les groupes —OH, -halo, —$CO_2R^8$ et —$CONR^6R^7$, dans lesquels

$R^8$ représente un atome d'hydrogène ou un groupe alkyle en $C_1—C_6$ non-substitué ou substitué avec au moins un groupe de formule OR ou —NRCOR, dans lequel

R représente un atome d'hydrogène ou un groupe alkyle en $C_1—C_3$, et

$R^6$ et $R^7$ représentent indépendamment l'un de l'autre, un atome d'hydrogène ou un groupe alkyle en $C_1—C_5$, ou les groupes

$R^6$ et $R^7$ forment ensemble avec l'atome d'azote auquel ils sont liés, un cycle pyrrolidino-, pipéridino-, morpholino-, pipérazino ou N-(alkyle en $C_1—C_3$)pipérazino;

un groupe —$N(R^{8'})_2$, dans lequel $R^{8'}$ représente un groupe alkyle en $C_1—C_3$,

un groupe alkényle en $C_2—C_5$,

un groupe (carbocyclique)aryle en $C_6—C_{10}$,

un groupe —$CO_2R^8$ dans lequel $R^8$ représente un atome d'hydrogène ou un groupe alkyle en $C_1—C_6$ non-substitué ou substitué avec au moins un groupe de formule OR ou —NRCOR dans lequel

R représente un atome d'hydrogène ou un groupe alkyle en $C_1—C_3$, ou

un groupe hétérocyclique à 5 ou 6 chaînons choisi parmi un groupe imidazo, thiazolo, oxazolo, pipéridino, pyridino et pyrazino; et

$R^5$ représente un atome d'hydrogène ou alkyle en $C_1—C_6$.

3. Composé de formule I selon la revendication 2 ou un sel pharmaceutiquement acceptable de celui-ci, dans lequel:

Ar représente un groupe $R^1$,$R^2$-benzo[b]furo- ou $R^1$,$R^2$-benzo[b]thiéno- dans lequel

$R^1$ et $R^2$ représentent un atome d'hydrogène;

$R^3$ représente un groupe méthyle;

$R^4$ représente un groupe alkyle en $C_1—C_6$, hydroxypropyle, hydroxyéthyle, diméthylamine, (alcoxy en $C_1—C_3$)carbonyléthyle ou diméthylaminocarbonylméthyle; et

$R^5$ représente un atome d'hydrogène.

4. Composé de formule I selon la revendication 2, qui est:

a) le N-(1,3,4,6,7,12bα-hexahydro-2H-benzo[b]furo[2,3-a]quinolizin-2β-yl)-N-méthyl-2-hydroxyéthane-sulfonamide;

b) le N-(1,3,4,6,7,12bα-hexahydro-2H-benzo[b]furo[2,3-a]quinolizin-2β-yl)-N-méthyl-3-hydroxy-propanesulfonamide;

c) le N-(1,3,4,6,7,12bα-hexahydro-2H-benzo[b]furo[2,3-a]quinolizin-2β-yl)-N-méthyl-méthane-sulfonamide;

d) le (+)-N-(1,3,4,6,7,12bα-hexahydro-2H-benzo[b]furo[2,3-a]quinolizin-2β-yl)-N,N',N'-triméthyl-sulfamide; ou

e) le N-(1,3,4,6,7,12bα-hexahydro-2H-benzo[b]thiéno[2,3-a]quinolizin-2β-yl)-N-méthyl-méthanesulfon-amide; ou

un sel pharmaceutiquement acceptable des composés (a) à (e).

5. Procédé de préparation des composés de formule I:

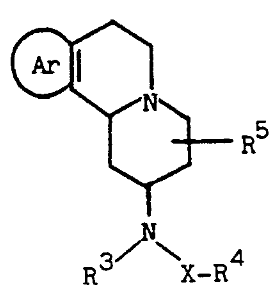

I

selon la revendication 1, ou des sels pharmaceutiquement acceptables des composés de formule I dans laquelle:

les groupes Ar, $R^1$, $R^2$, $R^3$, X, $R^4$ et $R^5$ sont tels que définis dans la revendication 1, caractérisé en ce qu'un composé de formule IIa:

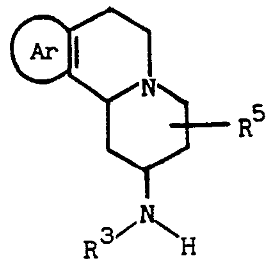

IIa ,

dans laquelle les groupes Ar, $R^1$, $R^2$, $R^3$ et $R^5$ sont tels que définis dans la formule I, est acylé avec un réactif acylant qui introduit le groupe acyle de formule:

$$—X—R^4$$

dans laquelle les groupes $R^4$ et X sont tels que définis dans la formule I, cet agent acylant étant un halogénure d'acide, un ester activé, un anhydride d'acide ou un mélange d'anhydrides d'acide, et on isole les composés de formule I sous la forme libre ou sous la forme de sels pharmaceutiquement acceptables de ceux-ci.

6. Procédé de préparation de composés de formule développée Ia:

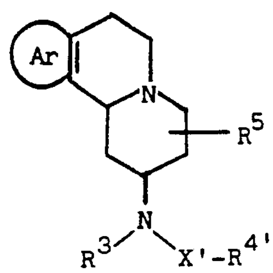

Ia

selon la revendication 1, ou des sels pharmaceutiquement acceptables de ceux-ci, dans lesquels les groupes Ar, $R^1$, $R^2$, $R^3$ et $R^5$ sont tels que définis dans la revendication 1, et le groupe $—X'—R^{4'}$ représente un groupe de formule:

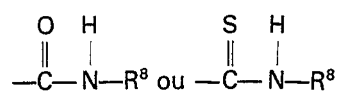

ou

dans lequel $R^8$ représente un atome d'hydrogène ou un groupe alkyle en $C_1—C_6$ non-substitué ou substitué avec au moins un groupe de formule OR ou —NRCOR dans lequel R représente un atome d'hydrogène ou un groupe alkyle en $C_1—C_3$ caractérisé en ce qu'on traite un composé de formule IIa:

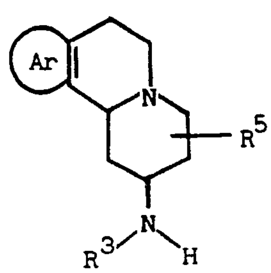

IIa ,

dans laquelle les groupes Ar, $R^1$, $R^2$, $R^3$ et $R^5$ sont tels que définis dans la formule Ia, avec un isocyanate de formule

$$O{=}C{=}NR^8$$

ou un isothiocyanate de formule:

$$S{=}C{=}NR^8$$

et on isole les composés résultants de formule Ia ou les sels pharmaceutiquement acceptables de ceux-ci.

7. Procédé de préparation de composés de formule Ib:

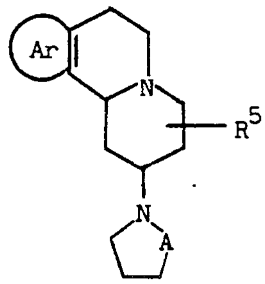

Ib

selon la revendication 1 ou des sels pharmaceutiquement acceptables des composés de formule Ib dans laquelle les groupes Ar, $R^1$, $R^2$ et $R^5$ sont tels que définis dans la revendication 1, et le groupe A représente un groupe:

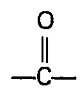 ou 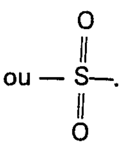.

caractérisé en ce qu'on cyclise un composé de formule:

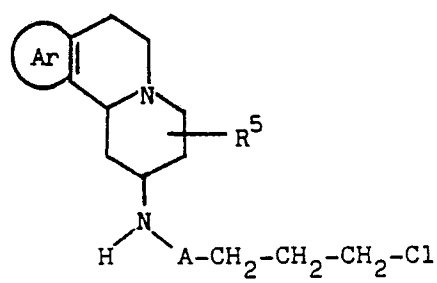 ,

dans laquelle les groupes Ar, $R^1$, $R^2$, $R^5$ et A sont tels que définis dans la formule Ib, en présence d'une base forte, et on isole les composés résultants de formule Ib ou les sels pharmaceutiquement acceptables de ceux-ci.

8. Procédé de préparation de composés de formule Ic:

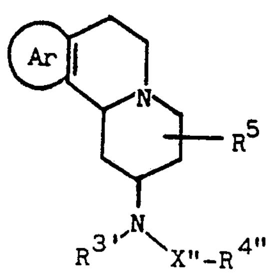

Ic

selon la revendication 1 ou des sels pharmaceutiquement acceptables des composés de formule Ic dans laquelle les groupes Ar, $R^1$, $R^2$ et $R^5$ sont tels que définis dans la revendication 1, et le groupe $R^{3'}$ ainsi que le groupe —X''—$R^{4''}$ forment ensemble avec l'atome d'azote auquel ils sont liés, un groupe hétérocyclique choisi parmi un groupe 2-oxazolidinon-1-yle et succinimidoyle; caractérisé en qu'on acyle un composé de IIb:

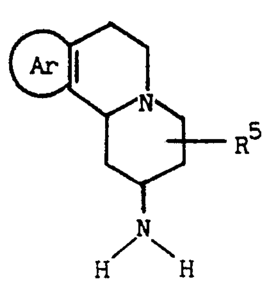

IIb ,

dans laquelle les groupes Ar, $R^1$, $R^2$ et $R^5$ sont tels que définis dans la formule Ic, avec un anhydride cyclique dicarboxylique de formule:

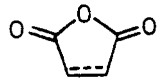

ou

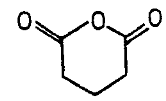

et en ce qu'on isole les composés résultants de formule Ic sous la forme libre ou sous la forme de sels pharmaceutiquement acceptables desdits composés de formule Ic.

9. Procédé de préparation de composé de formule développée Id

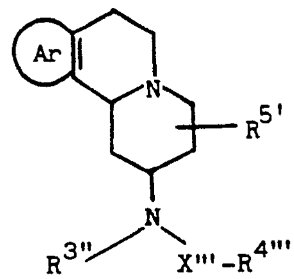

Id

selon la revendication 1 ou des sels pharmaceutiquement acceptables des composés de formule Id dans laquelle les groupes Ar, $R^1$ et $R^2$ sont tels que définis dans la revendication 1, et le groupe $R^{5'}$ est en position 1 ou en position 3, et ce groupe $R^{5'}$ forme ensemble avec le groupe $R^{3''}$ et avec l'atome d'azote auquel il est lié, ou avec le groupe:

$$—X'''—R^{4'''}$$

et l'atome d'azote auquel ce groupe est lié, un cycle à 5 ou 6 chaînons qui forme en carbamate cyclique, une urée cyclique ou un sulfamide cyclique, en traitant un produit de départ comportant un substituant correspondant en position 1 ou en position 3, avec du carbonyldiimidazole ou du chlorure de sulfonyle.

10. Composition pharmaceutique ayant une activité antagoniste sur les récepteurs $\alpha_2$-adrénergiques, caractérisée en ce qu'elle comprend comme ingrédient pharmaceutiquement actif, un composé de formule développée selon la revendication 1 ou un sel pharmaceutiquement acceptable du composé de formule I.

11. Composition pharmaceutique selon la revendication 10, caractérisée en ce qu'elle comprend comme ingrédient pharmaceutiquement actif, un composé selon l'une des revendications 2 à 4, ou un sel selon l'une des revendications 2 à 4, ou un sel pharmaceutiquement acceptable dudit composé, et éventuellement en plus, un véhicule pharmaceutiquement acceptable.

**Revendications pour l'Etat contractant: AT**

1. Procédé de préparation de composés de formule développée I:

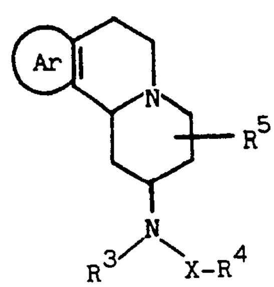

I

ou des sels pharmaceutiquement acceptables des composés de formule I, dans laquelle:

Ar représente un groupe hétérocyclique aromatique choisi parmi les groupes:

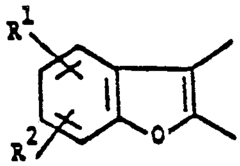 benzofuro-, 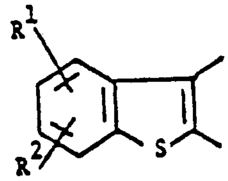 benzothiéno-, 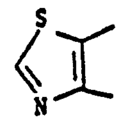 thiazolo-, 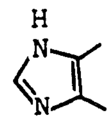 imidazo-, 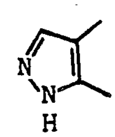 pyrazolo,

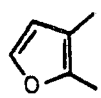 furo-, 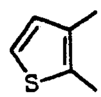 thiéno-, 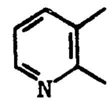 pyridino-;

$R^1$ et $R^2$ représentent indépendamment:
1) un atome d'hydrogène,
2) un atome d'halogène,
3) un groupe hydroxy,
4) un groupe alcoxy en $C_1$—$C_3$, ou
5) un groupe alkyle en $C_1$—$C_6$;

$R^3$ représente:
1) un atome d'hydrogène,
2) un groupe

$$-\overset{\overset{\displaystyle O}{\|}}{C}-R,$$

dans lequel R représente un atome d'hydrogène ou un groupe alkyle en $C_1$—$C_3$,

3) un groupe alkyle en $C_1$—$C_6$, non-substitué ou substitué avec au moins un groupe choisi parmi:
a) un groupe hydroxy,
b) un groupe carboxy,
c) un groupe (alcoxy en $C_1$—$C_3$) carbonyle,
d) un atome d'halogène,
e) un groupe alcoxy en $C_1$—$C_3$,
f) un groupe —$CONR^6R^7$, dans lequel $R^6$ et $R^7$ représentent indépendamment l'un de l'autre, un atome d'hydrogène ou un groupe alkyle en $C_1$—$C_5$, ou les groupes $R^6$ et $R^7$ forment ensemble avec l'atome d'azote auquel ils sont liés, un cycle pyrrolidino-, pipéridino-, morpholino-, pipérazino- ou N-(alkyle en $C_1$—$C_3$)pipérazino, ou
g) un groupe —$NR^6R^7$, dans lequel $R^6$ et $R^7$ sont tels que définis en 3f);

X représente un groupe

$$-\overset{\overset{\displaystyle O}{\|}}{C},\ -\overset{\overset{\displaystyle S}{\|}}{C}-\ \text{ou}\ -\underset{\underset{\displaystyle O}{\|}}{\overset{\overset{\displaystyle O}{\|}}{S}}-;\ \text{et}$$

$R^4$ représente:

1) un groupe —$OR^8$ dans lequel $R^8$ représente un atome d'hydrogène ou un groupe alkyle en $C_1$—$C_6$, non-substitué ou substitué avec au moins un groupe de formule OR ou —NRCOR dans lequel R représente un atome d'hydrogène ou un groupe alkyle en $C_1$—$C_3$,
2) un groupe —$N(R^8)_2$, dans lequel $R^8$ est tel que défini en (1),
3) un groupe —$CO_2R^8$, dans lequel $R^8$ est tel que défini en (1),
4) un groupe —$CONR^6R^7$, dans lequel $R^6$ et $R^7$ représentent indépendamment l'un de l'autre, un atome d'hydrogène ou un groupe alkyle en $C_1$—$C_5$, ou les groupes $R^6$ et $R^7$ forment ensemble avec l'atome d'azote auquel ils sont liés, un cycle pyrrolidino-, pipéridino-, morpholino-, pipérazino- ou N-(alkyle en $C_1$—$C_3$)pipérazino,
5) un groupe alkyle en $C_1$—$C_6$ non-substitué ou substitué avec des groupes choisis parmi les groupes —$OR^8$, -halo, —$CO_2R^8$ et le groupe —$CONR^6R^7$ dans lesquels $R^6$ et $R^7$ sont tels que définis en (4) et le groupe $R^8$ est tel que défini en (1),
6) un groupe alkényle en $C_2$—$C_5$,
7) un groupe alkynyle en $C_2$—$C_5$,
8) un groupe cycloalkyle en $C_3$—$C_6$,

9) un groupe hétérocyclique à 5 ou 6 chaînons choisi parmi un groupe imidazo, thiazolo, oxazolo, pipéridino, pipérazino, pyridino et pyrazino, ou,

10) un groupe (carbocyclique)aryle comportant de 6 à 10 atomes de carbone, non-substitué avec au moins un groupe choisis parmi un groupe halo et OR dans lequel R représente un atome d'hydrogène ou un groupe alkyle en $C_1$—$C_3$; ou le groupe

$R^3$ ainsi que le groupe —X—$R^4$ forment ensemble avec l'atome d'azote auquel ils sont liés, un groupe hétérocyclique choisi parmi un groupe 2-oxazolidinon-1-yle et succinimidoyle; et

$R^5$ représente:

1) un atome d'hydrogène,

2) un groupe alkyle en $C_1$—$C_6$ non-substitué ou substitué avec au moins un groupe de formule —$OR^8$, —$NR^8COR^8$ ou —$CO_2R^8$, dans lequel $R^8$ représente un atome d'hydrogène ou un groupe alkyle en $C_1$—$C_6$, non-substitué ou substitué avec au moins un groupe de formule OR ou —NRCOR dans lequel R représente un atome d'hydrogène ou un groupe alkyle en $C_1$—$C_3$,

3) un groupe —$CO_2R^8$ dans lequel $R^8$ est tel que défini en (2), ou

4) un groupe —$CONR^6R^7$ dans lequel $R^6$ et $R^7$ représentent indépendamment l'un de l'autre, un atome d'hydrogène ou un groupe alkyle en $C_1$—$C_5$, ou les groupes $R^6$ et $R^7$ forment ensemble avec l'atome d'azote auquel ils sont liés, un cycle pyrrolidino-, pipéridino-, morpholino-, pipérazino- ou N-(alkyle en $C_1$—$C_3$)pipérazino; ou

à condition que le groupe $R^5$ soit en position 1 ou en position 3, le groupe $R^5$ puisse former avec le groupe $R^3$ et l'atome d'azote auquel il est lié, ou avec le groupe —X—$R^4$ et l'atome d'azote auquel ce groupe est lié, un cycle à 5 ou 6 chaînons; et

à condition que si le groupe —X—$R^4$ est un groupe de formule:

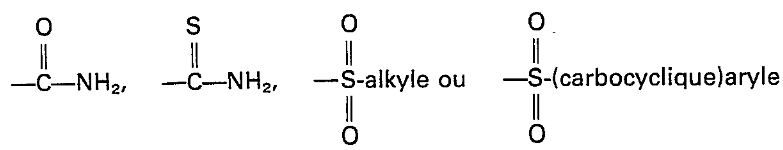

le groupe Ar ne puisse alors pas être le groupe hétérocyclique aromatique thiéno ou pyridino, caractérisé en ce qu'on acyle un composé de formule IIa

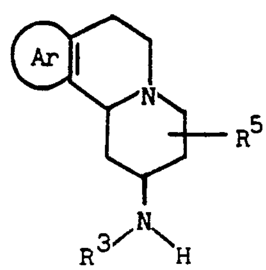

IIa ,

dans laquelle les groupes Ar, $R^1$, $R^2$, $R^3$ et $R^5$ sont tels que définis dans la revendiction 1, avec un réactif acylant qui introduit le groupe acyle de formule:

—X—$R^4$

dans laquelle les groupes $R^4$ et X sont tels que définis dans la formule I, et cet agent acylant étant un halogénure d'acide, un ester activé, un anhydride d'acide ou un mélange d'anhydrides d'acide, et on isole les composés de formule I sous la forme libre ou sous la forme de sels pharmaceutiquement acceptables de ceux-ci.

2. Procédé de préparation de composés de formule développée Ia:

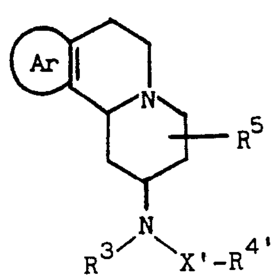

Ia

ou des sels pharmaceutique acceptables de ceux ci, dans lesquels les groupes Ar, $R^1$, $R^2$, $R^3$ et $R^5$ sont tels que définis dans la formule I, et le groupe —X'—$R^{4'}$ représente un groupe de formule:

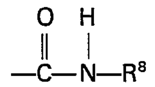 ou 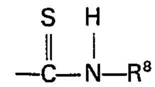

dans lequel $R^8$ représente un atome d'hydrogène ou un groupe alkyle en $C_1$—$C_6$ non-substitué ou substitué avec au moins un groupe de formule OR ou —NRCOR dans lequel R représente un atome d'hydrogène ou un groupe alkyle en $C_1$—$C_3$ caractérisé en ce qu'on traite un composé de formule IIa:

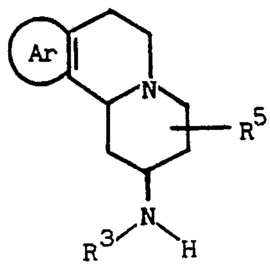

IIa ,

dans laquelle les groupes Ar, $R^1$, $R^2$, $R^3$ et $R^5$ sont tels que définis dans la formule Ia, avec un isocyanate de formule:

$$O{=}C{=}NR^8$$

ou un isothiocyanate de formule:

$$S{=}C{=}NR^8$$

et on isole les composés résultants de formule Ia ou les sels pharmaceutiquement acceptables de ceux-ci.

3. Procédé de préparation de composés de formule Ib:

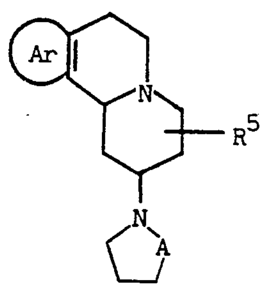

Ib

ou des sels pharmaceutiquement acceptables des composés de formule Ib dans laquelle les groupes Ar, $R^1$, $R^2$ et $R^5$ sont tels que définis dans la formule I, et le groupe A représente un groupe:

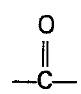 ou 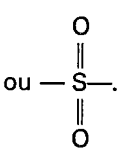.

caractérisé en ce qu'on cyclise un composé de formule:

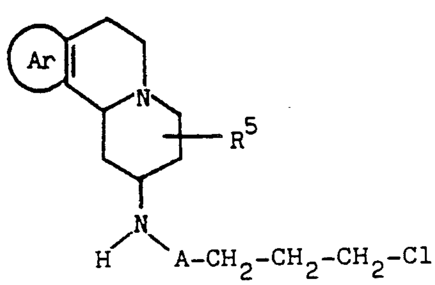

,

dans laquelle les groupes Ar, $R^1$, $R^2$, $R^5$ et A sont tels que définis dans la formule Ib, en présence d'une base forte, et on isole les composés résultants de formule Ib ou les sels pharmaceutiquement acceptables de ceux-ci.

4. Procédé de préparation de composés de formule Ic:

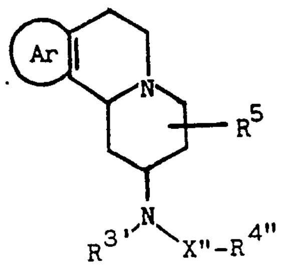

Ic

ou des sels pharmaceutiquement acceptables des composés de formule Ic dans laquelle les groupes Ar, $R^1$, $R^2$ et $R^5$ sont tels que définis dans la formule I, et le groupe $R^{3'}$ ainsi que le groupe $-X''-R^{4''}$ forment ensemble avec l'atome d'azote auquel ils sont liés, un groupe hétérocyclique choisi parmi un groupe 2-oxazolidinon-1-yle et succinimidoyle; caractérisé en qu'on acyle un composé de IIb:

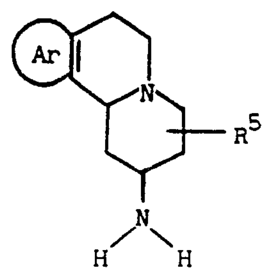

IIb ,

dans laquelle les groupes Ar, $R^1$, $R^2$ et $R^5$ sont tels que définis dans la formule Ic, avec un anhydride dicarboxylique cyclique de formule:

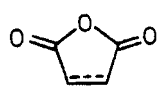

ou

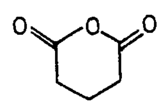

et en ce qu'on isole les composés résultants de formule Ic sous la forme libre ou sous la forme de sels pharmaceutiquement acceptables desdits composés de formule Ic.

5. Procédé de préparation de composé de formule développée Id:

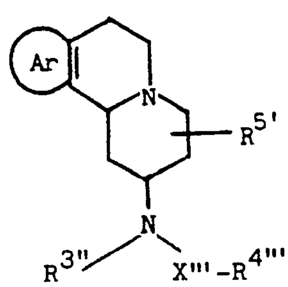

Id

ou des sels pharmaceutiquement acceptables des composés de formule Id dans laquelle les groupes Ar, $R^1$ et $R^2$ sont tels que définis dans la formule 1, et le groupe $R^{5'}$ est en position 1 ou en position 3, et ce groupe $R^{5'}$ forme ensemble avec le groupe $R^{3''}$ et avec l'atome d'azote auquel il est lié, ou avec le groupe:

$$-X'''-R^{4'''}$$

ainsi que l'atome d'azote auquel ce groupe est lié, un cycle à 5 ou 6 chaînons qui forme en carbamate cyclique, une urée cyclique ou un sulfamide cyclique, en traitant un produit de départ comportant un substituant correspondant en position 1 ou en position 3, avec du carbonyldiimidazole ou du chlorure de sulfonyle.

6. Procédé selon l'une des revendications 1 à 5, caractérisé en ce qu'on prépare un composé de formule I dans laquelle:

Ar représente un groupe $R^1$,$R^2$-benzo[b]furo- ou $R^1$,$R^2$-benzo[b]thiéno dans lequel les groupes $R^1$ et $R^2$ représentent un atome d'hydrogène ou d'halogène;

$R^3$ représente un groupe alkyle en $C_1$—$C_6$,

X représente un groupe

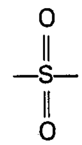

$R^4$ représente un groupe alkyle en $C_1$—$C_6$ non-substitué ou monosubstitué avec des groupes choisis parmi les groupes —OH, -halo, —$CO_2R^8$ et —$CONR^6R^7$,

$R^8$ représente un atome d'hydrogène ou un groupe alkyle en $C_1$—$C_6$ non-substitué ou substitué avec au moins un groupe de formule OR ou —NRCOR, dans lequel

R représente un atome d'hydrogène ou un groupe alkyle en $C_1$—$C_3$, et les groupes

$R^6$ et $R^7$ représentent indépendamment l'un de l'autre, un atome d'hydrogène ou un groupe alkyle en $C_1$—$C_5$, ou les groupes

$R^6$ et $R^7$ forment ensemble avec l'atome d'azote auquel ils sont liés, un cycle pyrrolidino-, pipéridino-, morpholino-, pipérazino ou N-(alkyle en $C_1$—$C_3$)pipérazino;

un groupe —$N(R^{8'})_2$, dans lequel $R^{8'}$ représente un groupe alkyle en $C_1$—$C_3$,

un groupe alkényle en $C_2$—$C_5$,

un groupe (carbocyclique)aryle en $C_6$—$C_{10}$,

un groupe —$CO_2R^8$ dans lequel $R^8$ représente un atome d'hydrogène ou un groupe alkyle en $C_1$—$C_6$ non-substitué ou substitué avec au moins un groupe de formule OR ou —NRCOR dans lequel

R représente un atome d'hydrogène ou un groupe alkyle en $C_1$—$C_3$, ou

un groupe imidazo, thiazolo, oxazolo, pipéridino, pyridino et pyrazino; et

$R^5$ représente un atome d'hydrogène ou un groupe alkyle en $C_1$—$C_6$; ou on prépare des sels pharmaceutiquement acceptables de ce composé de formule I.

7. Procédé selon l'une des revendications 1 à 6, caractérise en ce qu'on prépare un composé de formule I dans laquelle:

Ar représente un groupe $R^1$,$R^2$-benzo[b]furo- ou $R^1$,$R^2$-benzo[b]thiéno- dans lequel les groupes $R^1$ et $R^2$ représentent un atome d'hydrogène;

$R^3$ représente un groupe méthyle;

$R^4$ représente un groupe alkyle en $C_1$—$C_6$, hydroxypropyle, hydroxyéthyle, diméthylamine, (alcoxy en $C_1$—$C_3$)carbonyléthyle ou diméthylaminocarbonylméthyle; et

$R^5$ représente un atome d'hydrogène; ou on prépare des sels pharmaceutiquement acceptables de ce composé de formula I.

8. Procédé selon l'une des revendictions 1 à 6, caractérisé en ce qu'on prépare:

a) le N-(1,3,4,6,7,12bα-hexahydro-2H-benzo[b]furo[2,3-a]quinolizin-2β-yl)-N-méthyl-2-hydroxyéthanesulfonamide;

b) le N-(1,3,4,6,7,12bα-hexahydro-2H-benzo[b]furo[2,3-a]quinolizin-2β-yl)-N-méthyl-3-hydroxypropanesulfonamide;

c) le N-(1,3,4,6,7,12bα-hexahydro-2H-benzo[b]furo[2,3-a]quinolizin-2β-yl)-N-méthyl-méthanesulfonamide;

d) le (+)-N-(1,3,4,6,7,12bα-hexahydro-2H-benzo[b]furo[2,3-a]quinolizin-2β-yl)-N,N',N'-triméthylsulfamide; ou

e) le N-(1,3,4,6,7,12bα-hexahydro-2H-benzo[b]thiéno[2,3-a]quinolizin-2β-yl)-N-méthyl-méthanesulfonamide; ou

un sel pharmaceutiquement acceptable des composés (a) à (e).

9. Procédé de préparation d'une composition pharmaceutique ayant une activité antagoniste sur les réceuteurs $\alpha_2$-adrénergiques, caractérisé en ce qu'on mélange des composés de formule I ou des sels pharmaceutiquement acceptables de ces composés de formule I préparés conformément au procédé de la revendiction 1, avec des véhicules pharmaceutiquement accpetables ou d'autres ingrédients pharmaceutiquement actifs choisis parmi des antidépresseurs, des inhibiteurs du recaptage de la sérotonine et ldes inhibiteurs de la monoamine oxyxase afin de former la compositoin pharmaceutique.

10. Procédé selon la revendication 9, caractérisé en ce que les ingrédients actics préparées selon les procédés revendications 2 à 8, sont utilisés comme ingrédients actifs pour préparer lesdites compositions pharmaceutiques.